# EUROPEAN PATENT SPECIFICATION

(11) **EP 1 141 028 B1**
(45) Date of publication and mention of the grant of the patent: **17.02.2010**
(21) Application number: 99966644.9
(22) Date of filing: 23.12.1999
(51) Int. Cl.: C07K 16/28, C12N 5/071, G01N 33/53, G01N 33/68

(54) **HUMAN MONOCLONAL ANTIBODIES TO CTLA-4**
HUMANE MONOKLONALE ANTIKÖRPER GEGEN CTLA-4
ANTICORPS MONOCLONAUX HUMAINS DIRIGES CONTRE L'ANTIGENE CTLA-4

(30) Priority: 23.12.1998 US 113647 P
(43) Date of publication of application: 10.10.2001
(60) Divisional application: 09007161.4
(73) Proprietor: Pfizer Inc., New York, NY 10017 (US); Amgen Fremont Inc., Fremont, CA 94555 (US)
(72) Inventor: HANSON, Douglas, Charles, Niantic, CT 06357 (US); NEVEU, Mark, Joseph, Mystic, CT 06355 (US); MUELLER, Eileen, Elliot, Old Lyme, CT 06371 (US); HANKE, Jeffrey, Herbert, Reading, MA 01867 (US); GILMAN, Steven, Christopher, Old Lyme, CT 06371 (US); DAVIS, C., Geoffrey, Burlingame, CA 94010 (US); CORVALAN, Jose, Ramon, Foster City, CA 94404 (US)
(74) Representative: Vossius & Partner
(86) International application number: PCT/US1999/030895
(87) International publication number: WO 2000/037504

(56) References cited:
- WO-A-95/33770
- WO-A-96/33735
- WO-A-98/42752
- WO-A-98/50433
- A. JAKOBOVITS: "The long-awaited magic bullets: therapeutic human monoclonal antibodies from transgenic mice." EXPERT OPINION ON INVESTIGATIONAL DRUGS, vol. 7, no. 4, April 1998 (1998-04), pages 607-614, XP002084509 London, GB
- P. MARRACK ET AL.: "The staphylococcal enterotoxins and their relatives." SCIENCE, vol. 248, no. 4956, 11 May 1990 (1990-05-11), pages 705-711, XP002138564 New York, NY, USA
- LUIS H. CAMACHO: "NOVEL THERAPIES TARGETING THE IMMUNE SYSTEM: CTLA4 BLOCKADE WITH TREMELIMUMAB (CP-675,206), A FULLY HUMAN MONOCLONAL ANTIBODY" EXPERT OPIN. INVESTIG. DRUGS, vol. 17, no. 3, 2008, pages 371-385,
- STEVEN J. O'DAY ET AL: "TARGETING CYTOTOXIC t-LYMPHOCYTE ANTIGEN-4 (CTLA-4)" CANCER, vol. 110, no. 12, 15 December 2007 (2007-12-15), pages 2614-2627,
- LEE D. CRANMER ET AL: "THE ROLE OF THE CTLA4 BLOCKADE IN THE TREATMENT OF MALIGNANT MELANOMA" CANCER INVESTIGATION, vol. 25, 2007, pages 613-631,

## Description

### BACKGROUND OF THE INVENTION

### 1. Cross-Reference to Related Applications

The present application claims priority to U.S. Provisional Patent Application, Serial No. 60/113,647, filed December 23, 1998, the disclosure of which is hereby incorporated in its entirety herein.

### 2. Summary of the Invention

In accordance with the present invention, there are provided fully human monoclonal antibodies against human cytotoxic T-lymphocyte antigen 4 (CTLA-4). Nucleotide sequences encoding and amino acid sequences comprising heavy and light chain immunoglobulin molecules, particularly contiguous heavy and light chain sequences spanning the complementarity determining regions (CDRs), specifically from within FR1 and/or CDR1 through CDR3 and/or within FR4, are provided.

### 3. Background of the Technology

regulation of immune response in patients would provide a desirable treatment of many human diseases that could lead to a specificity of action that is rarely found through the use of conventional drugs. Both up-regulation and down-regulation of responses of the immune system would be possible. The roles of T cells and B cells have been extensively studied and characterized' in connection with the regulation of immune response. From these studies, the role of T cells appear, in many cases, to be particularly important in disease prevention and treatment.

T cells possess very complex systems for controlling their interactions. Interactions between T cells utilize numerous receptors and soluble factors for the process. Thus, what effect any particular signal may have on the immune response generally varies and depends on the particular factors, receptors and counter-receptors that are involved in the pathway. The pathways for drown-regulating responses are as important as those required for activation. Thymic education leading to T cell tolerance is one mechanism for preventing an immune response to a particular antigen. Other mechanisms, such as secretion of suppressive cytokines, are also known.

Activation of T cells requires not only stimulation through the antigen receptor (T cells receptor (PCR)), but additional signaling through costimulatory surface molecules such as CD28. The ligands for CD28 are the B7-1 (CD80) and B7-2 (CD86) proteins, which are expressed on antigen-presenting cells such as dendritic cells, activated B/cells or monocytes that interact with T cell C28 or CTLA-4 to deliver a costimulatory signal. The role of costimulatory signaling was studied in experimental allergic encephalomyelitis (EAE) by Perrin et al., Immunol. Res.14:189-99 (1995). EAE is an autoimmune disorder, induced by Th1 cells directed against myelin antigens that provides an *in vivo* model for studying the role of B7-mediated costimulation in the induction of a pathological immune response. Using a soluble fusion protein ligand for the B7 receptors, as well as monoclonal antibodies specific for either CD80 or CD86, Perrin et al. demonstrated that B7 costimulation plays a prominent role in determining clinical disease outcome in EAE.

The interaction between B7 and CD28 is one of several costimulatory signaling pathways that appear to be sufficient to trigger the maturation and proliferation of antigen specific T cells. Lack of costimulation, and the concomitant inadequacy of IL-2 production, prevent subsequent proliferation of the T cells and induce a state of non-reactivity termed "anergy". A variety of viruses and tumors may block T cell activation and proliferation, leading to insufficient activity or non-reactivity of the host's immune system to the infected or transformed cells. Among a number of possible T cell disturbances, anergy may be at least partly responsible for the failure of the host to clear the pathogenic or tumorgenic cells.

The use of the B7 protein to mediate anti-tumor immunity has been described in Chen et al., Cell 71:1093-1102 (1992), and Townsend and Allison, Science 259:368 (1993). Schwartz, Cell 71:1065 (1992), reviews the role of CD28, CTLA-4, and B7 in IL-2 production and immunotherapy Harding et al., Nature 356:607-609 (1994) demonstrates that CD28 mediated signaling co-stimulates murine T cells and prevents the induction of anergy in T cell clones. *See also* U.S. Patent Nos. 5,434,131, 5,770,197, and 5,773,253, and International Patent Application Nos. WO 93/00431, WO 95/01994, WO 95/03408, WO 95/24217, and WO 95/33770.

From the foregoing, it was clear that T cells required two types of signals from the antigen-presenting cell (APC) for activation and subsequent differentiation to effector function. First, there is an antigen-specific signals generated by interactions between the TCR on the T-cell and MHC molecules presenting peptides on the APC. Second, there is an antigen-independent signal that is mediated by the interaction of CD28 with embers of the B7 family (B7-1 (CD80) or B7-2 (CD86)). Exactly, where CTLA-4 fits into the milieu of immune responsiveness was initially evasive. Murine CTLA-4 was first identified and cloned by Brunet et al., Nature 328:267-270 (1987), as part of a quest for molecules that are preferentially expressed on cytotoxic T-lymphocytes. Human CTLA-4 was identified and cloned shortly thereafter by Dariavach et al., Eur. J. Immunol. 18:1901-1905 (1988). The murine and human CTLA-4 molecules possess approximately 76% overall sequence homology and approach complete sequence identity in their cytoplasmic domains (Dariavach et al., Eur. J. Immunol. 18:1901-1905 (1988)). CTLA-4 is a member of the immunoglobulin (Ig) superfamily of proteins. The Ig superfamily is a group of proteins that share key structural features of either a variable (V) or constant (C) domain of Ig molecules. Members of the Ig superfamily include, but are not limited to, the immunoglobulins themselves, major histocompatibility complex (MHC) class molecules (i.e., MHC class I and II), and TCR molecules.

In 1991, Linsley et al., J. Exp. Med. 174:561-569 (1991), proposed that CTLA-4 was a second receptor for B7. Similarly, Harper et al., J. Immunol. 147:1037-44 (1991), demonstrated that the CTLA-4 and CD28 molecules are closely related in both mouse and human as to sequence, message expression, gene structure, and chromosomal location. *See also* Balzano et al., Int. J. Cancer Suppl. 7:28-32 (1992) Further evidence of this role arose through functional studies For example, Lenschow et al., Science 257:789-792 (1992), demonstrated that CTLA-4-Ig induced long term survival of pancreatic islet grafts. Freeman et al., Science 262:907-909 (1993), examined the role of CTLA-4 in B7 deficient mice. Examination of the ligands for CTLA-4 is described in Lenschow et al., P.N.A.S. 90:11054-11058 (1993) Linsley et al., Science 257:792-795 (1992), describes immunosuppression *in vivo* by a soluble form of CTLA-4. Linsley et al., J. Exp. Med. 176:1595-604 (1992), prepared antibodies that bound CTLA-4 and that were not cross-reactive with CD28 and concluded that CTLA-4 is coexpressed with CD28 on activated T-lymphocytes and cooperatively regulates T cell adhesion and activation by B7. Kuchroo et al., Cell 80:707-18 (1995), demonstrated that the B7-1 and B7-2 costimulatory molecules differentially activated the Th1/Th2 developmental pathways. Yi-qun et al., Int.Immunol.8:37-44 (1996), demonstrated that there are differential requirements for costimulatory signals from B7 family members by resting versus recently activated memory T cells towards soluble recall antigens. *See also* de Boer et al., Eur. J.Immunol.23:3120-5 (1993).

Several groups proposed alternative or distinct receptor/ligand interactions for CTLA-4 as compared to CD28 and even proposed a third B7 complex that was recognized by a BB1 antibody. *See, for example,* Hathcock et al., Science 262:905-7 (1993), Freeman et al., Science 262:907-9 (1993), Freeman et al., J.Exp.Med.178:2185-92 (1993), Lenschow et al., Proc.Natl.Acad. Sci. U.S.A. 90:11054-8 (1993), Razi-Wolf et. al., Proc. Natl. Acad. Sci. U.S.A. 90:11182-6 (1993), and Boussiotis et al., Proc.Natl.Acad.Sci.U.S.A.90:11059-63 (1993). *But, see,* Freeman et al., J. Immunol.161:2708-15 (1998), who discuss finding that BB1 antibody binds a molecule that is identical to the cell surface form of CD74 and, therefore, the BB1 mAb binds to a protein distinct from B7-1, and this epitope is also present on the B7-1 protein. Thus, this observation required the field to reconsider studies using BB1 mAb in the analysis of CD80 expression and function.

Beginning in 1993 and culminating in 1995, investigators began to further delineate the role of CTLA-4 in T cell stimulation. First, through the use of monoclonal antibodies against CTLA-4, Walunas et al., Immunity 1:405-13 (1994), provided evidence that CTLA-4 can function as a negative regulator of T cell activation. Thereafter, Waterhouse et al., Science 270:985-988 (1995), demonstrated that mice deficient for CTLA-4 accumulated T cell blasts with up-regulated activation markers in their lymph nodes and spleens: The blast cells also infiltrated liver, heart, lung, and pancreas tissue, and amounts of serum immunoglobulin were elevated and their T cells proliferated spontaneously and strongly when stimulated through the T cell receptor, however, they were sensitive to cell death induced by cross-linking of the Fas receptor and by gamma irradiation. Waterhouse et al. concluded that CTLA-4 acts as a negative regulator of T cell activation and is vital for the control of lymphocyte homeostasis. In a comment in the same issue, Allison and Krummel, Science 270:932-933. (1995), discussed the work of Waterhouse et al. as demonstrative that CTLA-4 acts to down-regulate T cell responsiveness or has an inhibitory signaling role in T cell activation and development. Tivol et al., Immunity 3:541-7 (1995), also generated CTLA-4-deficient mice and demonstrated that such mice rapidly develop lymphoproliferative disease with multiorgan lymphocytic infiltration and tissue destruction, with particularly severe myocarditis and pancreatitis. They concluded that CTLA-4 plays a key role in down-regulating T cell activation and maintaining immunologic homeostasis. Also, Kummel and Allison, J. Exp. Med.182:459-65 (1995), further clarified that CD28 and CTLA-4 have opposing effects on the response of T cells to stimulation. They generated an antibody to CTLA-4 and investigated the effects of its binding to CTLA-4 in a system using, highly purified T cells. In their report, they showed that the presence of low levels of B7-2 on freshly explanted T cells can partially inhibit T cell proliferation, and this inhibition was mediated by interactions with CTT.A-4. Cross-linking of CTLA-4 together with the TCR and CD28 strongly inhibits proliferation and IL-2 secretion by T cells. Finally, the results showed that CD28 and CTLA-4 deliver opposing signals that appear to be integrated by the T cell in determining the response to antigen. Thus, they concluded that the outcome of T cell antigen receptor stimulation is regulated by CD28 costimulatory signals, as well as inhibitory signals derived from CTLA-4. *See also* Kummel et al., Int. Immunol. 8:519-23 (1996), and U.S. Patent No. 5,811,097, and International Patent Application No. WO 97/20574.

A variety of additional experiments have been conducted further elucidating the above function of CTLA-4. For example, Walunas et al., J. Exp. Med.183:2541-50 (1996), through the use of anti-CTLA-4 antibodies, suggested that CTLA-4 signaling does not regulate cell survival or responsiveness to IL-2, but does inhibit CD28-dependent IL-2 production. Also, Perrin et al., J. Immunol. 157:1333-6 (1996), demonstrated that anti-CTLA-4 antibodies in experimental allergic encephalomyelitis (EAE), exacerbated the disease and enhanced mortality. Disease exacerbation was associated with enhanced production of the encephalitogenic cytokines TNF-alpha, IFN-gamma and It-2. Thus, they concluded that CTLA-4 regulates the intensity of the autoimmune response in EASE, attenuating inflammatory cytokine production and clinical disease manifestations, *See also* Hurwitz et al., J. Neuroimmunol. 73:57-62 (1997), and Cepero et al., J. Exp. Med.188:199-204 (1998) (an anti-CTLA-4 hairpin ribozyme that specifically abrogates CTLA-4 expression after gene transfer into a murine T-cell models).

In addition, Blair et al., J. Immunol.160:12-5 (1998), assessed the functional effects of a panel of CTLA-4 monoclonal antibodies (mAbs) on resting human CD4+ T cells. Their results demonstrated that some CTLA-4 mAbs could inhibit proliferative responses of resting CD4+ cells and cell cycle transition from G0 to G1. The inhibitory effects of CTLA-4 were evident within , 4 h, at a time when cell surface CTLA-4 expression remained undetectable. Other CTLA-4 mAbs, however, had no detectable inhibitory effects, indicating that binding of mAbs to CTLA-4 alone was not sufficient to mediate down-regulation of T cell responses. Interestingly, while IL-2 production was shut off, inhibitory anti-CTLA-4 mAbs permitted induction and expression of the cell survival gene bcl-X(L). Consistent with this observation, cells remained viable and apoptosis was not detected after CTLA-4 ligation.

In connection with anergy, Perez et al., Immunity 6:411-7 (1997), demonstrated that the induction of T cell anergy was prevented by blocking CTLA-4 and concluded that the outcome of antigen recognition by T cells is determined by the interaction of CD28 or CTLA-4 on the T cells with B7 molecules. Also, Van Parijs et al., J. Exp. Med.186:1119-28 (1997), examined the role of interleukin 12 and costimulators in T cell anergy *in vivo* and found that through inhibiting CTLA-4 engagement during anergy induction, T cell proliferation was blocked, and full Th1 differentiation was not promoted. However, T cells exposed to tolerogenic antigen in the presence of both IL-12 and anti-CTLA-4 antibody were not anergized, and behaved identically to T cells which have encountered immunogenic antigen. These results suggested that two processes contribute to the induction of anergy *in vivo:* CTLA-4 engagement, which leads to a block in the ability of T cells to proliferated, and the absence of a prototypic' inflammatory cytokine, IL-12, which prevents the differentiation of T cells into Th1 effector cells. The combination of IL-12 and anti-CTLA-4 antibody was sufficient to convert a normally tolerogenic stimulus to an immunogenic one.

In connection with infections, McCoy et al., J. Exp. Med. 186:183-7 (1997), demonstrated that anti-CTLA-4 antibodies greatly enhanced and accelerated the T cell immune response to *Nippostrongylus brasiliensis,* resulting in a profound reduction in adult worm numbers and early termination of parasite egg production. *See also* Murphy et al., J. Immunol. 161:4153-4160 (1998) (*Leishmania donovani*)*.*

In connection with cancer, Kwon et al., PNAS USA 94:8099-103 (1997), established a syngeneic murine prostate cancer model and examined two distinct manipulations intended to elicit an antiprostate cancer response through enhanced T cell costimulation: (i) provision of direct costimulation by prostate cancer cells transduced to express the B7-1 ligand and (ii) *in vivo* antibody-mediated blockade of T cell CTLA-4, which prevents T cell down-regulation. It was demonstrated that *in vivo* antibody-mediated blockade of CTLA-4. enhanced antiprostate cancer immune responses. Also, Yang et al., Cancer Res. 57:4036-41 (1997), investigated whether the blockade of the CTLA-4 function leads to enhancement of antitumor T cell responses at various stages of tumor growth. Based on *in vitro* and *in vivo* results they found that CTLA-4 blockade in tumor-bearing individuals enhanced the capacity to generate antitumor T cell responses, but the expression of such an enhancing effect was restricted to early stages of tumor growth in their model. Further, Hurwitz et al., Proc.Natl.Acad. Sci. U.S.A.95:10067-71 (1998), investigated the generation of a T cell-mediated antitumor response that depends on T cell receptor engagement by major histocompatibility complex/antigen as well as CD28 ligation by B7. Certain tumors, such as the SM1 mammary carcinoma, were refractory to anti-CTLA-4 immunotherapy. Thus, through use of a combination of CTLA-4 blockade and a vaccine consisting of granulocyte-macrophage colony-stimulating factor-expressing SM1 cells, regression of parental SM1 tumors was observed, despite the ineffectiveness of either treatment alone. This combination therapy resulted in long-lasting immunity to SM1 and depended on both CD4(+) and CD8(+) T cells. The findings suggested that CTLA-4 blockade acts at the level of a host-derived antigen-presenting cell.

In connection with diabetes, Luhder et al., J. Exp. Med.187:427-32 (1998), injected an anti-CTLA-4 mAb into a TCR-transgenic mouse model of diabetes at different stages of disease. They found that engagement of CTLA-4 at the time when potentially diabetogenic T cells are first activated is a-pivotal event; if engagement is permitted, invasion of the islets occurs, but remains quite innocuous for months. If not, insulitis is much more aggressive, and diabetes quickly ensues.

In connection with vaccine immunization, Horspool et al., J. Immunol. 160:2706-14 (1998), found that intact anti-CTLA-4 mAb but not Fab fragments suppressed the primary humoral response to pCIA/beta gal without affecting recall responses, indicating CTLA-4 activation inhibited Ab production but not T cell priming. Blockade of the ligands for CD28 and CTLA-4, CD80 (B7-1) and CD86 (B7-2), revealed distinct and nonoverlapping function. Blockade of CD80 at initial immunization completely abrogated primary and secondary Ab responses, whereas blockade of CD86 suppressed primary but not secondary responses. Simultaneous blockade of CD80 + CD86 was less effective at suppressing Ab responses than either alone. Enhancement of costimulation via coinjection of B7-expressing plasmids augmented CTL responses but not Ab responses, and without evidence of Th1 to Th2 skewing. These findings suggest complex and distinct roles for CD28, CTLA-4, CD80, and CD86 in T cell costimulation following nucleic acid vaccination.

In connection with allograft rejection, Markees et al., J. Clin. Invest. 101:2446-55 (1998), found in a mouse model of skin allograft rejection that acceptance initially depended on the presence of IFN-gamma, CTLA-4, and CD4(+) T cells. Addition of anti-CTLA-4 or anti-IFN-gamma mAb to the protocol was associated with prompt graft rejection, whereas anti-IL-4 mAb had no effect.

In connection with the role of CTLA-4 in relation to CD28, Fallarino et al., J. Exp. Med. 188:205-10 (1998), generated TCR-transgenic/recombinase activating gene 2-deficient/CD28-wild-type or CD28-deficient mice which were immunized with an antigen-expressing tumor. Primed T cells from both types of mice produced cytokines and proliferated in response to stimulator cells lacking B7-expression. However, whereas the response of CD28+/+ T cells was augmented by costimulation with B7-1, the response of the CD28-/- T cells was strongly inhibited. This inhibition was reversed by monoclonal antibody against B7-1 or CTLA-4. Thus, CTLA-4 can potently inhibit T cell activation in the absence of CD28, indicating that antagonism of a TCR-mediated signal is sufficient to explain the inhibitory effect of CTLA-4. Also, Lin et al., Exp. Med. 188:199-204 (1998), studied rejection of heart allografts in CD28-deficient mice. H-2(q) hearts were transplanted into allogeneic wild-type or CD28-deficient mice (H-2(b)). Graft rejection was delayed in CD28-deficient compared with wild-type mice. Treatment of wild-type recipients with CTLA-4-immunoglobulin (Ig), or with anti-B7-1 plus anti-B7-2 - mAbs significantly prolonged allograft survival. In contrast, treatment of CD28-deficient mice with CTLA-4-Ig, anti-B7-1 plus anti-B7-2 mAbs, or a blocking anti-CTLA-4 mAb induced acceleration of allograft rejection. This increased rate of graft rejection was associated with more severe mononuclear cell infiltration and enhanced levels of IFN-gamma and IL-6 transcripts in donor hearts of untreated wild-type and CTLA-4-Ig- or anti-CTLA-4 mAb-treated CD28-deficient mice. Thus, the negative regulatory role of CTLA-4 extends beyond its potential ability to prevent CD28 activation through ligand competition. Even in the absence of CD28 CTLA-4 plays an inhibitory role in the regulation of allograft rejection.

Also, further characterization of the expression of CTLA-4 has been investigated. For Example, Alegre et al., J. Immunol. 157:4762.70 (1996), proposed that surface CTLA-4 is rapidly internalized, which may explain the low levels of expression generally detected on the cell surface. They concluded that both CD2g and IL-2 play important roles in the up-regulation of CTLA-4 expression. In addition, the cell surface accumulation of CTLA-4 appeared to be primarily regulated by its rapid endocytosis. Also, Castan et al., Immunology 90:265-71 (1997), based on *in situ* immunohistological analyses of the expression of CTLA-4, suggested that germinal center T cells, which were CTLA-4 positive, could be important to immune regulation.

Accordingly, in view of the broad and pivotal role that CTLA-4 appears to possess in immune responsiveness, it would be desirable to generate antibodies to CTLA-4 that can be utilized effectively in immunotherapy. Moreover, it would be desirable to generate antibodies against CTLA-4 that can be utilized in chronic diseases in which repeat administrations of the antibodies are required.

### BRIEF DESCRIPTION OF THE DRAWING FIGURES

Figure 1 provides a series of nucleotide and amino acid sequences of heavy chain and kappa light chain immunoglobulin molecules 4.1.1 (Figure 1A), 4.8.1 (Figure 1B), 4.14.3 (Figure 1C). 6.1.1 (Figure 1D), 3.1.1 (Figure 1D). 4.10.2 (Figure 1F), 2.1.3 (Figure 1G), 4.13.1 (Figure 1H), 11.2.1 (Figur 1I in accordance with the invention), 11.6.1 (Figure 1J), in accordance with the invention 11.7.1 (Figure 1K), 12.3.1.1 (Figure 1L), and 12.9.1.1 (Figure 1M).
Figure 2 provides a sequence alignment between the predicted heavy chain amino acid sequences from the clones 4.1.1, 4.8.1. 4.14.3. 6.1.1, 3.1.1, 4.10.2, 4.13.1, 11.2.1, 11.6.1, 11.7.1, 12.3.1.1, and 12.9.1.1 and the germline DP-50 (3-33) amino acid sequence. Differences between the DP-50 germline sequence and that of the sequence in the clones are indicated in bold. The Figure also shows the positions of the CDR1, CDR2, and CDR3 sequences of the antibodies as shaded.
Figure 3 provides a sequence alignment between the predicted heavy chain amino acid sequence of the clone 2.1.3 and the germline DP-65 (4-31) amino acid sequence. Differences between the DP-65 germline sequence and that of the sequence in the clone are indicated in bold. The Figure also shows the positions of the CDR1, CDR2, and CDR3 sequences of the antibody as underlined.
Figure 4 provides a sequence alignment between the predicted kappa light chain amino acid sequence of the clones 4.1.1, 4.8.1, 4.14.3, 6.1.1, 4.10.2, and 4.13.1 and the germline A27 amino acid sequence. Differences between the A27 germline sequence and that of the sequence in the clone are indicated in bold. The Figure also shows the positions of the CDR1, CDR2, and CDR3 sequences of the antibody as underlined. Apparent deletions in the CDR1s of clones 4.8.1, 4.14.3, and 6.1.1 are indicated with "0s".
Figure 5 provides a sequence alignment between the predicted kappa light chain amino acid sequence of the clones 3.1.1, 11.2.1, 11.6.1, and 11.7.1 and the germline 012 amino acid sequence. Differences between the 012 germline sequence and that of the sequence in the clone are indicated in bold. The Figure also shows the positions of the CDR1, CDR2, and CDR3 sequences of the antibody as underlined.
Figure 6 provides a sequence alignment between the predicted kappa light chain amino acid sequence of the clone 2.1.3 and the germline A10/A26 amino acid sequence. Differences between the A10/A26 germline sequence and that of the sequence in the clone are indicated in bold. The Figure also shows the positions of the CDR1, CDR2, and CDR3 sequences of the antibody as underlined.
Figure 7 provides a sequence alignment between the predicted kappa light chain amino acid sequence of the clone 12.3.1 and the germline A17 amino acid sequence. Differences between the A17 germline sequence and that of the sequence in the clone are indicated in bold. The Figure also shows the positions of the CDR1, CDR2, and CDR3 sequences of the antibody as underlined.
Figure 8 provides a sequence alignment between the predicted kappa light chain amino acid sequence of the clone 12.9.1 and the germline A3/A19 amino acid sequence. Differences between the A3/A19 germline sequence and that of the sequence in the clone are indicated in bold. The Figure also shows the positions of the CDR1, CDR2, and CDR3 sequences of the antibody as underlined.
Figure 9 provides a summary of N-terminal amino acid sequences generated through direct protein sequencing of the heavy and light chains of the antibodies.
Figure 10 provides certain additional characterizing information about certain antibodies. In Figure 10A, data related to clones 3.1.1, 4.1.1, 4.8.1, 4.10.2, 4.14.3, and 6.1.1 is summarized. Data related to concentration, isoelectric focusing (IEF), SDS-PAGE, size exelusion chromatography, liquid chromatography/mass spectroscopy (LCMS), mass spectroscopy (MALDI), light chain N-terminal sequences is provided. Additional detailed information related to IEF is provided in Figure 10B; related to SDS-PAGE is provided in 10C; and SEC of the 4.1.1 antibody in 10D.
Figure 11 shows the expression of B7-1 and B7-2 on Raji cells using anti-CD80-PE and anti-CD86-PE mAbs.
Figure 12 shows the concentration dependent enhancement of IL-2 production in the T cell blast/Raji assay induced by anti-CTLA-4 blocking antibodies (BNI3, 4.1.1, 4.8.1, and 6.1.1).
Figure 13 shows the concentration dependent enhancement of IFN-γ production in the T cell blast/Raji assay induced by anti-CTLA-4 blocking antibodies (BNI3, 4.1.1, 4.8.1, and 6.1.1)(same donor T cells).
Figure 14 shows the mean enhancement of IL-2 production in T cells from 6 donors induced by anti-CTLA-4 blocking antibodies in the T cell blast/Raji assay.
Figure 15 shows the mean enhancement of IFN-γ production in T cells from 6 donors induced by anti-CTLA-4 blocking antibodies in the T cell blast/Raji assay.
Figure 16 shows the enhancement of IL-2 production in hPBMCs from 5 donors induced by anti-CTLA-4 blocking mAbs as measured at 72 hours after stimulation with SEA.
Figure 17 shows the enhancement of IL-2 production in whole blood from 3 donors induced by anti-CTLA-4 blocking mAbs as measured at 72 and 96 hours after stimulation with SEA.
Figure 18 shows the inhibition of tumor growth with an anti-murine CTLA-4 antibody in a murine fibrosarcoma tumor model.
Figure 19 shows enhancement of IL-2 production induced by anti-CTLA-4 antibodies (4.1.1 and 11.2.1) in a 72 hour T blast/Raji and superantigen (whole blood and peripheral blood mononuclear cells from 6 donors) assays.
Figure 20 shows dose dependent enhancement of IL-2 production induced by anti-CTLA-4 antibodies (4.1.1 andi 11.2.1) in a 72 hour T blast/Raji assay.
Figure 21 shows dose dependent enhancement of IL-2 production induced by anti-CTLA-4 antibodies (4.1.1 and 11.2.1) in a 72 hour superantigen whole blood assay stimulated with 100 ng/ml superantigen.

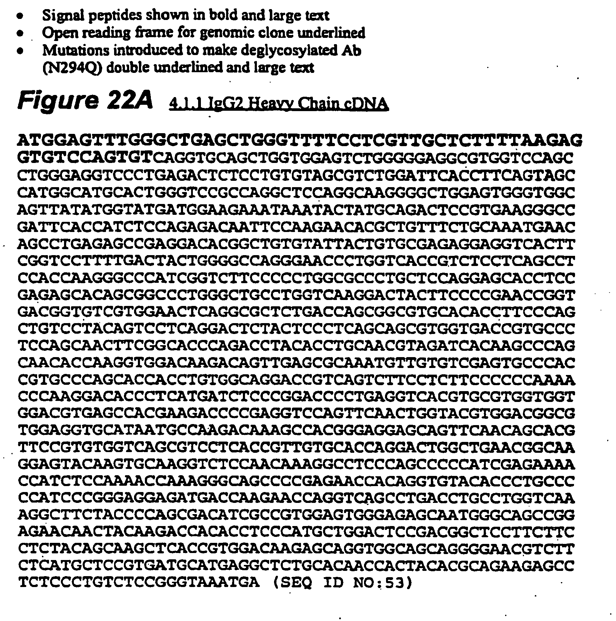

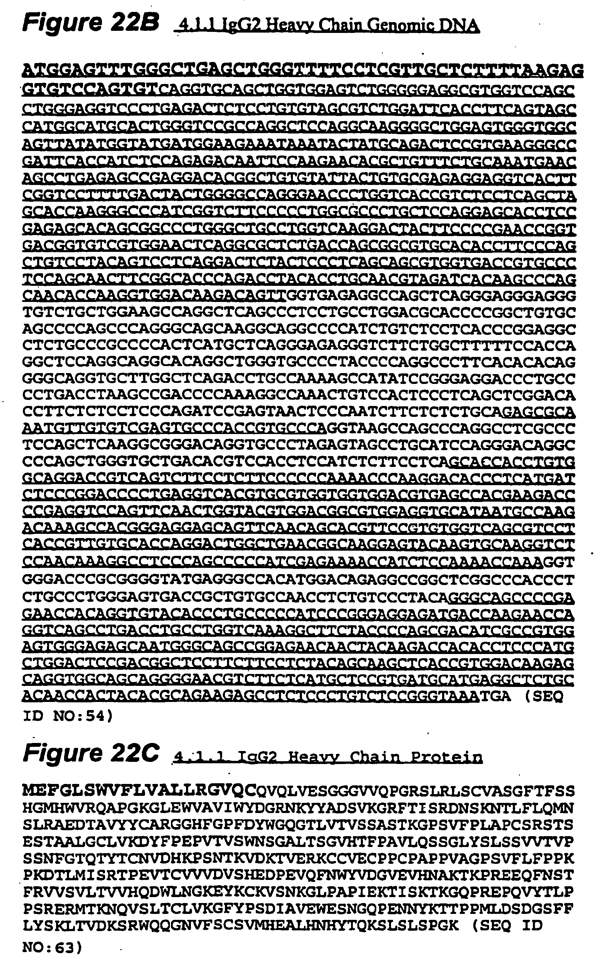

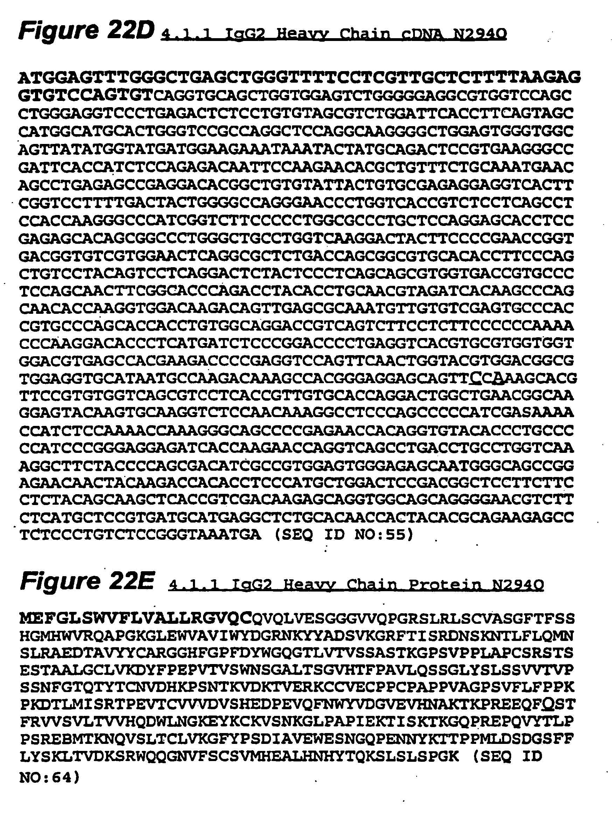

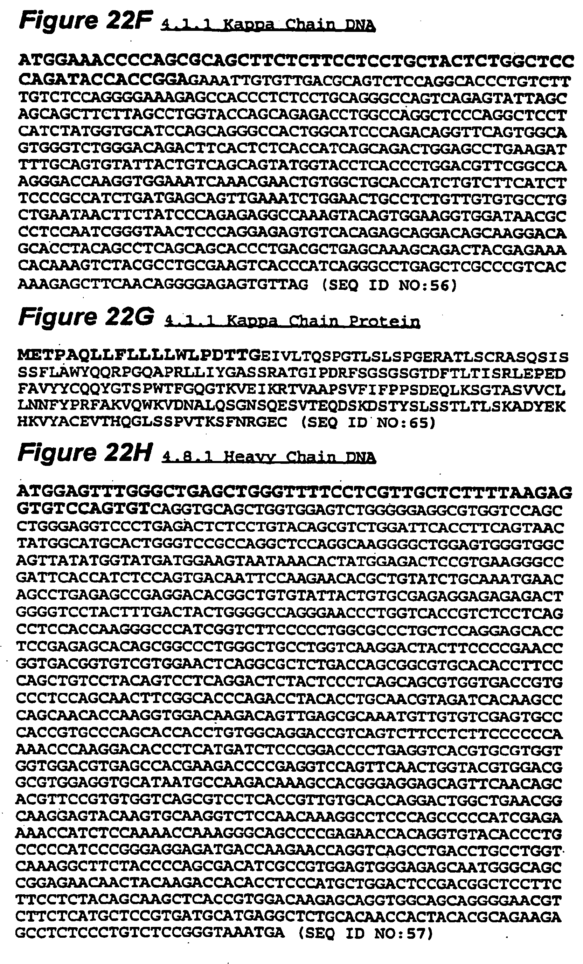

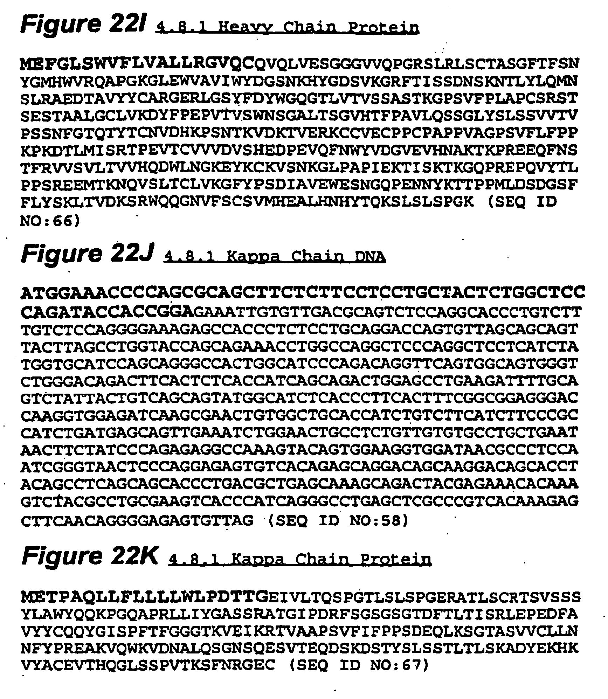

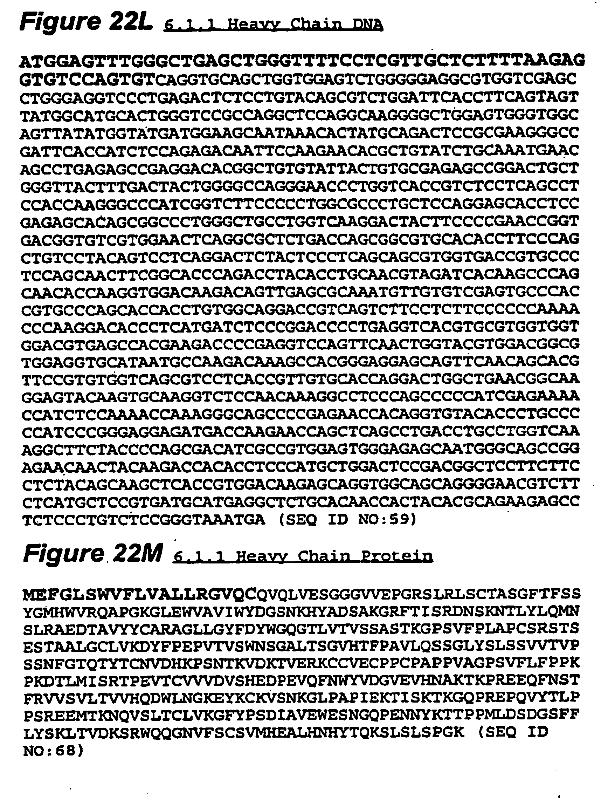

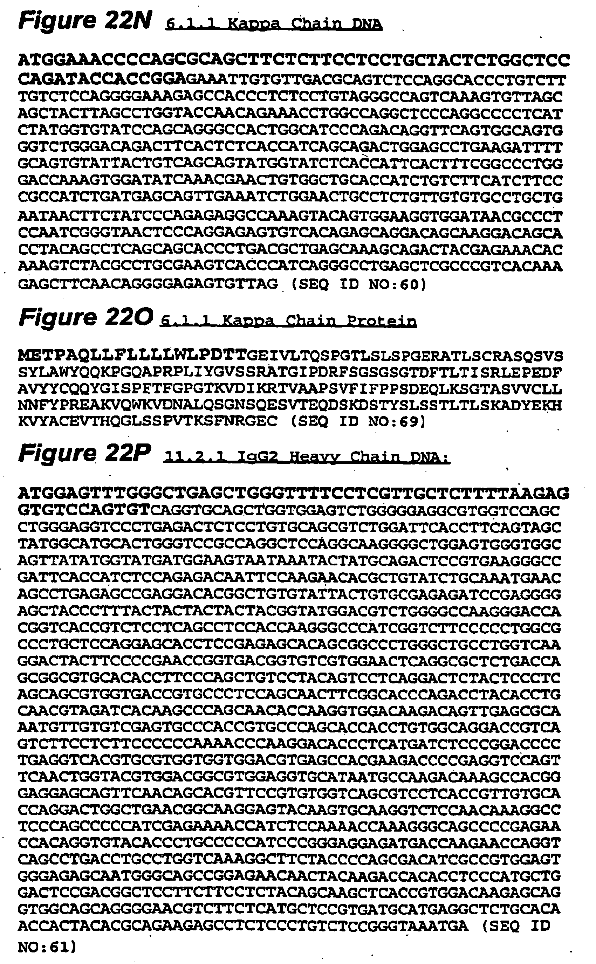

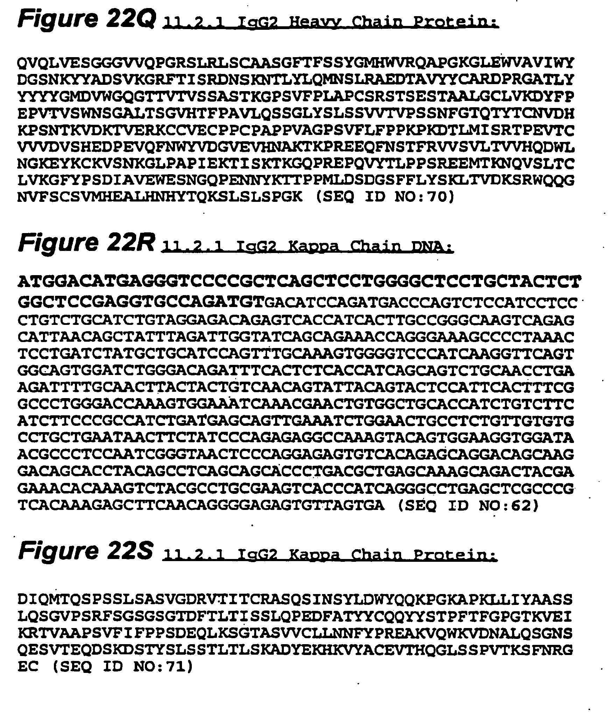

Figure 22 provides a series of additional nucleotide and amino acid sequences of the following anti-CTLA-4 antibody chains: full length 4.1.1 heavy chain (cDNA 22(a), genomic 22(b), and amino acid 22(c)), full length aglycosylated 4.1.1 heavy chain (cDNA 22(d) and amino acid 22(e)), 4.1.1 light chain (cDNA 22(f) and amino acid 22(g)), full length 4.8.1 heavy chain (cDNA 22(h) and amino acid 22(i)), 4.8.1 light chain (cDNA 22(j) and amino acid 22(k)), full length 6.1.1 heavy chain (cDNA 22(l) and amino acid 22(m)), 6.1.1 light chains (cDNA 22(n) and amino acid 22(o)), full length 11.2.1 heavy chain (cDNA 22(p) and amino acid 22(q)), and 11.2.1 light chain (cDNA 22 (r) and amino acid 22(s)).

### SUMMARY OF THE INVENTION

In accordance with the present invention, there is provided an antibody that is capable of binding CTLA-4, which is further characterized in the claims.

### DETAILED DESCRIPTION OF THE PREFERRED EMBODIMENTS

In accordance with the present invention, there are provided fully human monoclonal antibodies against human CTLA-4. Nucleotide sequences encoding and amino acid sequences comprising heavy and light chain immunoglobulin molecules, particularly sequences corresponding to contiguous heavy and light chain sequences from FR1 and CDR1 through CDR3 and FR4, are provided. Hybridomas expressing such immunoglobulin molecules and monoclonal antibodies are also provided.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well know in the art and as described in various general and more specific references that are cited and discussed throughout the present specification. *see e.g.,* Sambrook et. al., Molecular Cloning: A Laboratory Manual (2d ed., Cold Spring Harbor Laboratory Press, Cold Spring Harbor, N.Y. (1989)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The term "isolated polynucleotide" as used herein shall mean a polynucleotide of genomic, cDNA, or synthetic origin or some combination thereof, which by virtue of its origin the "isolated polynucleotide" (1) is not associated with all or a portion of a polynucleotide in which the "isolated polynucleotide" is found in nature, (2) is operably linked to a polynucleotide which it is not linked to in nature, or (3) does not occur in nature as part of a larger sequence.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, e.g. free of murine proteins, (3) is expressed by a cell from a different species, or (4) does not occur in nature.

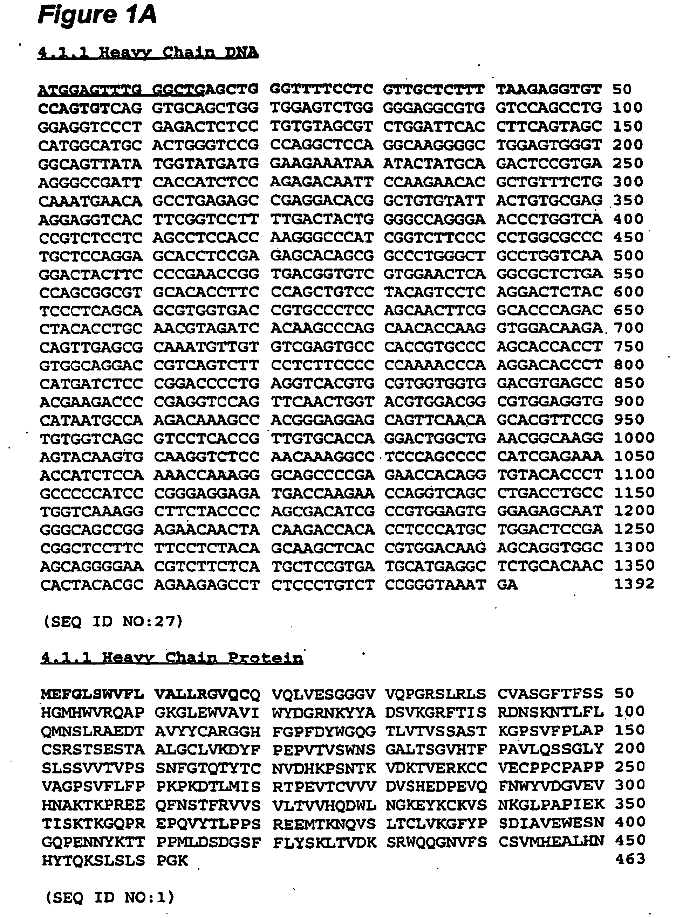

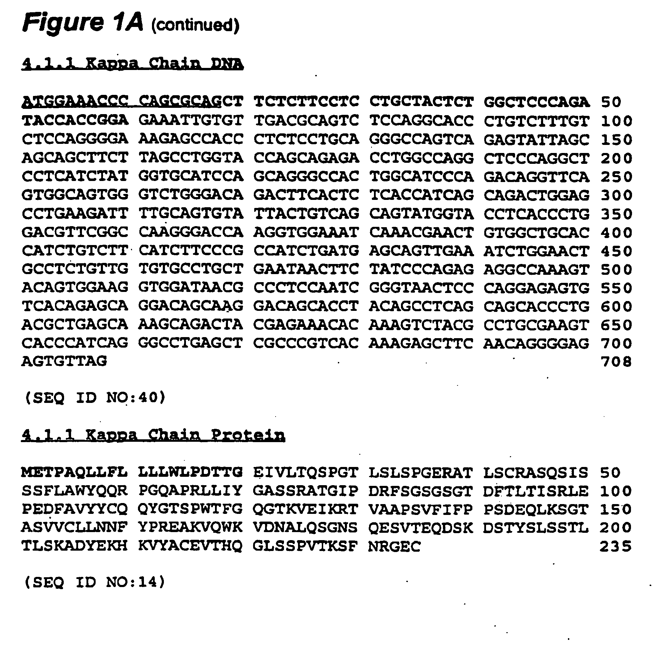

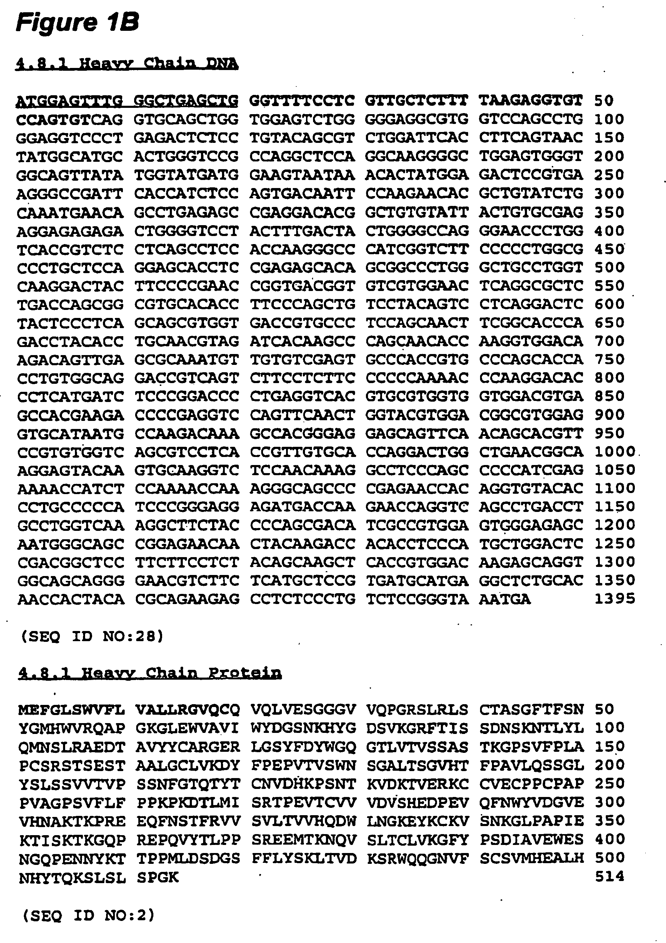

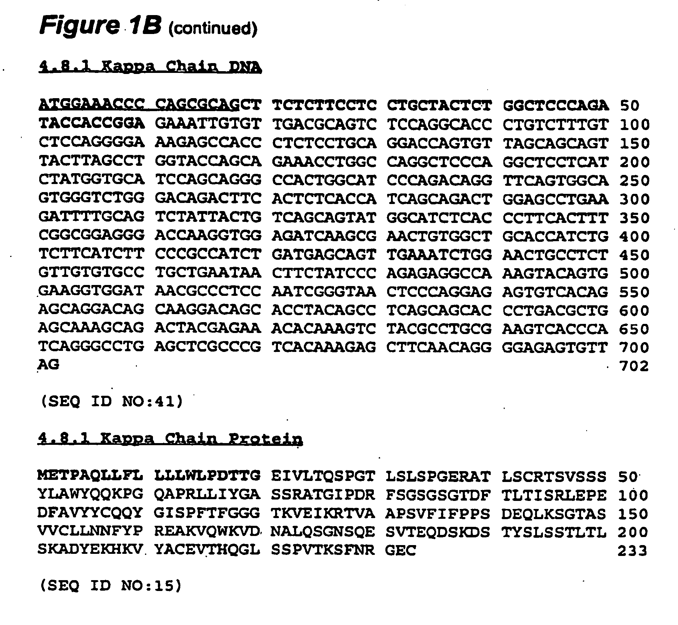

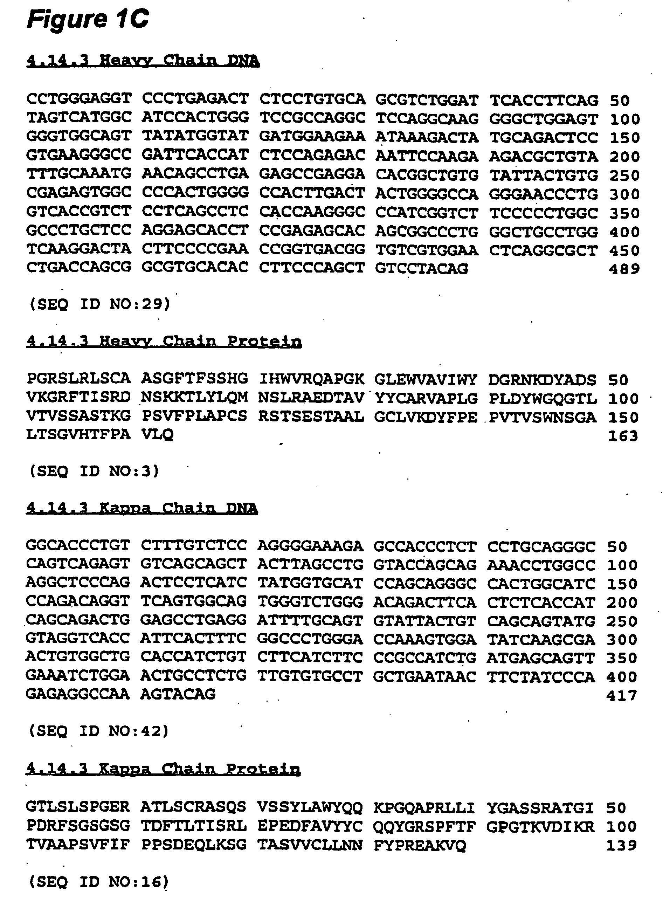

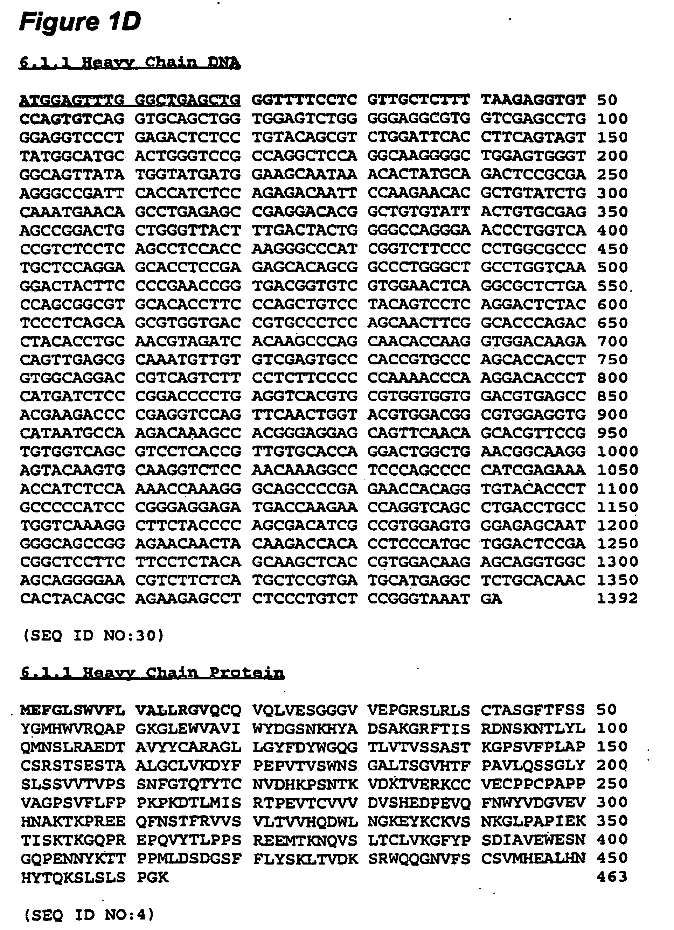

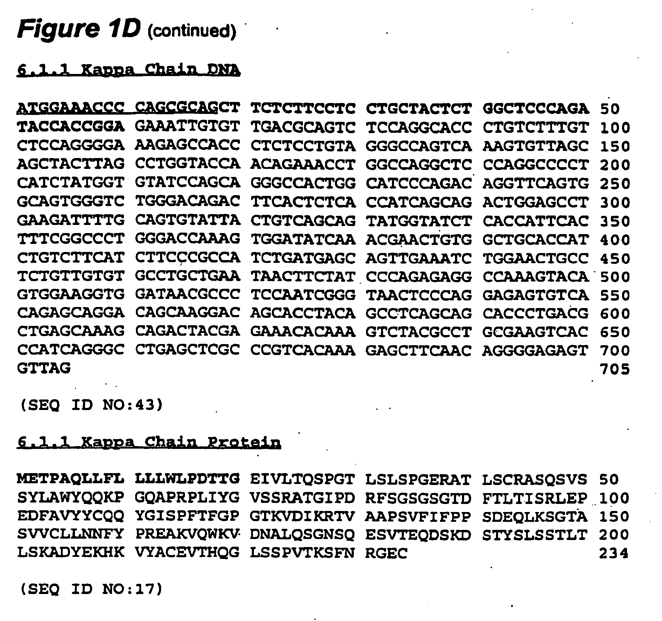

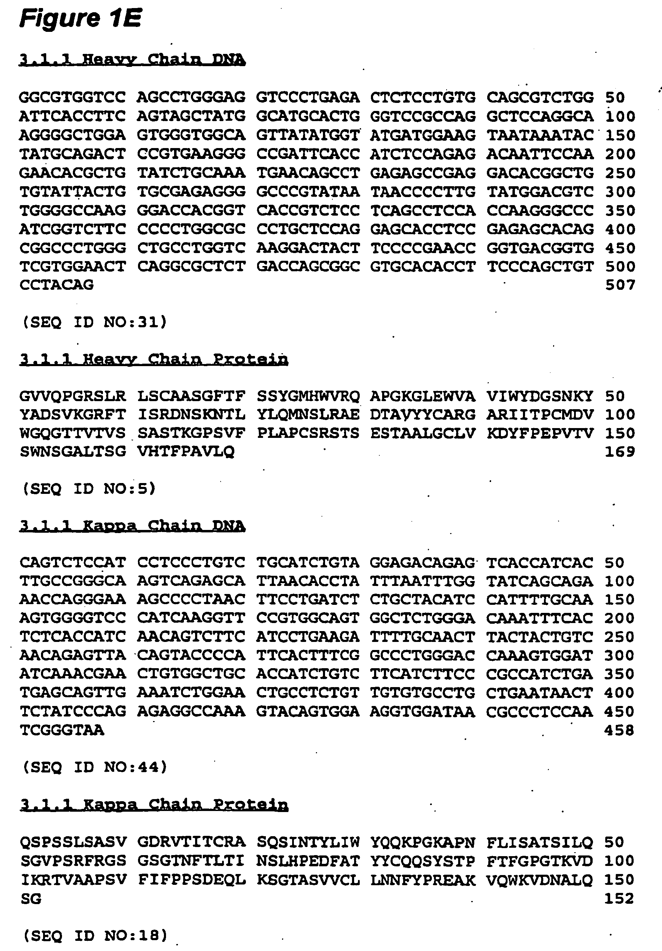

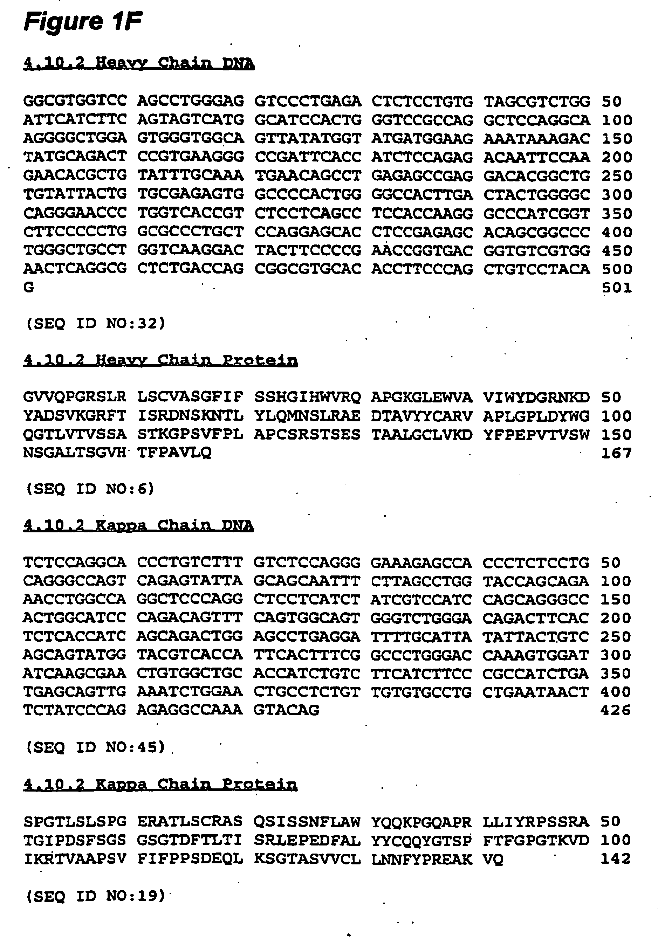

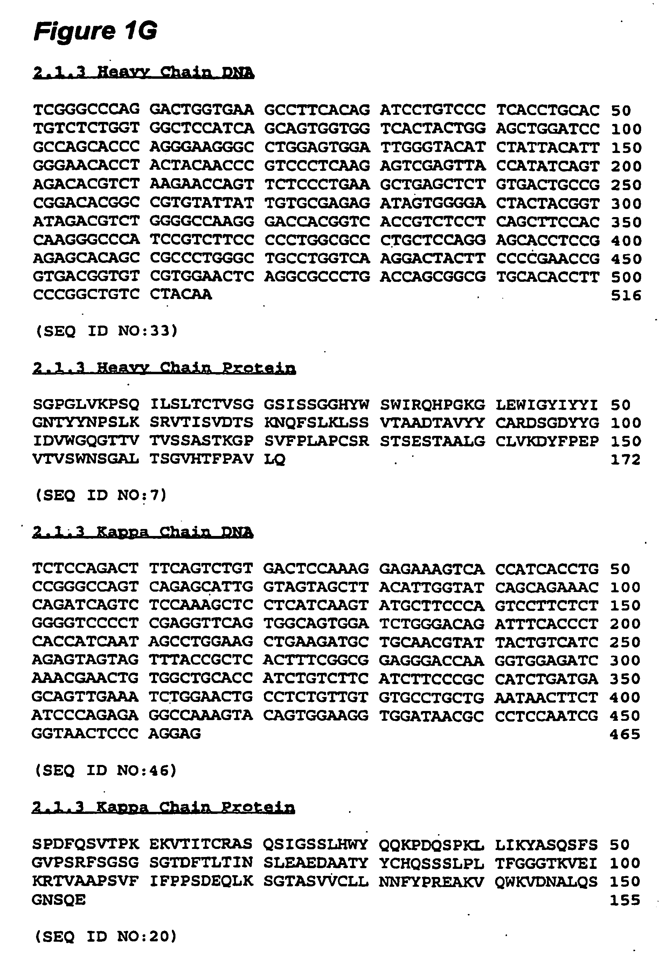

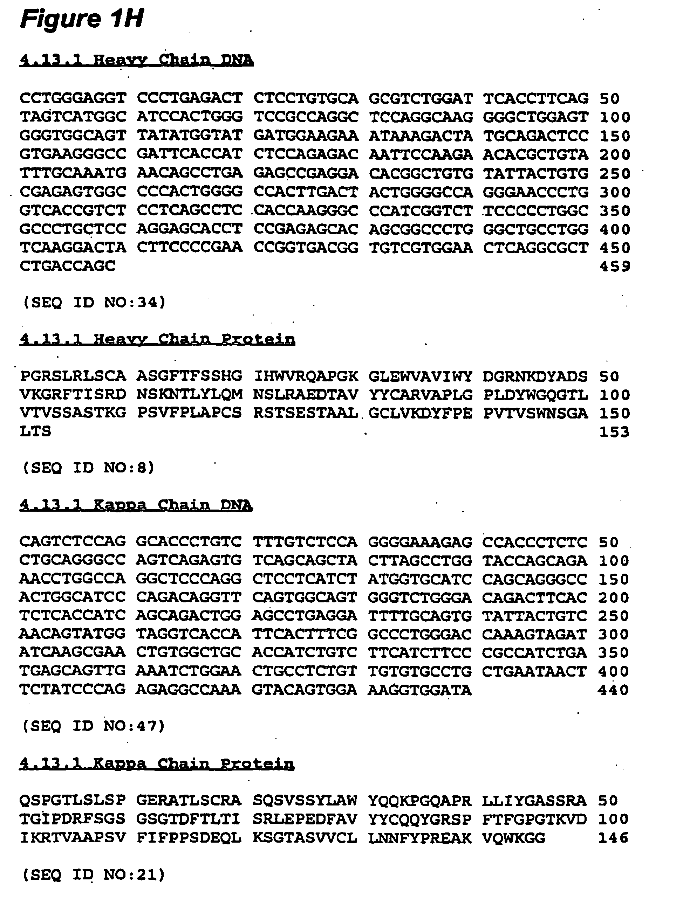

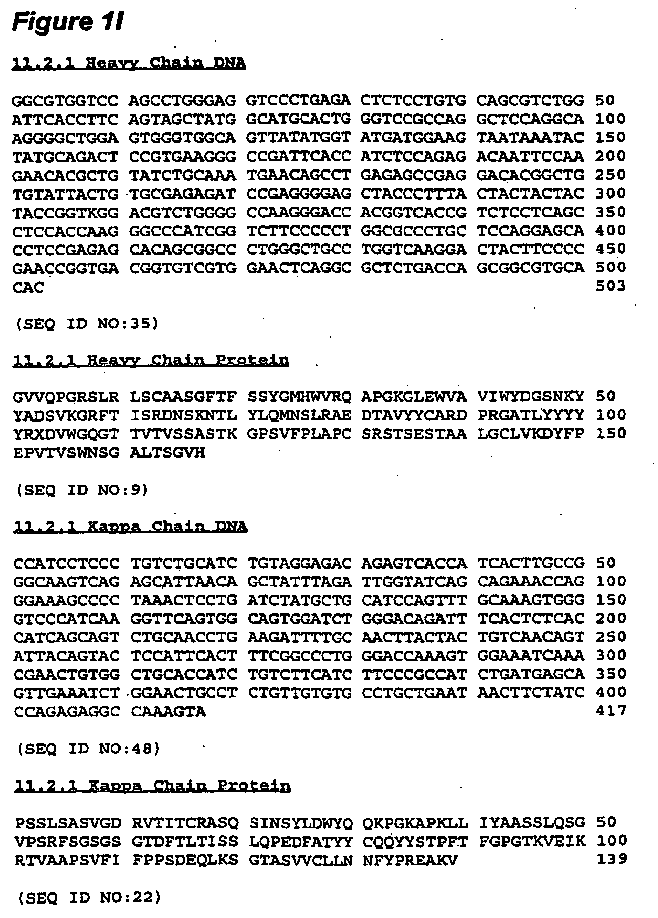

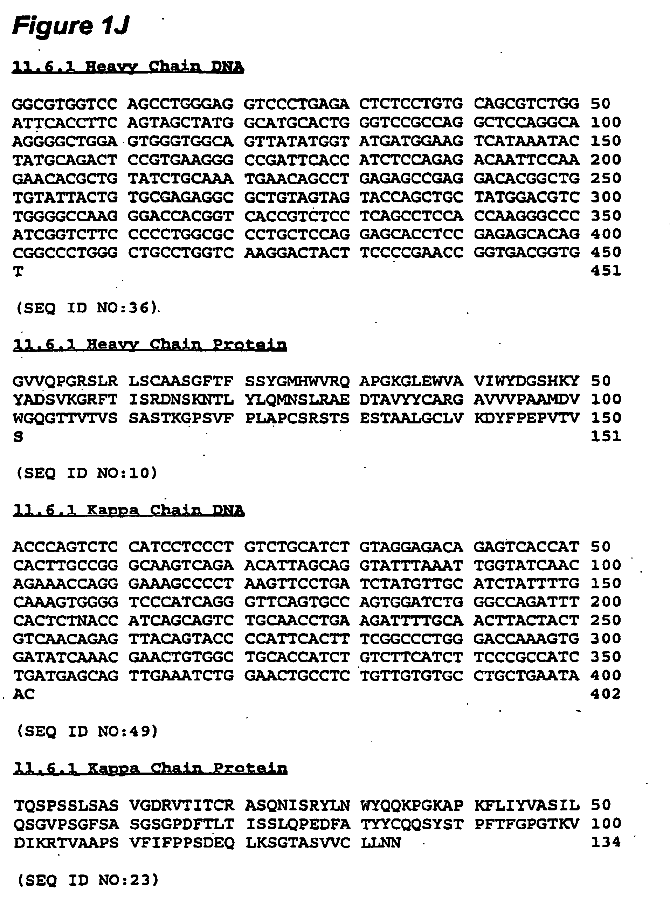

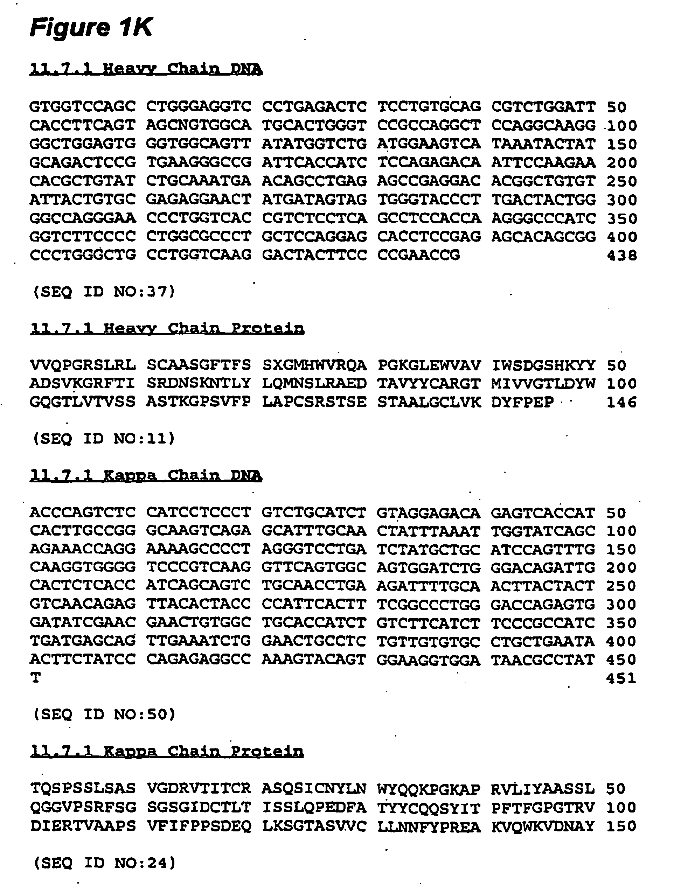

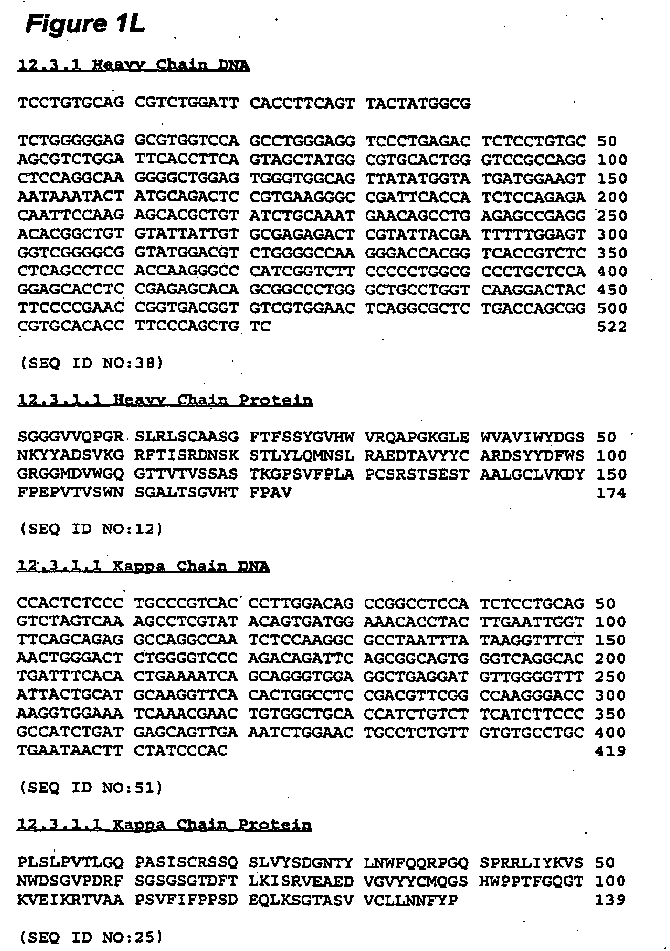

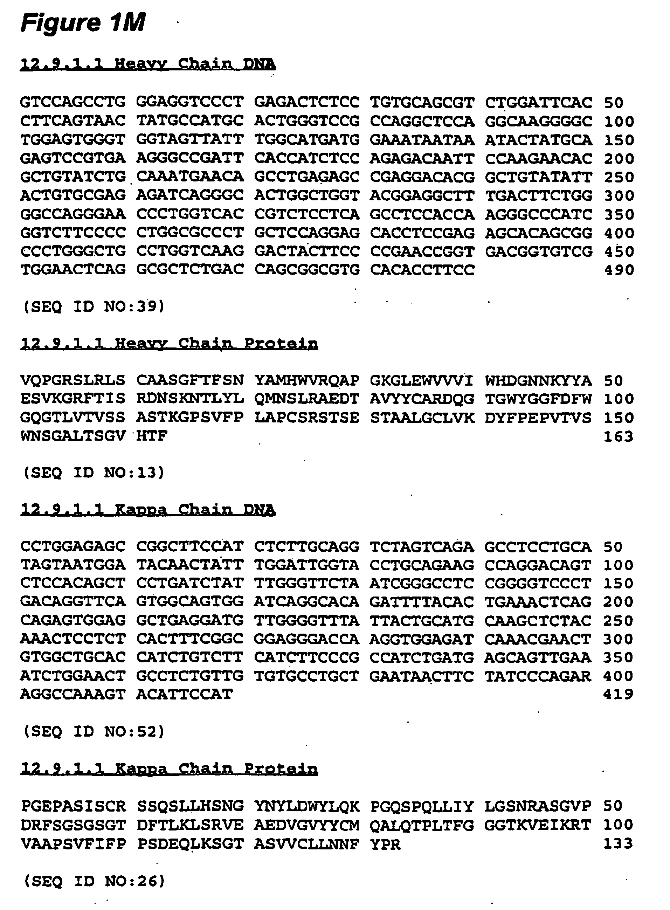

The term "polypeptide", as used herein as a generic term, refers to native protein, fragments, or analogs of a polypeptide sequence. Hence, native protein, fragments, and analogs are species of the polypeptide genus. Preferred polypeptides in accordance with the invention comprise the human heavy chain immunoglobulin molecules and the human kappa light chain immunoglobulin molecules represented in Figure 1, as well as antibody molecules formed by combinations comprising the heavy chain immunoglobulin molecules with light chain immunoglobulin molecules, such as the kappa light chain immunoglobulin molecules, and vice versa, as well as fragments and analogs thereof.

The term "naturally-occurring", as used herein as applied to an object, refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to the positions of components so described that are in a relationship permitting them to function in their intended manner. A control sequence "operably linked" to a coding sequence is ligated in such a way that expression of the coding sequence is achieved under conditions compatible with the control sequence.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include a promoter, ribosomal binding site, and transcription termination sequence; in eukaryotes, generally, such control sequences include promoters and a transcription termination sequence. The term "control sequences" is intended to include, at a minimum, all components whose presence is essential for expression and processing, and can also include additional components whose presence is advantageous, for example, leader sequences and fusion partner sequences.

The term "polynucleotide" as referred fo herein means a polymeric form of nucleotide of at least 10 bases in length, either ribonucleotides or deoxynucleotides or a modified form of either type of nucleotide. The term includes single and double stranded forms of DNA.

The term "oligonucleotide" referred to herein includes naturally-occurring, and modified nucleotides linked together by naturally-occurring, and non-naturally-occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, e.g. for probes; although oligonucleotides may be double stranded, e.g. for use in the construction of a gene mutant. Oligonucleotides can be either sense or antisense oligonucleotides.

The term "naturally-occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phospheroselenoate, phosphorodiselenoate, phosphoroanilothioate, phosphoraniladate, phosphoroamidate, and the like. *See e.g.,* LaPlanche et al., Nucl. Acids Res. 14:9081 (1986); Stec et al., J. Am. Chem. Soc. 106:6077 (1984); Stein et al., Nucl. Acids Res. 16:3209 (1988); Zon et al., Anti-Cancer Drug Design 6:539 (1991); Zon et al., Oligonucleotides and Analogues: A Practical Approach, pp. 87-108 (F. Eckstein, Ed., Oxford University Press, Oxford England (1991)); Stec et al., U.S. Patent No. 5,151,510; Uhlmann and Peyman, Chemical Reviews 90:543 (1990), the disclosures of which are hereby incorporated by reference. An oligonucleotide can include a label for detection, if desired.

The term "selectively hybridize" referred to herein means to detectably and specifically bind. Polynucleotides, oligonucleotides and fragments thereof in accordance with the invention selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the invention and a nucleic acid sequence of interest will be at least 80%, and more typically with preferably increasing homologies of at least 85%, 90%, 95%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotide or amino acid sequences: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, frequently at least 24 nucleotides or 8 amino acids in length, and often at least 48 nucleotide or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences; sequence comparisons between two (or more) molecules are typically performed by comparing sequences of the two molecules over a "comparison window" to identify and compare local regions of sequence similarity. A "comparison window", as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman, Adv. Appl. Math. 2:482 (1981), by the homology alignment algorithm of Needleman and Wunsch, J. Mol. Biol. 48:443 (1970), by the search for similarity method of person and Lipman, Proc. Natl. Acad. Sci. (U.S.A.) 85:2444 (1988), by computerized implementations of these algorithms (GAP, BESTFIT, FASTA, and TFASTA in the Wisconsin Genetics Software Package Release 7.0 (Genetics Computer Group, 575 Science Dr., Madison, Wis.), Geneworks, or MacVector software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The term "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity, as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See:*Immunology - A Synthesis (2nd Edition, E.S. Golub and D.R. Gren, Eds., Sinauer Associates, Sunderland, Mass. (1991)), which is incorporated herein by reference. Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for polypeptides of the present invention. Examples of unconventional amino acids include: 4-hydroxyproline, γ -carboxyglutamate, ε-N,N,N-trimethyllysine; ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino-terminal direction and the righthand direction is the carboxy-terminal direction, in accordance with standard usage and convention.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated as being encompassed by the present invention, providing that the variations in the amino acid sequence maintain at least 90%. In particular, conservative amino acid replacements are contemplated. Conservative replacement are those that take place within a family of amino acids that are related in their side chains. Genetically encoded amino acids are generally divided into families: (1) acidic=asparate, glutamate; (2) basic=lysine, arginine, histidine; (3) nonpolar=alaninc. valine, leucine, isoleucine, proline, phenylalanine, methionine, trypiophan: and (4) uncharged polar=glycine, asparagine, glutamine, cysteine, serine, threonine, tyrosine. More preferred families are: serine and threonine are an aliphatic=hydroxy family; asparagine and glutamine are an amide-containing family; alanine, valine, leucine and isoleucine are an aliphatic family: and phenylalanine, tryptophan, and tyrosine are an aromatic family. For example, it is reasonable to expect that an isolated replacement of a leucine with an isoleucine or valine, an aspartate with a glutamate, a threonine with a serine, or a similar of an amino acid with a structurally related amino acid will not have a major effect on the binding or properties of the resulting molecule, especially if the replacement does not involve an amino acid within a framework sites Whether an amino acid change results in a functional peptide can readily be determined by assaying the specific activity of the polypeptide derivative- Assay are described in detail herein. Fragments of antibodies or immunoglobulin molecules can be readily prepared by those of ordinary skill in the art Preferred amino- and carboxy-termini of fragments or analogs occur near boundaries of functional domains. Structural and functional domains can be identified by comparison of the nucleotide and/or amino acid sequence data to public our proprietary sequence databases. Preferably, computerize comparison methods are used to identify sequence motifs or predicted protein conformation domains that occur in other proteins of known structure and/or function. Methods to identify protein sequences that fold into a known three-dimensional structure are known. Bowie et al., Science 253:164 (1991). Thus, the foregoing examples demonstrate that those of skill in the art can recognize sequence motifs and structural conformations that may be used to define structural and functional domains in accordance with the invention.

Preferred amino acid substitution are those which: (I) reduce susceptibility to proteolysis, (2) reduce susceptibility to oxidation. (3) alters binding affinity for forming protein complexes, (4) alter binding affinities, and (4) confer or modify other physicochemical or functional properties of such analogs. Analogs can include various muteins of a sequence other than the naturally-occurring peptide sequence, For example, single or multiple amino acid substitutions (preferably conservative amino acid substitutions) may be made in the naturally-occurring sequence (preferably in the portion of the polypeptide outside the domain(s) forming intermolecular contacts). A. conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed. W. H. Freeman and Company, New York. (1984)); Introduction to Protein Structure (C: Branden and J. Tooze, eds., Garland Publishing, New York, N.Y. (1991)): and. Thornton et al., Nature 354:105 (1991), which are each incorporated herein by reference.

The term polypeptide fragment" as used herein refers to a polypeptide that has an ammo-terminal and/or earboxy-tenninal deletion, but where the remaining amino acid sequence is identical to the corresponding positions in the naturally-occumng sequence deduced, for example, from a full-length DNA sequence. Fragments typically are at least 5, 6, 8 or 10 amino acids long, preferably at least 14 amino acids long, more preferably at least 20 amino acids long, usually at least 50 amino acids long; and even more preferably at least 70 amino acids long.

"Antibody" or "antibody peptide(s)" refer to an intact antibody, or a binding fragment thereof that competes with the intact antibody for specific binding. Binding fragments are produced by recombinant RNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody substantially inhibits adhesion of a receptor to a counter-receptor when an excess of antibody reduces the quantity of receptor bound to counter-receptor by at least about 20%, 40%, 60% or 80%, and more usually greater than about 85% (as measured in an *in vitro* competitive binding assay).

The term "epitope" includes any protein determinant capable of specific binding to an immunoglobulin or T cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is ≤1 µM, preferably ≤100 nM and most preferably ≤ 10 nM.

The term "agent" is used herein to denote a chemical compound, a mixture of chemical compounds, a biological macromolecule, or an extract made from biological materials.

As used herein, the terms "label" or "labeled" refer to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). In certain situations, the label or marker can also be therapeutic. Various methods of labeling polypeptides and glycoproteins are known in the art and may be used. Examples of labels for polypeptides include, but are not limited to, the following: radioisotopes our radionuclides (*e.g*., ³H, ¹⁴C, ¹⁵N, ³⁵S, ⁹⁰Y ⁹⁹Tc, ^{III}In ¹²⁵I, ¹³¹I), fluorescent labels (*e.g.,* FITC, rhodamine, lanthanide phosphor), enzymatic labels (*e.g*., horseradish peroxidase, β-galactosidase, luciferase, alkaline phosphatase), chemiluminescent labels, biotinyl groups, or predetermined polypeptide epitopes, recognized by a secondary reporter (e.g., leucine zipper pair sequences, binding sites for secondary antibodies, metal binding domains, epitope tags). In some embodiments, label are attached by spacer arms of various lengths to reduce potential steric hindrance.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of chemical Terms (Parker, S., Ed., McGraw-Hill, San Francisco (1985)), incorporated herein by reference).

The term "antineoplastic agent" is used herein to refer to agents that have the functional property of inhibiting the development or progression of a neoplasm in a human, particularly a malignant (cancerous) lesion, such a carcinoma, sarcoma, lymphoma; or leukemia. Inhibition of metastasis is frequently a property of antineoplastic agents.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all, macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

The term patient includes human and veterinary subjects.

### Antibody Structure

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 1.00 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12. or more amino acids, with the heavy chains also including a "D" region of about 10 more amino acids. *See generally,* Fundamental Immunology, Ch. 7 (Paul, W., ed., 2nd ed. Raven Press, N.Y. (1989)) (incorporated by reference in its entirety for all purposes). The variable regions of each light/heavy chain pair form the antibody binding site.

Thus, an intact IgG antibody has two binding sites. Except in bifunctional or bispecific antibodies, the two binding sites are the same.

The chains all exhibit the same general structure of relatively conserved framework regions (FR) joined by three hyper variable regions, also called complementarity determining regions or CDRs. The CDRs from the two chains of each pair are aligned by the framework regions, enabling binding to a specific epitope. From N-tenninal to C-terminal, both light and heavy chains comprise the domains FR1, CDR1, FR2, CDR2, FR3, CDR3 and For4. The assignment of amino acids to each domain is in accordance with the definitions of Kabat, Sequences of Proteins of Immunological Interest (National Institutes of Bethesda, MD (1987 and 1991)), or Chothia & Lesk, J. Mol. Biol. 196:901-917 (1987); Chothia et al., Nature 342:878-883 (1989).

A bispecific or bifunctional antibody is an artificial hybrid antibody having two different heavy/light chain pairs and two different binding sites. Bispecific antibodies can be produced by a variety of methods including fusion of hybridomas or linking of Fab' fragments. *See, e.g.,* Songsivilai & Lachmann, Clin. Exp. Immunol. 79: 315-321 (1990); Kostelny et al., J. Immunol. 148:1547-1553 (1992). In addition, bispecific antibodies may be formed as "diabodies" (Holliger et al., '"Diabodies': Small bivalent and bispecific antibody fragments", PNAS USA 90:6444-6448 (1993)) or "Janusins" (Traunecker et al., "Bispecific single chain molecules (Janusins) target cytotoxic lymphocytes on HIV infected cells", EMBO J. 10:3655-3659 (1991); and Traunecker et al. "Janusin: New molecular design for bispecific reagents", Int. J. Cancer, Suppl. 7:51-52 (1992)). Production of bispecific antibodies can be a relatively labor intensive prowess compared with production of conventional antibodies and yields and degree of purity are generally lower for bispecific antibodies. Bispecific antibodies do not exist in the form of fragments having a single binding site (e.g., Fab, Fab', and Fv).

### Human Antibodies and Humanization of Antibodies

Human .antibodies avoid certain of the problems associated with antibodies that possess murine or rat variable and/or constant regions. The presence of such murine or rat derived proteins can lead to the rapid clearance of the antibodies or can lead to the generation of an immune response against the antibody by a patient. In order to avoid the utilization of murine or rat derived antibodies, it has been postulated that one can, develop humanizes antibodies or generate fully human antibodies through the introduction of human antibody function into a rodent so that the rodent would produce antibodies having fully human sequence.

### Human Antibodies

The ability to clone and reconstruct megabase-sized human loci in YACs and to introduce them into the mouse germline provides a powerful approach to elucidating the functional components of very large or crudely mapped loci as well as generating useful models of human disease. Furthermore, the utilization of such technology for substitution of mouse loci with their human equivalents could provide unique insights into the expression and regulation of human gene products during development, their communication with other systems, and their involvement in disease induction and progression

An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B cell development. Furthermore, such a strategy could provide an ideal source for production of fully human monoclonal antibodies (Mabs) an important milestone towards fulfilling the promise of antibody therapy in human disease. Fully human-antibodies are expected to minimize the immunogenic and allergic responses intrinsic to mouse or mouse-detivatized Mabs and thus to increase the efficacy and safety of the administered antibodies. The use of fully human antibodies can be expected to provide a substantial advantage in the treatment of chronic and recurring human diseases, such as inflammation, autoimmunity, and cancer, which require repeated antibody administrations.

One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against, any antigen of interest, including human antigens. Using the hybridoma technology, antigen-specific human Mabs with the desired specificity could be readily produced and selected.

This general strategy was demonstrated in connection with our generation of the first XenoMouse^{™} strains, as published in 1994. *See*, Green et al., Nature Genetics 7:13-21 (1994). The XenoMouse^{™} strains were engineered with yeast artificial chromosomes (YACs) containing 245 kb and 190 kb-sized germline configuration fragments of the human heavy chain locus and kappa light chain locus, respectively, which contained core variable and constant region sequences. *Id*. The human Ig containing YACs proved to be compatible with the mouse system for both rearrangement and expression of antibodies and were capable of substituting for the inactivated mouse Ig genes . This was demonstrated by their ability to induce B cell development, to produce an adult-like human repertoire of fully human antibodies, and to generate antigen-specific human Mabs. These results also suggested that introduction of larger portions of the human Ig loci containing greater numbers of V genes, additional regulatory elements, and human Ig Constant regions might recapitulate substantially the full repertoire that is characteristic of the human humoral response to infection and immunization. The work of Green et al. was recently extended to the introduction of greater than approximately 80% of the human antibody repertoire through introduction of megabase-sized; germline configuration YAC fragments of the human heavy chain loci and kappa light chain loci, respectively, to produce XenoMouse^{™} mice. *See*, Mendez et al., Nature Genetics 15:146-156 (1997), Green and Jakobovits, J. Exp. Med. 188:483-495 (1998); and U.S. Patent Application Serial No. 08/759,620, filed December 3, 1996, the disclosures of which are hereby incorporated by reference.

Such approach is further discussed and delineated in U.S. Patent Application Serial Nos: 07/466,008, filed January 12,1990, 07/610,515, filed November 8, 1990, 07/919,297, filed July 24, 1992, 07/922,649, filed July 30, 1992, 08/031,801, filed March 15, 1993, 08/112,848, filed August 27, 1993, 08/234,145, filed april 28, 1994, 08/376,279, filed January 20, 1995, 08/430, 938, filed April 27, 1995, 08/464,584, filed June 5,1995, 08/464,582, filed June 5, 1995, 08/463,191, filed June 5, 1995, 08/462,837, filed June 5, 1995, 08/486,853, filed June 5, 1995, 08/486,857, filed June 5, 1995, 08/486,859. filed June 5, 1995, 08/462,513, filed June 5, 1995, 08/724,752, filed October 2, 1996, and 08/759,620, filed December 3, 1996. *See also* Mendez et al., Nature Genetics 15:146-156 (1997), and Green and Jakobovits, J. Exp. Med. 188:483-495 (1998). *See also* European Patent No. EP 0 463 151 B1, grant published June 12, 1996, International Patent Application No., WO 94/02602, published February 3, 1994, International Patent Application No., WO 96/34096, published October 31, 1996, and WO 98/24893, published June 11, 1998. The disclosures of each of the above-cited patents, applications, and references are hereby incorporated by reference in their entirety.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V_{H} genes, one or more D_{H} genes, one or more J_{H-} genes, a mu constant region, and a second constant region (preferably a gamma constant region) are formed into a construct for insertion into an animal. This approach is described in U.S. Patent No. 5,545,807 to Surani et al.; and U.S. Patent Nos. 5,545,806, 5,625,825, 5,625,126, 5,633;425, - 5,661,016, 5,770,429, 5,789,650, and 5,814,318 each to Lonberg and Kay, U.S. Patent No. 5,591,669 to Krimpenfort and Berns; U.S. Patent Nos. 5,612,205, 5,721,367, 5,789,215 to Bems et al.; and U.S. Patent No. 5,643,763 to Choi and Dunn, and GenPharm International U.S. Patent Application Serial Nos. 07/574,748, filed August 29, 1990, 07/575,962, filed August 31, 1990, 07/810,279, filed December 17, 1991, 07/853,408, filed March 18, 1992, 07/904,068, filed June 23, 1992, 07/990,860, filed December 16, 1992, 08/053,131, filed April 26, 1993, 08/096,762, filed July 22, 1993, 08/155,301, filed November 18, 1993, 08/161,739, filed December 3, 1993, 08/165,699, filed December 10, 1993, and 08/209,741, filed March 9, 1994, the disclosures of which are hereby incorporated by reference. *See also* European Patent No. 0 546 073 B1; and International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884, the disclosures of which are hereby incorporated by reference in their entirety. *See further* Taylor et al., 1992; Chen et al., 1993; Tuaillon et al., 1993; Choi et al., 1993; Lonberg et al., 1994; Taylor et al., 1994; Tuaillon et al., 1995; and Fishwild et al., 1996, the disclosures of which are hereby incorporated by reference in their entirety.

The inventors of Surani et al., cited above and assigned to the Medical Research Counsel (the "MRC"), produced a transgenic mouse possessing an Ig locus through use of the minilocus approach. The inventors on the GenPharm International work, cited above, Lonberg an Kay, hollowing the lead of the present inventors, proposed inactivation of the endogenous mouse Ig locus coupled with substantial duplication of the Surani et al. work.

An advantage of the minilocus approach is the rapidity with which constructs including portions of the Ig locus can be generated and introduced into animas. Commensurately, however, a significant disadvantage of the minilocus approach is that, in theory, insufficient diversity is introduced through the inclusion of small numbers of V, D, and J genes. Indeed, the published work appears to support this concern. B cell development and antibody production of animals produced through use of the minilocus approach appear stunted. Therefore, research surrounding the present invention has consistently been directed towards the introduction of large portions of the Ig locus in order to achieve greater diversity and in an effort to reconstitute the immune repertoire of the animals.

Human anti-mouse antibody (HAMA) responses have led the industry to prepare chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. Thus, in would be desirable to provide fully human antibodies against CTLA-4 in order to vitiate concerns and/or effects of HAMA or HACA response.

### Humanization and Display Technologies

As was discussed above in connection with human antibody generation, there are advantages to producing antibodies with reduced immunogenicity. To a degree, this can be accomplished in convection with techniques of humanization and display techniques using appropriate libraries. It will be appreciated that murine antibodies or antibodies from other species can be humanized or primatized using techniques well known in the art. *See e.g*., Winter and Harris, Immunol Today 14:43-46 (1993), and Wright et al., Crit. Reviews in Immunol. 12: 125-168. (1992). The antibody of interest may be engineered by recombinant DNA techniques to substitute the CH1, CH2, CH3, hinge domains, and/or the framework domain with the corresponding human sequence (*see* WO 92/02190, and U.S. Patent Nos. 5,530,101, 5,585,089, 5,693,761, 5,693,792, 5,714,350, and 5,777,085). Also, the use of Ig cDNA for construction of chimeric immunoglobulin genes is known in the art (Liu et al., P.N.A.S. 84:3439 (1987), and J.Immunol.139:3521 (1987)). mRNA is isolated from a hybridoma or other cell producing the antibody and used to produce cDNA. The cDNA of interest may be amplified by the polymerase chain reaction using specific primers (U.S. Pat. Nos. 4,683,195 and 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991), Sequences of Proteins of Immunological Interest, N.I.H. Publication No. 91-3242. Human C region genes are readily available from known clones. The choice of isotype will be guided by the desired effector functions, such as complement fixation, or activity in antibody-dependent cellular cytotoxicity. Preferred isotypes are IgG1, IgG2, IgG3 and IgG4. Particularly preferred isotypes for antibodies of the invention are IgG2 and IgG4. Either of the humain light chain constant regions, kappa or lambda, may be used. The chimeric, humanized antibody is then expressed by conventional methods.

Antibody fragments, such as Fv, F(ab')₂ and Fab may be prepared by cleavage of the intact protein, e.g., by protease or chemical cleavage. Alternatively, a truncated gene is designed. For example, a chimeric gene encoding a portion of the F(ab')₂ fragment would include DNA sequences encoding the CH1 domain and hinge region of the H chain, followed by a translational stop codon to yield the truncated molecule.

In one approach, consensus sequences encoding the heavy and light chain J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, cosmids, YACs, EBV-derived episodes, and the like. A convenient vector is one that encodes a functionally complete human CH or CL immunoglobulin sequence, with appropriate restriction sites, engineered, so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, including retroviral LTRs, e.g., SV-40 early promoter, (Okayama et al., Mol. Cell. Bio. 3:280 (1983)), Rous sarcoma virus LTR (Gorman et al., P.N.A.S. 79:6777 (1982)); and moloney murine leukemia virus LTR (Grosschedl et al., Cell 41:885 (1985)); native Ig promoters, etc.

Further, human antibodies or antibodies from other species can be generated through display-type technologies, including, without limitation, phage display, retroviral display, ribosomal display, and other techniques, using techniques well known in the art, and the resulting molecules can be subjected to additional maturation, such as affinity maturation, as such techniques are well known in the art. Wright and Harris, *supra*., Hanes and Plucthau, PNAS USA 94:4937-4942 (1997) (ribosomal display); Parmley and Smith Gene 73:305-318 (1988) (phage display); Scott, TIBS 17:241-245 (1992); Cwirla et al., PNAS USA 87:6378-6382 (1990); Russel et al., Nucl. Acids Research 21:1081-1085 (1993); Hoganboom et al. Immunol. Reviews 130:43-68 (1992), Chiswell and McCafferty, TIBTECH 10:80-84 (1992); and U.S. Patent No. 5,733,743. If display technologies are utilized to produce antibodies that are not human, such antibodies can be humanized as described above.

Using these techniques, antibodies can be generated to CTLA-4 expressing cells, CTLA-4 itself, forms of CTLA-4, epitopes or peptides thereof, and expression libraries thereto (*see e.g.*, U.S. Patent No. 5,703,057) which can thereafter be screened, as described above, for the activities described above.

### Additional Criteria for Antibody Therapeutics

As will be appreciated, it is generally not desirable to kill CTLA-4 expressing cells. Rather, one generally desires to simply inhibit CTLA-4 binding with its ligands to mitigate T cell down-regulation. One of the major mechanism through which antibodies kill cells is through fixation of complement and participation in CDC. The constant region of an antibody plays an important role in connection with an antibody's ability to fix complement and participate in CDC. Thus, generally one selects the isotype of an antibody to either provide the ability of complement fixation, or not. In the case of the present invention, generally, as mentioned above, it is generally not preferred to utilize an antibody that kills the cells. There are a number of isotypes of antibodies that are capable of complement fixation and CDC, including, without limitation, the following: murine IgM, murine IgG2a, murine IgG2b, murine IgG3, human IgM, human IgG1; and human. IgG3. Those isotypes that do not include, without limitation, human IgG2 and human IgG4.

It will be appreciated that antibodies that are generated need not initially possess a particular desired isotype, but, rather, the antibody as generated can possess any isotype and the antibody can be isotype-switched thereafter using conventional techniques that are well known in the art. Such techniques include the use of direct recombinant techniques (*see* e.g., U.S. Patent No. 4,816,397), cell-cell fusion techniques (*see e.g*., U.S. Patent application No. 08/730.639, filed October 11, 1996), among others.

In the cell-cell fusion technique, a myeloma or other cell line is prepared that possesses a heavy chain with any desired isotype, and another myeloma or other cell line is prepared that possesses the light chain. Such cells can, thereafter, be fused and a cell line expressing an intact antibody can be isolated.

By way of example, the majority of the CTLA-4 antibodies discussed herein are human anti-CTLA-4 IgG2 antibodies. Since such antibodies possess desired binding to the CTLA-4 molecule, any one of such antibodies can be readily isotype-switched to generate a human IgG4 isotype, for example, while still possessing the same variable region (which defines the antibody's specificity and some of its affinity).

Accordingly, as antibody candidates are generated that meet desired "structural" attributes as discussed above, they can generally be provided with at least certain additional "functional" attributes that are desired through isotype-switching.

### Design and Generation of Other Therapeutics

As discussed above, the effector function of the antibodies of the invention may be changed by isotype-switching to an IgG1, IgG2, IgG3, IgG4, IgD, IgA, IgE, or IgM for various therapeutic uses.

In connection with the generation of advanced antibody therapeutics, where complement fixation is a desirable attribute, it may be possible to sidestep the dependence on complement for cell killing through the use of bispecifics, immunotoxins, or radiolabels, for example.

In connection with bispecific antibodies, bispecific antibodies can be generated that comprise (i) two antibodies, one with a specificity to CTLA-4 and another to a second molecule that are conjugated together, (ii) a single antibody that has one chain specific to CTLA-4 and a second chain specific to a second molecule, or (iii) a single chain antibody that has specificity to CTLA-4 and the other molecule. Such bispecific antibodies can be generated using techniques that are well known, for example, in connection with (i) and (ii) *see e.g.,* Fanger et al., Immunol. Methods 4:72-81 (1994), and Wright and Harris, *supra.*; and in connection with (iii) *see e.g.,* Traunecker et al., Int. J. Cancer (Suppl.) 7:51-52 (1992).

In addition, "Kappabodies" (III et al., "Design and construction of a hybrid immunoglobulin domain with properties of both heavy and light chain variable regions", Protein Eng.10:949-57 (1997)), "Minibodies" (Martin et. al., "The affinity-selection of a minibody polypeptide inhibitor of human interleukin-6", EMBO J. 13:5303-9 (1994.)), "Diabodies" (Holliger et al., "'Diabodies': Small bivalent and bispecific antibody fragments", PNAS USA 90:6444-6448 (1993)), or "Janusins" (Traunecker et al., "Bispecific single chain molecules (Janusins) target cytotoxic lymphocytes on HIV infected cells", EMBO J 10:3655-3659 (1991), and Traunecker et al., "Janusin: New molecular design for bispecific reagents", Int.J.Cancer, Suppl.7:51-52 (1992)) may also be prepared.

In connection with immunotoxins, antibodies can be modified to act as immunotoxins utilizing techniques that are well known in the art. *See e.g.,* Vitetta, Immunol. Today 14:252 (1993). *See also* U.S. Patent No. 5,194,594. In connection with the preparation of radiolabeled antibodies, such modified antibodies can also be readily prepared utilizing techniques that are well known in the art. *See e.g*., Junghans et al., in Cancer Chemotherapy and Biotherapy 655-686 (2d edition, Chafner and Longo, eds., Lippincott Raven (1996)). *See also* U.S. Patent Nos. 4,681,581, 4,735,210, 5,101,827, 5,102,990 (RE 35,500), 5,648,471, and 5,697,902. Each of immunotoxins and radiolabeled molecules would be likely to kill cells expressing CTLA-4, and particularly those cells in which the antibodies of the invention are effective.

### Therapeutic Administration and Formulations

It will be appreciated that administration of therapeutic entities in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (15th ed, Mack Publishing Company, Easton, PA (1975)), particularly Chapter 87 by Blaug, Seymour, therein. These formulations include, for example, powders, pastes, ointments, jellies, waxes, oils, lipids, lipid (cationic or anionic) containing vesicles (such as Lipofectin^{™}), DNA conjugates, anhydrous absorption pastes, oil-in-water and water-in-oil emulsions, emulsions of carbowax (polyethylene glycols of various molecular weighs), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable, with the route of administration, *See also.* Powell , et al., "Compendium of excipients for parenteral formulations", PDA J.Pharm.Sci.Technol. 52:238-311 (1998), and the citations therein for additional information related to excipients and carriers well known to pharmaceutical chemists.

### Preparation of Antibodies

Antibodies in accordance with the invention are preferably prepared through the utilization of a transgenic mouse that has a substantial portion of the human antibody producing genome inserted but that is rendered deficient in the production of endogenous, murine, antibodies. Such mice, then, are capable of producing human immunoglobulin molecules and antibodies and are deficient in the production of murine .immunoglobulin molecules and antibodies. Technologies utilized for achieving the same are disclosed in the patents, applications, and references disclosed in the Background, herein. In particular, however, a preferred embodiment of transgenic production of mice and antibodies therefrom is disclosed in U.S. Patent. Application Serial No. 08/759,620, filed December 3, 1996, the disclosure of which is hereby incorporated by reference. *See also* Mendez et al., Nature Genetics 15:146-156 (1997), the disclosure of which is hereby incorporated by reference.

Through use of such technology, we have produced fully human monoclonal antibodies to a variety of antigens. Essentially, we immunize XenoMouse™ lines of mice with an antigen of interest, recover lymphatic cells (such as B cells) from the mice that express antibodies, fuse such recovered cells with a myeloid-type cell line to prepare immortal hybridoma cell lines, and such hybridoma cell lines are screened and selected to identify hybridomas cell lines that produce antibodies specific to the antigen of interest. We utilized these techniques in accordance with the present invention for the preparation of antibodies specific to CTLA-4. Herein, we describe the production of multiple hybridoma cell lines that produce antibodies specific to CTLA-4. Further, we provide a characterization of the antibodies produced by such cell lines, including nucleotide and amino acid sequence analyses of the heavy and light chains of such antibodies.

The antibodies derived from hybridoma cell lines discussed herein are designated as 3.1.1, 4.1.1, 4.8.1, 4.10.2, 4.13.1, 4.14.3, 6.1.1, 11.2.1, 11.6.1, 11.7.1, 12.3.1.1, and 12.9.1.1. Each of the antibodies produced by the aforementioned cell lines are either fully human IgG2 or IgG4 heavy chains with human kappa light chains. In general, antibodies in accordance with the invention possess very high affinities, typically possessing Kd's of from about 10⁻⁹ through about 10⁻¹¹ M, when measured by either solid phase or solution phase.

As will be appreciated, antibodies in accordance with the present invention can be expressed in cell lines other than hybridoma cell lines. Sequences encoding the cDNAs or genomic clones for the particular antibodies can be used for transformation of a suitable mammalian or non-mammalian host cell. Transformation can be by any known method for introducing polynucleotides into a host cell, including, for example, packaging the polynucleotide in a virus (or into a viral vector) and transducing a host cell with the virus (or vector), or by transfection procedures known in the art, as exemplified by U.S. Patent Nos. 4,399,216, 4,912,040, 4,740,461, and 4,959,455 (which patents are hereby incorporated herein by reference). The transformation procedure used depends upon the host to be transformed. Methods for introduction of heterologous polynucleotides into mammalian cells are well known in the art and include, but are not limited to, dextran-mediated transfection, calcium phosphate precipitation, polybrene-mediated transfection, protoplast fusion, electroporation, particle bombardment, encapsulation of the polynucleotide(s) in liposomes, peptide conjugates, dendrimers, and direct microinjection of the DNA into nuclei.

Mammalian cell lines available as hosts for expression are well known in the an and include many immortalized cell lines available from the American Type Culture Collection (ATCC), including, but not limited to, Chinese hamster ovary (CHO) cells, NSO₀ cells, HeLa cells, baby hamster kidney (BHK) cells, monkey kidney cells (COS), human hepatocellular carcinoma cells (e.g., Hep G2), and a number of other cell lines. Non-mammalian cells, including, but not limited to, bacterial, yeast, insect, and plants, can also be used to express recombinant antibodies. Site directed mutagenesis of the antibody CH2 domain to eliminate glycosylation may be preferred in order to prevent changes in either the immunogenicity, pharmacokinetic, and/or effector functions resulting from non-human glycosylation. The expression methods are selected by determining which system generates the highest expression levels and produce antibodies with constitutive CTLA-4-binding properties.

Further, expression of antibodies of the invention (or other moieties therefrom) from production cell lines can be enhanced using a number of known techniques. For example, the glutamine synthetase and DHFR gene expression systems are common approaches for enhancing expression under certain conditions. High expressing cell clones can be identified using conventional techniques, such as limited dilution cloning and Microdrop technology. The GS-system is discussed in whole or part in connection with European Patent Nos. 0 216 846, 0 256 055, and 0 323 997, and European Patent Application No. 89303964.4.

Antibodies of the invention can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals. *See. e.g.,* U.S. Patent Nos. 5,827,690, 5,756,687, 5,750,172, and 5,741.957.

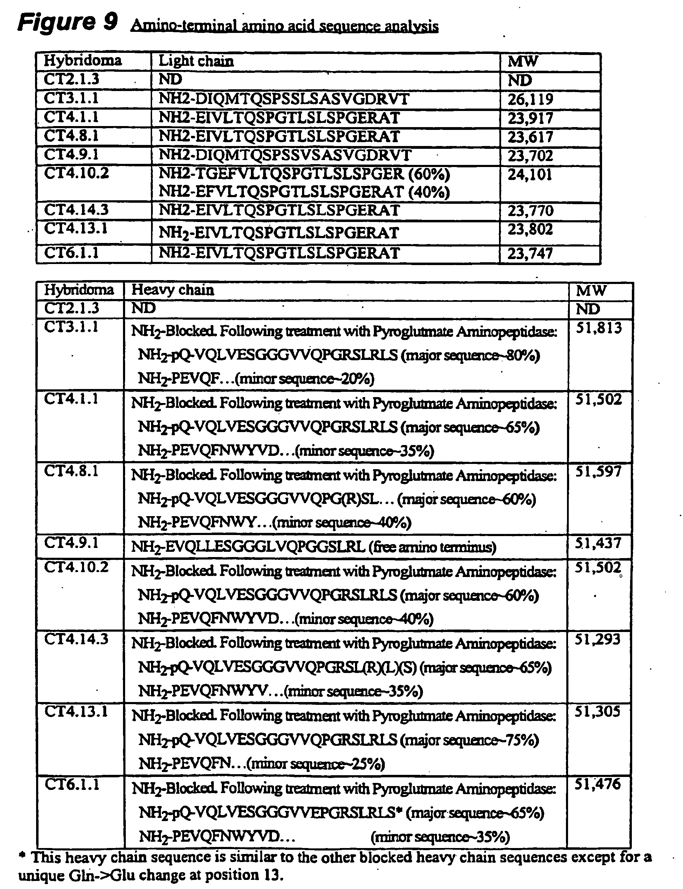

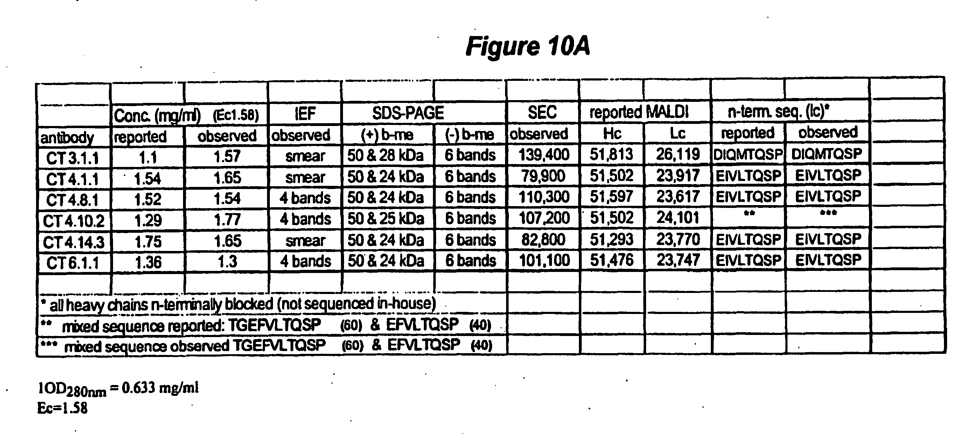

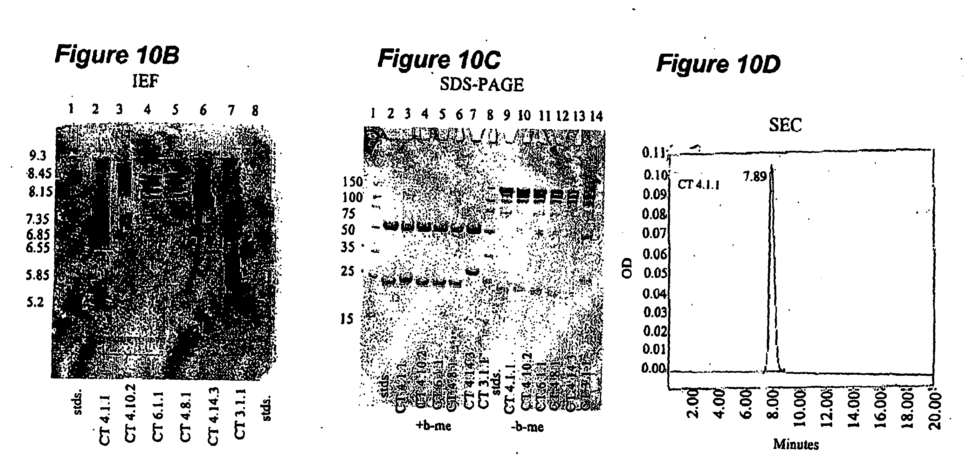

Antibodies in accordance with the present invention have been analyzed structurally and functionally. In connection with the structures of the antibodies, amino acid sequences of the heavy and kappa light chains have been predicted based on cDNA sequence obtained through RT-PCR of the hybridomas. *See* Examples 3 and 4 and Figures 1-8. N-terminal sequencing of the antibodies was also conducted in confirmation of the results discussed in Examples 3 and 4. *See* Example 5 and Figure 9. Kinetic analyses of the antibodies were conducted to determine affinities. *See* Examples 2. Further, antibodies were analyzed by isoelectric focusing (IEF), reducing gel electrophoresis (SDS-PAGE), seize exclusion chromatographs, liquid chromatography/mass spectroscopy, and mass spectroscopy, and the antibody production by the hybridomas was assessed. *See* Example 6 and Figure 10.

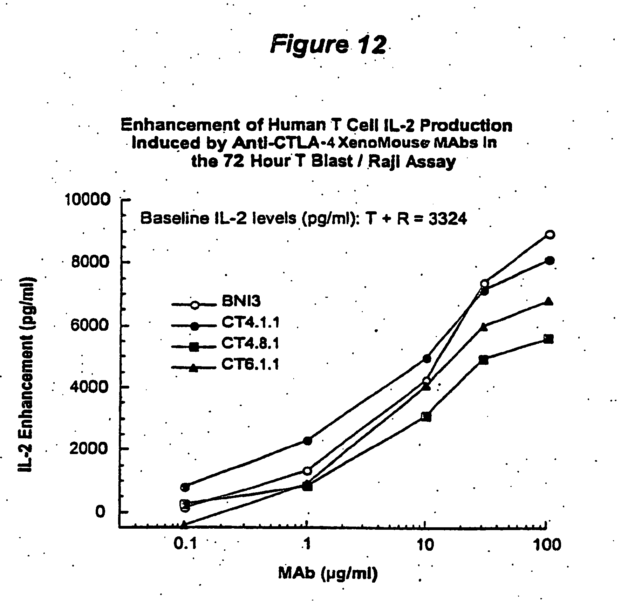

In connection with functional analysis of antibodies in accordance with the present invention, such antibodies proved to be potent inhibitors of CTLA-4 and its binding to its ligands of the B7 family of molecules. For example, antibodies in accordance with the present invention were demonstrated to block CTLA-4 binding to either B7-1 or B7-2. *See* Example 7. Indeed, many of the antibodies in accordance with the invention possess nanomolar and subnanomalar IC₅₀s with respect to inhibiting CTLA-4 binding to B7-1 and B7-2. Further, antibodies of the invention possess excellent selectivity for CTLA-4 as compared to CD28, CD44, B7-2 or hIgGl. See Example 8. Selectivity is a ratio that reflects the degree of preferential binding of a molecule with a first agent as compared to the molecule's binding with a second, and optionally other molecules. Herein, selectivity refers to the degree of preferential binding of an antibody of the invention to CTLA-4 as compared to the antibody's binding to other molecules such as CD28, CD44, B7-2, or hIgG1. Selectivity values of antibodies of the invention greater than 500:1 are common. Antibodies of the invention have also been demonstrated to induce or enhance expression of certain cytokine (such as IL-2 and IFN-γ) by cultured T cells in a T cell blast model. See Examples 9 and 10 and Figures 12-17. Further, it is expected that antibodies of the invention will inhibit the growth of tumors in appropriate *in vivo* tumor models. The design of which models are discussed in Example 11. and 12.

The results demonstrated in accordance with the present invention indicate that antibodies of the present invention possess certain qualities that may make the present antibodies more efficacious than current therapeutic antibodies against CTLA-4.

In particular, the 4.1.1, 4.8-1. and 6.1.1 antibodies possess highly desirable properties. Their structural characteristics, functions, or activities provide criteria that facilitate the design or selection of additional antibodies or other molecules as discussed above. Such criteria include one or more of the following:
Ability to compete for binding to CTLA-4 with one or more of the antibodies of the invention;
Similar binding specificity to CTLA-4 as one or more of the antibodies of the invention;
A binding affinity for CTLA-4 of about 10⁻⁹ M or greater and preferably of about 10⁻¹⁰M or greater;
Does not cross react with lower mammalian CTLA-4, including, preferably, mouse, rat, or rabbit, and preferably mouse or rat CTLA-4;.
Cross reacts with primate CTLA-4, including, preferably, cynomolgous and rhesus CTLA-4;
A selectivity for CTLA-4 over CD28, B7-2, CD44, or hIgG1 of at least about 100:1 or greater and preferably of about 300, 400, or 500:1 or greater,
An IC₅₀ in blocking CTLA-4 binding to B7-2 of about 100 nM or lower and preferably 5, 4, 3, 2, 1, 0.5, or 0.38 nM or lower,
An IC₅₀ in blocking CTLA-4 binding to B7-1 of about of about 100 nM or lower and preferably 5, 4, 3, 2, 1, 0.5, or 0.50 nM or lower,

An enhancement of cytokine production in one or more *in vitro* assays, for example:
An enhancement of IL-2 production in a T cell blast/Raji assay of about 500 pg/ml or greater and preferably 750, 1000, 1500, 2000, 3000, or 3846 pg/ml or greater;
An enhancement of IFN-γ production in a T cell blast/Raji assay of about 500 pg/ml or greater and preferably 750, 1000, or 1233 pg/ml or greater, or.
An enhancement of IL-2 production in a hPBMC or whole blood superantigen assay of about 500 pg/ml or greater and preferably 750, 1000, 1200, or 1511-pg/ml or greater. Expressed another way, it is desirable that IL-2 production is enhanced by about 30, 35, 40, 45, 50 percent or more relative to control in the assay.

It is expected that antibodies (or molecules designed or synthesized therefrom) having one or more of these properties will possess similar efficacy to the antibodies described in the present invention.

The desirable functional properties discussed above can often result from binding to and inhibition of CTLA-4 by a molecule (i.e., antibody, antibody fragments, peptide, or small molecule) in a similar manner as an antibody of the invention (i.e., binding to the same or similar epitope of the CTLA-4 molecule).

The molecule may either be administered directly (i.e., direct administration to a patient of such molecules). Or, alternatively, the molecule may be "administered" indirectly (i.e.. a peptide or the like that produces an immune response in a patient (similar to a vaccine) wherein the immune response include the generation of antibodies that bind to the same or similar epitope or an antibody or fragment that is produced *in situ* after administration of genetic materials that encode such antibodies or fragments thereof which bind to the same or similar epitope). Thus; it will be appreciated that the epitope on CTLA-4 to which antibodies of the invention bind to can be useful in connection with the preparation and/or design of therapeutics in accordance with the invention. In drug design, negative information is often useful as well (i.e., the fact that an antibody which binds to CTLA-4 does not appear to bind to an epitope that acts as an inhibitor of CTLA-4 is useful). Thus, the epitope to which antibodies of the invention bind that do not lead to the desired functionality can also be very useful. Accordingly, also contemplated in accordance with the present invention are molecules (and particularly antibodies) that bind to the same or similar epitope as antibodies of the invention.

In addition to the fact that antibodies of the invention and the epitopes to which they bind are contemplated in accordance with the invention, we have conducted some preliminary epitope mapping studies of certain antibodies in accordance with the invention and particularly the 4.1.1 antibody and the 11.2.1 antibody of the invention;

As a first step, we conducted .BIAeore competition studies to generate a rough map of binding as between certain antibodies of the invention in connection with their ability to compete for binding to CTLA-4. To this end, CTLA-4 was bound to a BIAcore chip, and a first antibody, under saturating conditions, was bound thereto and competition of subsequent secondary antibodies binding to CTLA-4 was measured. This technique enabled generation of a rough map in to which families of antibodies can be classified.

Through this process, we determined that certain antibodies could be categorized as falling into the following epitopic categories:

| Category | Antibodies | Competition for CTLA-4, Binding |
|---|---|---|
| A | BO1M* | Freely cross-compete with one another; cross-compete with category B; some cross-competition with category D |
| | BO2M** | |
| B | 4.1.1 | Freely cross-compete with one another; cross-compete with category A, C and D. |
| | 4.13.1 | |
| C | 6.1.1 | Freely cross-compete with one another, cross-compete with category B and category D |
| | 3.1.1 | |
| | 4.8.1 | |
| | 11.2.1 | |
| | 11.6.1 | |
| | 11.7.1 | |
| D | 4.14.3 | Cross-compete with category C and B; some cross-competition with category A |
| E | 4.9.1 | BNI3 blocks 4.9.1 binding to CTLA4 but not the reverse |
| | BNI3*** | |

| | | |
|---|---|---|
| (*)(**) Available from Biostride. (***) Available from Pharmingen. | | |

As a next step, we endeavored to determine if the antibodies recognized a linear epitope on CTLA-4 under reducing and non-reducing conditions on Western blots. We observed that pone of the 4.1.1, 3.1.1, 11.7.1, 11.6.1, or 11.2.1 antibodies appeared to recognize a reduced form of CTLA-4 on the Western blots. Accordingly, it appeared likely that the epitope to which each of these antibodies bound was not a linear epitope hut more likely was a conformational epitope, the structure of which may have been abrogated under seducing conditions.

Therefore, we sought to determined whether we could learn about residues within the CTLA-4 molecule that are important for binding of antibodies of the invention. One manner that we utilized was to conduct kinetic assessments of off-rates as between human CTLA-4 and two highly conserved primate CTLA-4 molecules (cynomologous and marmoset CTLA-4). BIAcore studies demonstrated that the 4.1.1 antibody bound to human, cynomologous, and marmoset CTLA-4 at the same rate. However, with respect to off-rates (affinity), the 4.1.1 antibody had the highest affinity (slowest off-rate) for human, a faster off-rate with cynomologous, and a much faster off-rate for marmoset. The 11.2.1 antibody of the invention, on the other hand, binds to human, cynomologous, and marmoset CTLA-4 at the about the same rate and has about the same relative off-rate for each of the three. This information further indicates that the 4.1.1 and 11.2.1 antibodies bind to different epitopes on CTLA-4.

to further study the epitope to which the category B and C antibodies of the invention bind, we conducted certain site directed mutagenesis studies. Marmoset CTLA-4 possesses two important changes at residues 105 and 106 relative to human CTLA-4 Such differences are a leucine to methionine change at residue 105 and a glycine to serine change at residue 106. Accordingly, we mutated cDNA encoding human CTLA-4 to encode a mutated CTLA-4 having the L105M and G106S changes. The homologue replacement mutant CTLA-4. did not effect binding of a B7.2-IgG1 fusion protein. Further, binding with the 11.2.1 antibody of the invention was not effected. However, such molecule was significantly inhibited in its ability to bind with the 4.1.1 antibody (similar to marmoset). Next, we mutated a cDNA encoding marmoset CTLA-4 create a mutant marmoset CTLA-4 having a S106G charge. Such change resulted in restoration of stable binding between the 4.1.1 antibody, and the marmoset CTLA-4 mutant. In addition, we mutated a cDNA encoding marmoset CTLA-4 to create a mutant marmoset CTLA-4 having a M105L charge. Such change partially restored binding between the 4.1.1 antibody and the mutant CTLA-4.

Each of the category B through D antibodies of the invention appear to possess similar functional properties and appear to have the potential to act as strong anti-CTLA-4 therapeutic agents. Further, each of the molecules exhibit certain cross-competition in their binding for CTLA-4. However, as will be observed from the above discussion, each of the molecules in the different categories appear to bind to separate conformational epitopes on CTLA-4.

From the foregoing, it will be appreciated that the epitope information discussed above indicates that antibodies (or other molecules, as discussed above) that cross-compete with antibodies of the invention will likely have certain therapeutic potential in accordance with the present invention. Further, it is expected that antibodies (or other molecules, as discussed above) that cross-compete with antibodies of the invention (i.e., cross-compete with category B, C and/or D antibodies) will likely have certain additional therapeutic potential in accordance with the present invention. Additionally, it is expected that antibodies (or other molecules, as discussed above) that cross-compete with antibodies of the invention (i.e., cross-compete with category B, C and/or. D antibodies) and that (i) are not reduced in their binding to marmoset CTLA-4 (similar to the 11.2.1 antibody) or (ii) are reduced in their binding to marmoset CTLA-4 similar to the 4.1.1 antibody) will likely have certain additional therapeutic potential in accordance with the present invention. Antibodies (or other molecules, as discussed above) that compete with categories A and E may also have certain therapeutic potential.

### EXAMPLES

The following examples, including the experiments conducted and results achieved are provided for illustrative purposes only and are not to be construed as limiting upon the present invention.

### EXAMPLE 1

### Generation of Anti-CTLA-4-Antibody Producing Hybridomas

Antibodies of the invention were prepared, selected, and assayed in accordance with the present Example.

***Antigen Preparation:*** Three distinct immunogens were prepared for immunization of the XenoMouse^{™} mice: (i) a CTLA-4-IgG fusion protein, (ii) a CTLA-4 peptide, and (iii) 300.19 murine lymphoma cells transfected with a mutant of CTLA-4 (Y201 V) that is constitutively expressed on the cell surface.

### (i) CTLA-4-IgG1 Fusion Protein:

### Expression Vector Construction:

The cDNA encoding the mature extracellular domains of CTLA-4 was PCR amplified from human thymus cDNA library (Clontech) using primers designed to published sequence (Eur. J. Immunol. 18:1901-1905 (1988)). The fragment was directionally subcloned into pSR5, a Sindbis virus expression plasmid (InVitrogen), between the human Oncostatin M signal peptide and human IgG gamma 1 (IgG1) CH1/CH2/CH3 domains. The fusion protein does not contain a hinge domain but contains cysteine 120 in the extracellular domain of CTLA-4 to form a covalent dimer. The resulting vector was called CTLA-4-IgG1/pSR5. The complete CTLA-4-IgG1 cDNA in the vector was sequence confirmed in both strands. The amino acid sequence of the CTLA4-Ig protein is shown below. The mature extracellular domain for CD44 was PCR amplified from human lymp4ocyte library (Clontech) and subcloned into pSinRep5 to generate a control protein with the identical IgG1 tail.

### OM-CTLA-4-IgG1 Fusion Protein:

MGVLLTQRTLLSLVLALLFPSMASMAMHVAQPAVVLASSRGIASFVC
EYASPGKATEVRVTVLRQADSQVTEVCAATYMMGNELTFLDDSICT
GTSSGNQVNLTIQGLRAMDTGLYICKVELMYPPPYYLGIGNGTQIY
VIDPEPCPDSDLEGAPSVFLFPPKPKDTLMISRTPEVTCVVVDVSHEDPE
VKFNWYVDGVEVHNAKTKPREEQYNSTYRVVSVLTVLHQDWLNGKE
YKCKVSNKALPTPIEKTISKAKGQPREPQVYTLPPSRDELTKNQVSLTCL
VKGFYPSDIAVEWESNGQPENNYKTTPPVLDSDGSFFLYSKLTVDKSR
WQQGNVFSCSVMHEALHNHYTQKSLSLSPGK

Underlined: signal peptide

Bold: CTLA-4 extracellular domain

The cDNAs for mature extracellular domain of CD28 were PCR amplified from human lymphocyte library (Clontech) and then subcloned into pCDM8 (J. Immunol. 151: 5261-71 (1993)) to produce a human IgG1 fusion protein containing both thrombin cleavage and hinge regions. Marmoset, cynomologous, and rhesus CTLA-4 were cloned from mRNA isolated from PHA stimulated PBMCs using standard techniques of degenerate PCR. Sequencing demonstrated that the rhesus and cynomologous amino acid sequences were identical with three differences from mature human CTLA-4 extracellular domain (S13N, 117T and L105M). Marmoset CTLA-4 demonstrated ten amino acid Differences from the mature human CTLA-4 extracellular domain (V21A, V33I, A41T, A51G, 54I, S71F, Q75K, T88M, L105M and G106S). Site directed mutagenesis was used to make single point mutations of all amino acids different in marmoset CTLA-4 to map amino acids important for interation of the antibodies with human CTLA-4 -IgG. Mutations of human and marmoset CTLA-4 -IgG for epitope mapping were generated by Matchmaker site-directed mutagenesis (Promega). The IgG fusion proteins were produced by transient transfection of COS7 cells and purified using standard Protein A techniques. Mutant CTLA-4-IgG proteins were evaluated for binding to antibodies by immunoblotting and using BIAcore analyses.

### Recombinant Protein Expression/Purification:

Recombinant sindbis virus was generated by electroporating (Gibco) baby hamster kidney cells with SP6 *in vitro*, transcribed CTLA-4-IgG1/pSR5 mRNA and DH-26S helper mRNA, as described by In Vitrogen. Forty eight hours later recombinant virus was harvested and titered for optimal protein expression in Chinese hamster ovary cells (CHO-K1). CHO-K1 cells were culture in suspension in DMEM/F12 (Gibco) containing 10% heat-inactivated fetal bovine serum (Gibco), non-essential amino acids (Gibco), 4mM glutamine (Gibco), penicillin/streptomycin (Gibco), 10mM HEPES pH 7.5 (Gibco). To produce CTLA-4-IgG, the CHO-K1 cells were resuspended at 1x10⁷ cells/ml in DMEM/F12 and incubated with sindbis virus for one hour at room temperature. Cells were then diluted to 1x10⁶/ml in DMEM/F12 containing 1% fetal bovine serum depleted of bovine IgG using Protein A Sepharose (Pharmacia), non-essential amino acids, 4mM glutamine, 12.5mM HEPES, pH 7.5, and penicillin/streptomycin. Forty eight hours post-infection cells were pelleted, and conditioned media was harvested and supplemented with complete protease inhibitor tablets (Boehringer Mannheim), pH adjusted to 7.5, and filtered 0.2µ (Nalgene). FPLC (Pharmacia) was used to affinity purify the fusion protein using a 5ml Protein A HiTrap column (Pharmacia) at a 10ml/min flow rate. The column was washed with 30 bed volumes of PBS and eluted with 0.1M glycine/HCl, pH 2.8 at 1ml/min. Fractions (1ml) were immediately neutralized to pH 7.5 with Tris, pH 9. The fractions containing CTLA-4-IgG1 were identified by SDS-PAGE and then concentrated using Centriplus 50 (Amicon) before applying to Sepharose 200 column (Pharmacia) at 1ml/min using PBS as the solvent. Fractions containing CTLA-4-IgG1 were pooled, sterile filtered 0.2µ (Millipore), aliquoted and frozen at -80°C. CD44-IgG1 was expressed and purified using the same methods. CD28-IgG was purified from conditioned media from transiently transfected COS7 cells.

### Characterization of CTLA-4-IgG1:

The purified CTLA-4-IgG1 migrated as a single band on SDS-PAGE using colloidal Coomassie staining (Novex). Under non-reducing conditions CTLA-4-IgG1 was a dimer (100kDa), that reduced to a 50kDa monomer when treated with 50mM DTT. Amino acid sequencing of the purified CTLA-4-IgG1 in solution confirmed the N-terminus of CTLA-4 (MHVAQPAVVLAS); and that the Oncostatin-M signal peptide was cleaved from the mature fusion protein.

The CTLA-4-IgG1 bound to immobilized (B7-1-IgG) in a concentration dependent manner and the binding was blocked by a hamster-anti-human anti-CTLA-4 antibody (BNI3: Pharmingen). The sterile CTLA-4-IgG was endotoxin free and quantitated by OD280 using 1.4 as the extinction coefficient. The yield of purified CTLA-4-IgG ranged between 0.5-3mgs/liter of CHO-K1 cells.

### (ii) CTLA-4 Peptide:

The following CTLA-4 peptide was prepared as described below:

NH₂:MHVAQPAVVLASSRGIASFVCEYASPGKATEVRVTVLRQADSQVT
EVCAATYMMGNELTFLDDSICTGTSSGNQVNLTIQGLRAMDTGLYICK
VELMYPPPYYLGIGNGTQIYVIDPEPC-CONH₂

### Abbreviations/Materials:

NMP, N-Methylpyrrolidinone; TFE, 2,2,2-Trifluoroethanol; DCM, Dichloromethane; FMOC, Fluorenyl Methoxycarbonyl. All reagents were supplied by Pekin Elmer, with the following exceptions: TFE, Aldrich Chemical_{;} FMOC-PAL-PEG resin, Perseptive Biosystems. Fmoc-Arg(PMC)-OH, FMOC-Asn(Trt)-OH, FMOC-Asp(tBu)-OH, FMOC-Cys(Trt)-OH, FMOC-Glu(tBu)-OH, FMOC-Gln(Trt)-OH, FMOC-His(Boc)-OH, FMOC-Lys(BOC)-OH, FMOC-Ser(tBu)-OH, FMOC-Thr(tBu)-OH and FMOC-Tyr(tBu)-OH were used for those amino acids requiring side chain protecting groups

### Peptide Synthesis:

Peptide synthesis was performed on a Perkin-Elmer 431a, retrofitted with feedback monitoring via UV absorbance at 301nm (Perkin-Elmer Model 759A detector). The peptide sequence was assembled on a FMOC-PAL-PEG resin using conditional double coupling cycles. Forced double couplings were performed at cycles 10,11,18,19,20 and 28 through 33. The resin was washed with a 50% mixture of DCM and TFE at the completion of each acylation cycle, followed by capping of unreacted amino groups with acetic anhydride in NMP. Resin was removed from the reactor after completing cycle 49 and the remainder continued to completion Peptide cleavage from the resin was performed using Reagent K (King et al., International Journal of Protein and Peptide Research 36:255-266 (1990)) for 6 hours on 415mg of resin affording 186mg crude CTLA-4 peptide.

### Peptide Characterization:

25mg aliquots of the crude CTLA-4 peptide were dissolved in 5ml 6M guanidine-HCl/100mM K₂PO₃ at pH6.4 and eluted over a Pharmacia Hi Load Superdex 75 16/60 column (16mm x 600mm, 120ml bed volume) with 2M guanidine-HCl / 100mM K₂PO₃ at pH6.4 at 2 ml/ min for 180 minutes collecting 5 ml fractions. The fractions where analyzed by loading 1.7µl of fractions onto a NuPAGE Laemmli gel running with MES running buffer and visualizing via Daichii silver stain protocol. Those fractions exhibiting a molecular weight of 12 KDa, as judged versus molecular weight standards, were pooled together and stored at 4°C. The combined fractions were analyzed by UV and gel electrophoresis. Amino acid sequencing was performed by absorbing a 100 microliter sample in a ProSorb cartridge (absorbed onto a PVDF membrane) and washing to remove the buffer salts. Sequencing was performed on an Applied Biosystems 420. The expected N-terminal sequence (M H V A Q P A V V L A) was observed. Immunoblotting demonstrated that the peptide was recognized by the BNI3 anti-human CTLA-4 (Pharmingen). To desalt, an aliquot containing 648µg of material was placed in 3500 Da MWCO dialysis tubing and dialyzed against 0.1 % TFA / H2O at 4°C for 9 days with stirring. The entire content of the dialysis bag was lyophilized to a powder.

### (iii) 300.19 cells transfected with CTLA-4 (Y201V)

The full length CTLA-4 cDNA was PCR amplified from human thymus cDNA library (Stratagene) and subcloned into pIRESneo (Clontech). A mutation of CTLA-4 that results in constitutive cell surface expression was introduced using MatchMaker Mutagenesis System (Promega). Mutation of tyrosine, Y201 to valine inhibit binding of the Adaptin protein, AP50, that is responsible for the rapid internalization of CTLA-4 (Chuang et al. J. Immunol. 159:144-151 (1997)). Mycoplasma-free 300.19 murine lymphoma cells were cultured in RPMI-1640 containing 10% fetal calf serum, non-essential amino acids; penicillin/streptomycin, 2mum glutamine, 12.5mM HEPES, pH. 7.5, and 25uM beta-mercaptoethanol. Cells were electroporated (3x10⁶/0.4ml serum-free RPMI) in a 1ml chamber with 20ug CTLA-4-Y201V/pIRESneo using 200V/1180uF (Gibco CellPorator). Cells were rested for 10 minutes and then 8mls of prewarmed complete RPMI media. At 48 hours cells were diluted to 0.5 x10⁶/ml in complete RPMI media containing 1mg/ml G418 (Gibco). Resistant cells were expanded and shown to express CTLA-4 on the cell surface using the BNI3 antibody conjugated with phycoerythrin (Pharmingen). High level expressing cells were isolated by sterile sorting.

***Immunization and hybridomas generation**:* XenoMouse mice (8 to. 10 weeks old) were immunized (i) subcutaneously at the base of tail with 1x10⁷ 300.19 cells that were transfected to express CTLA-4 as described above, resuspended in phosphate buffered saline (PBS) with complete Freund's adjuvant, or (ii) subcutaneously at the base df tail with (a) 10 µg the CTLA-4 fusion protein or (b) 10 µg CTLA-4 peptide, emulsified with complete Freund's adjuvant. In each case, the dose was repeated three or four times in incomplete Freund's adjuvant. Four days before fusion, the mice received a final injection of the immunogen or cells in PBS. Spleen and/or lymph node lymphocytes from immunized mice were fused with the murine non-secretory myeloma P3 cell line and were subjected to HAT selection as previously described (Galfre, G. and Milstein, C., "Preparation of monoclonal antibodies: strategies and procedures", Methods Enzymol. 73:3-46 (1981)). A large panel of hybridomas all secreting CTLA-4 specific human IgG₂κ or IgG₄κ (as detected below) antibodies were recovered.

***ELISA assay**:* ELISA for determination of antigen-specific antibodies in mouse serum and in hybridoma supernatants was carried out as described (Coligan et al., Unit 2.1, "Enzyme-linked immunosorbent assays," in Current protocols in immunology (1994)) using CTLA-4-Ig fusion protein to capture the antibodies. For animals that are immunized with the CTLA-4-Ig fusion protein, we additionally screen for non-specific reactivity against the human Ig portion of the fusion protein. This is accomplished using ELISA plates coated with human IgG1 as a negative control for specificity.

In a preferred ELISA assay, the following techniques are used:

ELISA plates are coated with 100 µl/well of the antigen in plate coating buffer (0.1 M carbonate buffer, pH 9.6, and NaHCO₃ (MW 84)_{,} 8.4g/L). Plates are then incubated at 4°C overnight. After incubation, coating buffer is removed and the plate is blocked with 200 µl/well blocking buffer (0.5% BSA, 0.1% Tween-20, 0.01% Thimerosal in 1x PBS) and incubated at room temperature for 1 hour. Alternatively, the plates are stored in refrigerator with blocking buffer and plate sealers. Blocking buffer is removed and 50 µl/well of hybridoma supernatant, serum or other hybridoma supernatant (positive control) and HAT media or blocking buffer (negative control) is added. The plates are incubated at room temperature for 2 hours. After incubation, the plate is washed with washing buffer (1x PBS). The detecting antibody (i.e., mouse anti-human IgG2-HRP (SB, #9070-05) for IgG2 antibodies or mouse anti-human IgG4-HRP (SB #9200-05) for IgG4 antibodies) is added at 100µl/well (mouse anti-human IgG2-HRP @ 1:2000 or mouse anti-human IgG4-HRP @ 1:1000 (each diluted in blocking buffer)). The plates are incubated at room temperature for 1 hour and then washed with washing buffer. Thereafter, 100 µl/well of freshly prepared developing solution (10 ml substrate buffer, 5 mg OPD (o-Phenylenediamine; Sigma, Cat.No. P-7288), and 10 µl 30% H₂O₂ (Sigma)) is added to the wells. The plates are allowed to develop 10-20 minutes, until negative control wells barely start to show color. Thereafter, 100 µl/well of stop solution (2 M H₂SO₄) is added and the plates are read on an ELISA plate reader at wavelength 490 nm.

### Determination of affinity constants of fully human Mabs by BIAcore:

Affinity measurement of purified human monoclonal antibodies. Fab fragments, or hybridoma supernatants by plasmon resonance was carried out using the BIAcore 2000 instrument, using general procedures outlined by the manufacturers.

Kinetic analysis of the antibodies was carried out using antigens immobilized onto the sensor surface at a low density. Three surfaces of the BIAcore sensorchip were immobilized with the CTLA-4-Ig fusion protein at a density ranging from approximately 390-900 using CTLA-4-Ig fusion protein at 20 or 50 µg/ml in 10 mM sodium acetate at pH 5.0 using the amine coupling kit supplies by the manufacturer (BIAcore, Inc.). The fourth surface of the BIAcore sensorchip was immobilized with IgG1 (900 RU) and was used as a negative control surface for non-specific binding. Kinetic analysis was performed at a flow rate of 25 or 50 microliters per minute and dissociation (kd or k_{off}) and association (ka or kₒₙ) rates were determined using the software provided by the manufacturer (BLA evaluation 3.0) that allows for global Siting calculations.

### EXAMPLE 2

### Affinity Measurement of_Anti-CTLA-4-Antibodies

In the following Table, affinity measurements for certain of the antibodies selected in this manner are provided:

**TABLE I**

| | **Solid Phase** **(by BIAcore)** | | | | |
|---|---|---|---|---|---|
| **Hybridoma** | **On-rates** **Kₐ** **(M⁻¹S⁻¹x10⁶)** | **Off-rates** **K_{d}** **(S⁻¹x10⁻⁴)** | **Association Constant** **KA (1/M)= kₐ/k_{d}x10¹⁰** | **Dissociation Constant** **KD(M)= K_{d}/kₐx10⁻¹⁰** | **Surface Density** **[RU]** |
| **Moab01** | 0.68 | 1.01 | 0.67 | 1.48 | 878.7 |
| | 0.70 | 4.66 | 0.15 | 6.68 | 504.5 |
| | 0.77 | 6.49 | 0.19 | 8.41 | 457.2 |
| | 0.60 | 3.08 | 0.20 | 5.11 | 397.8 |
| **4.1.1** | 1.85 | 0.72 | 2.58 | 0.39 | 878.7 |
| | 1.88 | 1.21 | 1.55 | 0.64 | 504.5 |
| | 1.73 | 1.54 | 1.13 | 0.88 | 457.2 |
| | 1.86 | 1.47 | 1.26 | 0.79 | 397.8 |
| **4.8.1** | 0.32 | 0.07 | 4.46 | 0.22 | 878.7 |
| | 0.31 | 0.23 | 1.33 | 0.75 | 504.5 |
| | 0.28 | 0.06 | 4.82 | 0.21 | 397.8 |
| **4.14.3** | 2.81 | 3.04 | 0.92 | 1.08 | 878.7 |
| | 2.88 | 3.97 | 0.73 | 1.38 | 504.5 |
| | 2.84 | 6.66 | 0.43 | 2.35 | 457.2 |
| | 3.17 | 5.03 | 0.63 | 1.58 | 397.8 |
| **6.1.1** | 0.43 | 0.35 | 1.21 | 0.83 | 878.7 |
| | 0.46 | 0.90 | 0.51 | 1.98 | 504.5 |
| | 0.31 | 0.51 | 0.61 | 1.63 | 457.2 |
| | 0.45 | 0.79 | 0.57 | 1.76 | 397.8 |
| **3.1.1** | 1.04 | 0.96 | 1.07 | 0.93 | 878.7 |
| | 0.95 | 1.72 | 0.55 | 1.82 | 504.5 |
| | 0.73 | 1.65 | 0.44 | 2.27 | 457.2 |
| | 0.91 | 2.07 | 0.44 | 2.28 | 397.8 |
| **4.9.1** | 1.55 | 13.80 | 0.11 | 8.94 | 878.7 |
| | 1.43 | 19.00 | 0.08 | 13.20 | 504.5 |
| | 1.35 | 20.50 | 0.07 | 15.20 | 397.8 |
| **4.10.2** | 1.00 | 2.53 | 0.39 | 2.54 | 878.7 |
| | 0.94 | 4.30 | 0.22 | 4.55 | 504.5 |
| | 0.70 | 5.05 | 0.14 | 7.21 | 457.2 |
| | 1.00 | 5.24 | 0.19 | 5.25 | 397.8 |
| **2.1.3** | 1.24 | 9.59 | 0.13 | 7.72 | 878.7 |
| | 1.17 | 13.10 | 0.09 | 11.20 | 504.5 |
| | 1.11 | 13.00 | 0.09 | 11.70 | 397.8 |
| **4.13.1** | 1.22 | 5.83 | 0.21 | 4.78 | 878.7 |
| | 1.29 | 6.65 | 0.19 | 5.17 | 504.5 |
| | 1.23 | 7.25 | 0.17 | 5.88 | 397.8 |

As will be observed, antibodies prepared in accordance with the invention possess high affinities and binding constants.

### EXAMPLE 3

### Structures of Anti-CTLA-4-Antibodies Prepared in Accordance with the Invention

In the following discussion, structural information related to antibodies prepared in accordance with the invention is provided.

In order to analyze structures of antibodies produced in accordance with the invention, we cloned genes encoding the heavy and light chain fragments out of the particular hybridoma. Gene cloning and sequencing was accomplished as follows:

Poly(A)⁺ mRNA was isolated from approximately 2 X 10⁵ hybridoma cells derived from immunized XenoMouse mice using a Fast-Track kit (Invitrogen). The generation of random primed cDNA was followed by PCR. Human V_{H} or human V_{K} family specific variable region primers (Marks et al., "Oligonucleotide primers for polymerase chain reaction amplication of human immunoglobulin variable genes and design of family-specific oligonucleotide probes", Eur. J. Immunol. 21:985-991 (1991)) or a universal human V_{H} primer, MG-30 (CAGGTGCAGCTGGAGCAGTCIGG) was used in conjunction with primers specific for the human Cγ2 constant region (MG-40d; 5'-GCTGAGGGAGTAGAGTCCTGAGGA-3') or Cκ constant region (hκP2; as previously described in Green et al., 1994). Sequences of human Mabs-derived heavy and kappa chain transcripts from hybridomas were obtained by direct sequencing of PCR products generated from poly(A⁺) RNA using the primers described above. PCR products were also cloned into pCRII using a TA cloning kit (Invitrogen) and both strands were sequenced using Prism dye-terminator sequencing kits and an ABI 377 sequencing machine. All sequences were analyzed by alignments to the "V BASE sequence directory" (Tomlinson et al., MRC Centre for Protein Engineering, Cambridge, UK) using MacVector and Geneworks software programs.

Further, each of the antibodies 4.1.1, 4.8.1, 11.2.1, and 6.1.1 were subjected to full length DNA sequencing. For such sequencing, Poly(A)⁺ mRNA was isolated from approximately 4 X 10⁶ hybridoma cells using mRNA Direct kit (Dynal). The RNA was reverse transcribed using oligo-dT(18) and the Advantage RT/PCR kit (Clonetech). The Variable region database (V Base) was used to design amplification primers beginning at the ATG start site of the heavy chain DP50 gene (5'-TATCTAAGCTCTAGACTCGACCGCCACCATGGAGTTTGGGCTGAGC TG-3') and to the stop codon of the IgG2 constant region (5'-TTCTCTGATCAGAATTCCTATCATTTACCCGGAGACAGGGAGAGCT-3'). An optimal Kozak sequence (ACCGCCACC) was added 5' to the ATG start site. The same method was used to design a primer to the ATG start site of the kappa chain A27 gene (5'-TCTTCAAGCTTGCCCGGGCCCGCCACCATGGAAACCCCAGCGCAG -3') and the stop codon of the kappa constant region (5'-TTCTTTGATCAGAATTCTCACTAACACTCTCCCCTGTTGAAGC-3'). The 012 cDNA was cloned by using a primer to the ATG start site (5'-TCTTCAAGCTTGCCCGGGCCCGCCACCATGGACATGAGGGTCCCCGC T-3) and the kappa constant region stop codon primer above. The heavy chain cDNAs were also cloned as genomic constructs by site directed mutagenesis to add an NheI site at the end of the variable J domain and subcloning an NheI-fragment containing the genomic IgG2 CH1/Hinge/CH2/CH3 regions. The point mutation to generate NheI site does not alter the amino acid sequence from germline. The primer pairs were used to amplify the cDNAs using Advantage High Fidelity PCR Kit (Clonetech). Sequence of the PCR was obtained, by direct sequencing using dye-terminator sequencing kits and an ABI sequencing machine. The PCR product was cloned into pEE glutamine synthetase mammalian expression vectors (Lonza) and three clones wee sequenced to confirm somatic mutations. For each clone, the sequence was verified on both strands in at least three reactions. An aglycosylated 4.1.1 antibody was generated by site directed mutagenesis of N294Q in the CH2 domain. Recombinant antibodies were produced by transient transfection of Cos7 cells in IgG-depleted FCS and purified using standard Protein A Sepharose techniques. Stable transfectants were generated by electroporation of murine NSO cells and selection in glutamine free media. Recombinant 4.1.1 with or without glycosylation exhibited identical specificity and affinity for CTLA-4 in the *in vitro* ELISA and BIAcore assays.

### Gene Utilization Analysis

The following Table sets forth the gene utilization evidenced by selected hybridoma clones of antibodies in accordance with the invention:

**TABLE II**

| **Heavy and Light Chain Gene Utilization** | | | | | | |
|---|---|---|---|---|---|---|
| | | | | | | |
| **Clone** | **Heavy Chain** | | | | **Kappa Light Chain** | |
| | **VH** | **D** | **JH** | | **VK** | **JK** |
| 4.1.1 | DP-50 | DIR4 or DIR3 | JH4 | | A27 | JK1 |
| 4.8.1 | DP-50 | 7-27 | JH4 | | A27 | JK4 |
| 4.14.3 | DP-50 | 7-27 | JH4 | | A27 | JK4 JK3 |
| 6.1.1 | DP-50 | DIR5 or DIR5rc | JH4 | | A27 | JK3 |
| 3.1.1. | DP-50 | 3-3 | JH6 | | 012 | JK3 |
| 4.10.2 | DP-50 | 7-27 | JH4 | | A27 | JK3 |
| 2.1.3 | DP-65 | 1-26 | JH6 | | A10/A26 | JK4 |
| 4.13.1 | DP-50 | 7-27 | JH4 | | A27 | JK3 |
| 11.2.1 | DP-50 | D1-26 | JH6 | | 012 | JK3 |
| 11.6.1 | DP-50 | D2-2 or D4 | JH6 | | 012 | JK3 |
| 11.7.1 | DP-50 | D3-22 or D21-9 | JH4 | | 012 | JK3 |
| 12.3.1.1 | DP-50 | D3-3 or DXP4 | JH6 | | A17 | JK1 |
| 12.9.1.1 | DP-50 | D6-19 | JH4 | | A3/A19 | JK4 |
| 4.9.1 | DP-47 | 5-24 and/or 6-19 | JH4 | | L5 | JK1 |

As will be observed, antibodies were generated with a strong bias towards the utilization of the DP-50 heavy chain variable region. The DP-50 gene is also referred to as a V_{H} 3-33 family gene. Only one antibody that was selected on the basis of CTLA-4 binding and preliminary *in vitro* functional assays showed a heavy chain gene utilization other than DP-50. That clone, 2.1.3, utilizes a DP-65 heavy chain variable region and is an IgG4 isotype. The DP-65 gene is also referred to as a V_{H} 4-31 family gene. On the other hand, the clone 4.9.1, which possesses a DP-47 heavy chain variable region binds to CTLA-4 but does not inhibit binding to B7-1 or B7-2. In XenoMouse mice, there are more than 30 distinct functional heavy chain variable genes with which to generate antibodies. Bias, therefore, is indicative of a preferred binding motif of the antibody-antigen interaction with respect to the combined properties of binding to the antigen and functional activity.

### Mutation Analysis

As will be appreciated, gene utilization analysis provides only a limited overview of antibody structure. As the B cells in XenoMouse animals stochastically generate V-D-J heavy or V-J kappa light chain transcripts, there are a number of secondary processes that occur, including, without limitation, somatic hypermutations, n-additions, and CDR3 extensions. *See, for example,* Mendez et al., Nature Genetics 15:146-156 (1997), and U.S. Patent Application Serial No. 08/759,620, filed December 3, 1996. Accordingly, to further examine antibody structure predicted amino acid sequences of the antibodies were generated from the cDNAs obtained from the clones. In addition, N-terminal amino acid sequences were obtained through protein sequencing.

Figure 1 provides nucleotide and predicted amino acid sequences of the heavy and kappa light chains of the clones 4.1.1 (Figure 1A), 4.8.1 (Figure 1B), 4.14.3 (Figure 1C), 6.1.1 (Figure 1D), 3.1.1 (Figure 1E), 4.10.2 (Figure 1F), 2.1.3 (Figure 1G), 4.13.1 (Figure 1H), 11.2.1 (Figure 1I), 11.6.1 (Figure 1J), 11.7.1 (Figure 1K), 12.3.1.1 (Figure IL), and 12.9.1.1 (Figure 1M). In Figures 1A, 1B, and 1D, extended sequences of the antibodies 4.1.1, 4.8.1, and 6.1.1 were obtained by full length cloning of the cDNAs as described above. In such Figures, the signal peptide sequence (or the bases encoding the same) are indicated in bold and sequences utilized for the 5' PCR reaction are underlined.

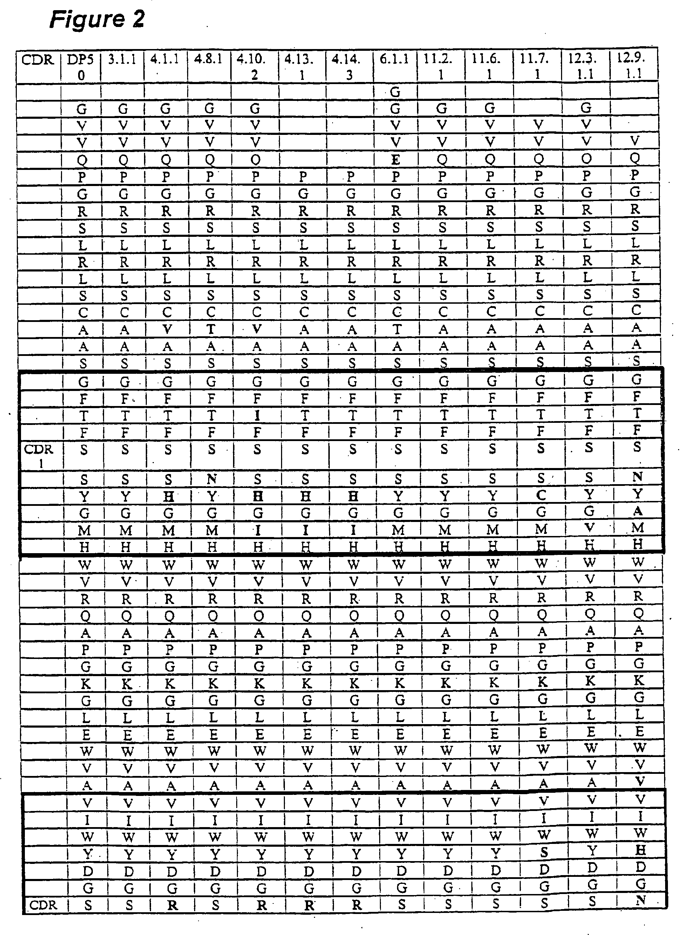

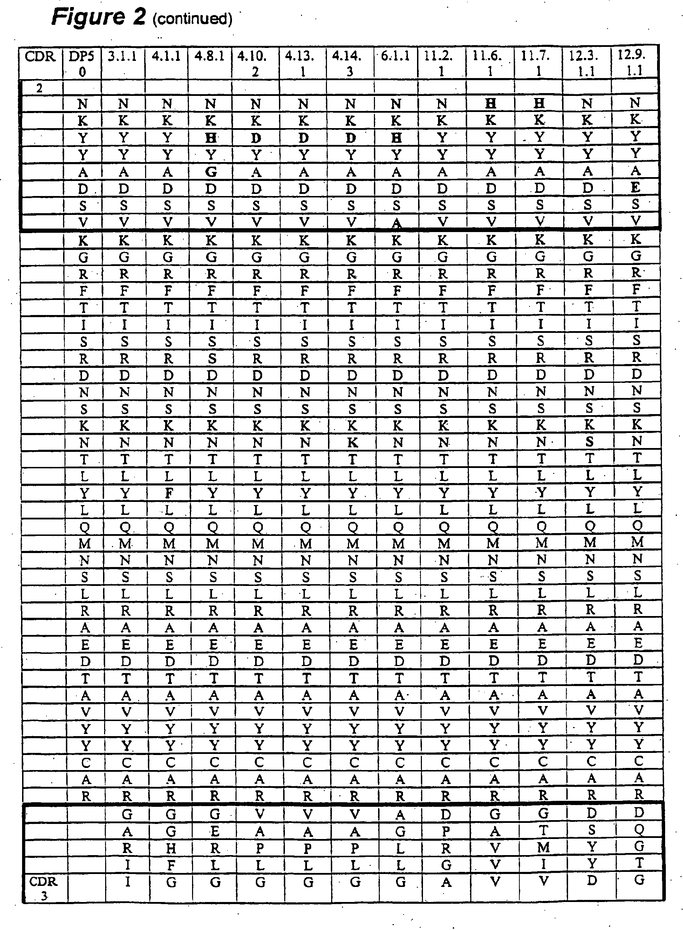

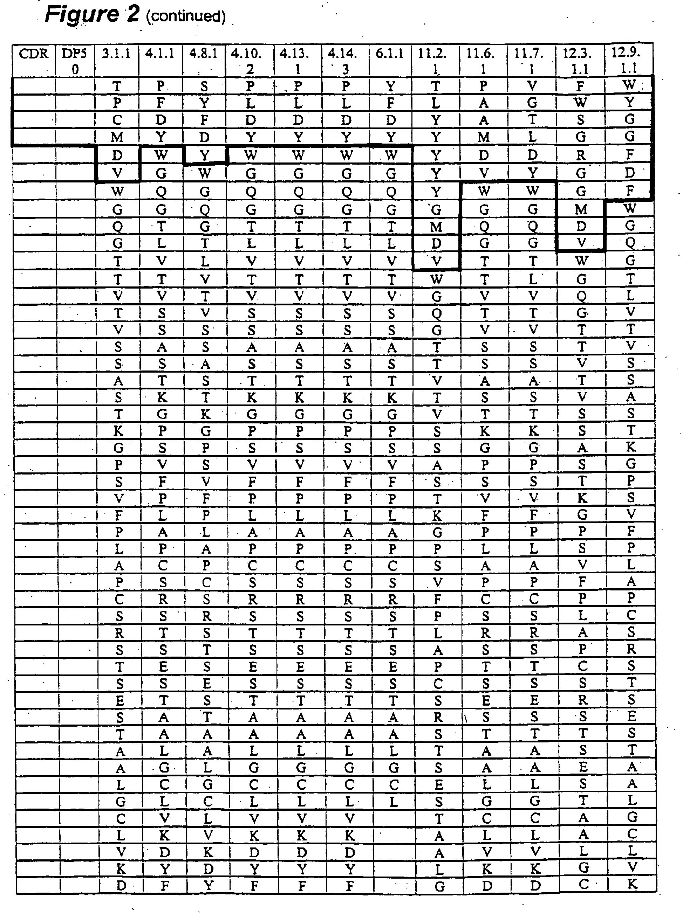

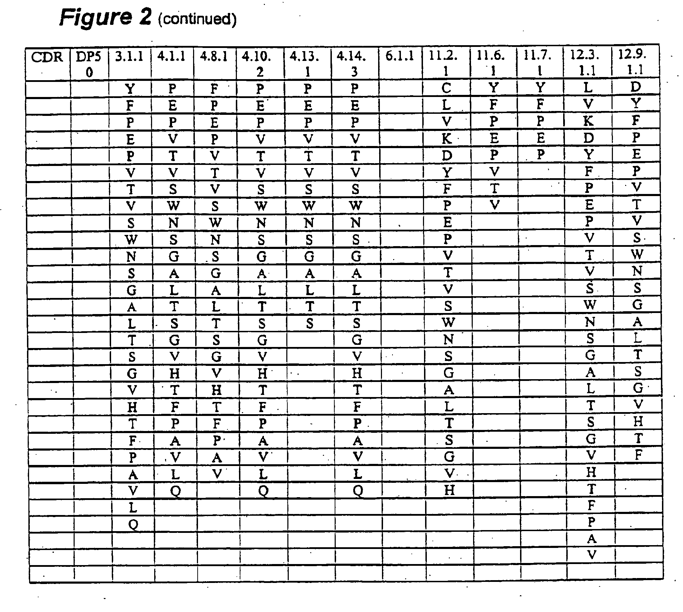

Figure 2 provides a sequence alignment between the predicted heavy chain amino acid sequences from the clones 4.1.1, 4.8.1, 4.14.3, 6.1.1, 3.1.1, 4.10.2, 4.13.1, 11.2.1, 11.6.1, 11.7.1, 12.3.1.1, and 12.9.1.1 and the germline DP-50 (3-33) amino acid sequence. Differences between the DP-50 germline sequence and that of the sequence in the clones are indicated in bold. The Figure also shows the positions of the CDR1, CDR2, and CDR3 sequences of the antibodies as shaded.

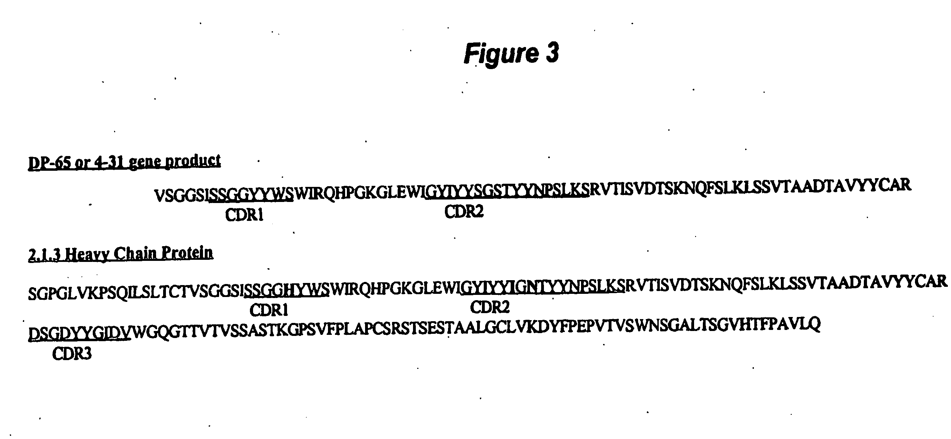

Figures 3 provides a sequence alignment between the predicted heavy chain amino acid sequence of the clone 2.1.3 and the germline DP-65 (4-31) amino acid sequence. Differences between the DP-65 germline sequence and that of the sequence in the clone are indicated in bold. The Figure also shows the positions of the CDR1, CDR2, and CDR3 sequences of the antibody as underlined.

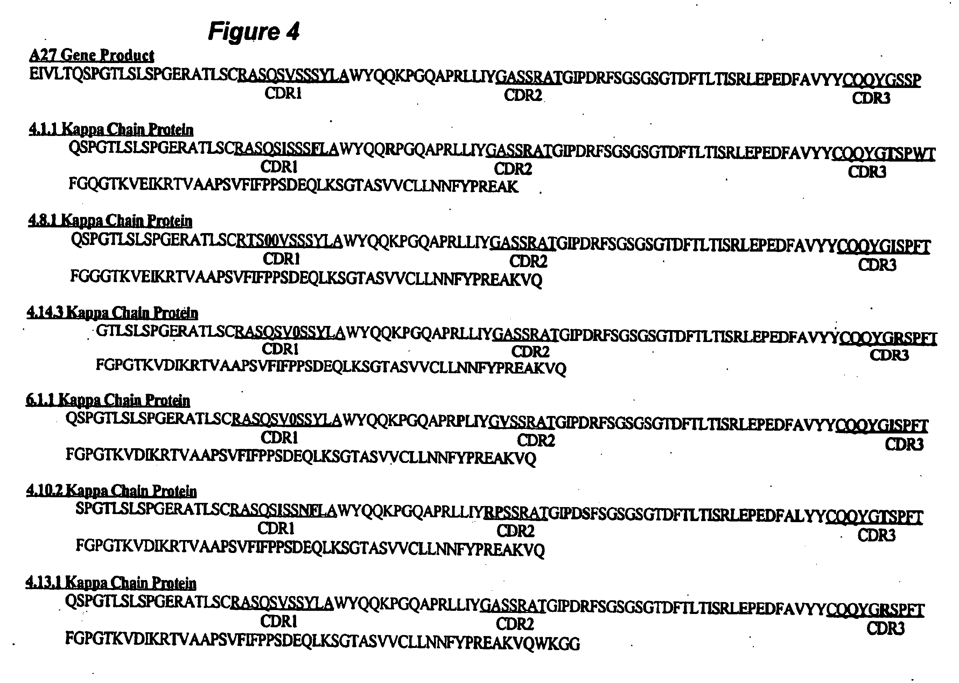

Figure 4 provides a sequence alignment between the predicted kappa light chain amino acid sequence of the clones 4.1.1, 4.8.1, 4.14.3, 6.1.1, 4.10.2, and 4.13.1 and the germline A27 amino acid sequence. Differences between the A27 germline sequence and that of the sequence in the clone are indicated in bold. The Figure also shows the positions of the CDR1, CDR2, and CDR3 sequences of the antibody as underlined. Apparent deletions in the CDR1s of clones 4.8.1, 4.14.3, and 6.1.1 are indicated with "0s".

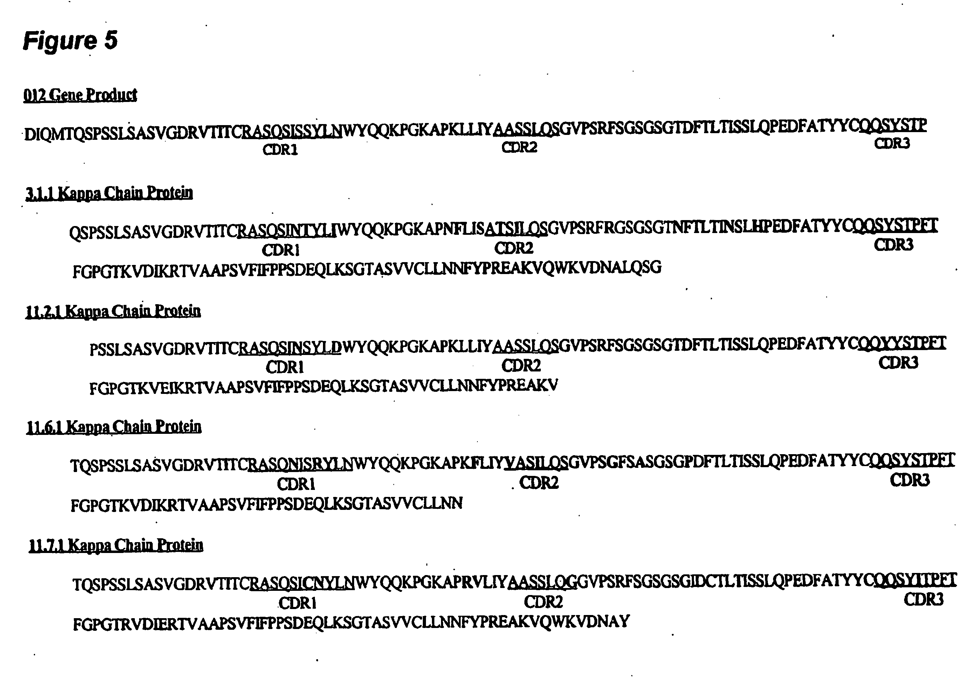

Figure 5 provides a sequence alignment between the predicted kappa light chain amino acid sequence of the clones 3.1.1, 11.2.1, 11.6.1, and 11.7.1 and the germline 012 amino acid sequence. Differences between the 012 germline sequence and that of the sequence in the clone are indicated in bold. The Figure also shows the positions of the CDR1, CDR2, and CDR3 sequences of the antibody as underlined.

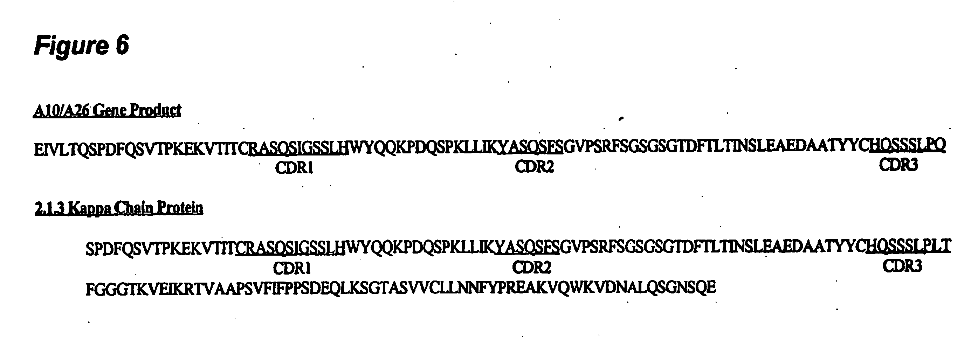

Figure 6 provides a sequence alignment between the predicted kappa light chain amino acid sequence of the clone 2.1.3 and the germline A10/A26 amino acid sequence. Differences between the A10/A26 germline sequence and that of the sequence in the clone are indicated in bold. The Figure also shows the positions of the CDR1, CDR2, and CDR3 sequences of the antibody as underlined.

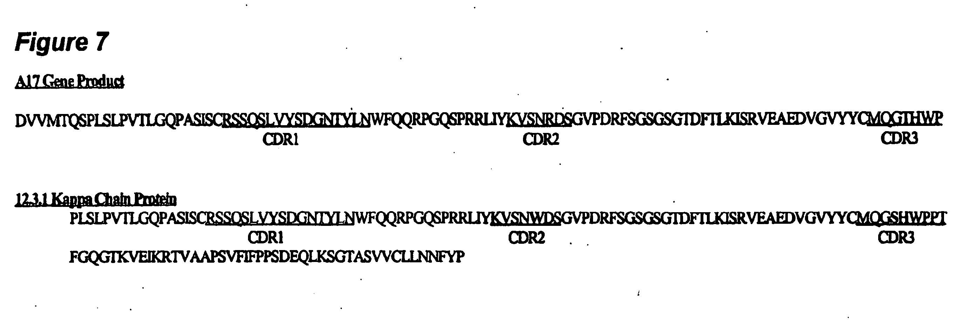

Figure 7 provides a sequence alignment between the predicted kappa light chain amino acid sequence of the clone 12.3.1 and the germline A17 amino acid sequence. Differences between the A17 germline sequence and that of the sequence in the clone are indicated in bold. The Figure also shows the positions of the CDR1, CDR2, and CDR3 sequences of the antibody as underlined.

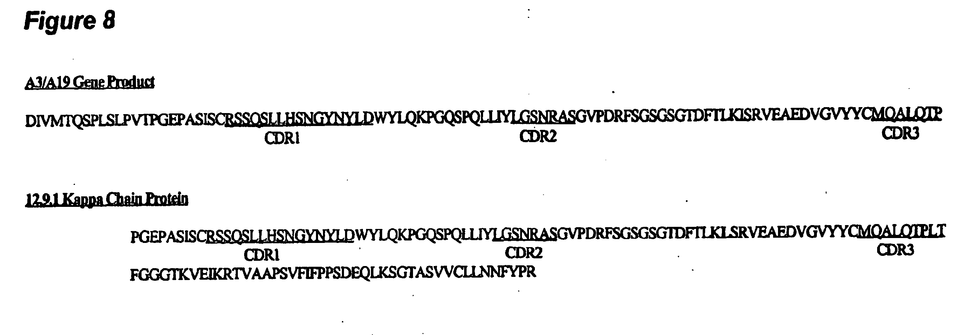

Figure 8 provides a sequence alignment between the predicted kappa light chain amino acid sequence of the clone 12.9.1 and the germline A3/A19 amino acid sequence. Differences between the A3/A19 germline sequence and that of the sequence in the clone are indicated in bold. The Figure also shows the positions of the CDR1, CDR2, and CDR3 sequences of the antibody as underlined.

Figure 22 provides a series of additional nucleotide and amino acid sequences of the following anti-CTLA-4 antibody chains:
*4.1.1*:
   full length 4.1.1 heavy chain (cDNA 22(a), genomic 22(b), and amino acid 22(c));
   full length aglycosylated 4.1.1 heavy chain (cDNA 22(d) and amino acid 22(e));
   4.1.1 light chain (cDNA 22(f) and amino acid 22(g));
*4.8.1:*
   full length 4.8.1 heavy chain (cDNA 22(h) and amino acid 22(i));
   4.8.1 light chain (cDNA 22(j) and amino acid 22(k));
*6.1.1:*
   full length 6.1.1 heavy chain (cDNA 22(1) and amino acid 22(m));
   6.1.1 light chain (cDNA 22(n) and amino acid 22(o));
*11.2.1:*
   full length 11.2.1 heavy chain (cDNA 22(p) and amino acid 22(q)); and
   11.2.1 light chain (cDNA 22 (r) and amino acid 22(s)).

Signal peptide sequences are shown in bold and large text. The open reading frames in the full length 4.1.1 genomic DNA sequence (Fig. 22(b)) are underlined. And, the mutations introduced to make the aglycosylated 4.1.1 heavy chain and the resulting change (N294Q) are shown in doble underline and bold text (cDNA (Fig. 22(b)) and amino acid (Fig. 22(c)).

### EXAMPLE 4

### Analysis of Heavy and Light Chain Amino Acid Substitutions

In Figure 2, which provides a sequence alignment between the predicted heavy chain amino acid sequences from the clones 4.1.1, 4.8.1, 4.14.3, 6.1.1, 3.1.1, 4.10.2, 4.13.1, 11.2.1, 11.6.1, 11.7.1, 12.3.1.1, and 12.9.1.1 and the germline DP-50 (3-33) amino acid sequence, an interesting pattern emerges. In addition to the fact of the bias for heavy chain DP-50 in the majority of the clones, there is relatively limited hypermutation in the antibodies relative to the germline DP-50 gene. For example, clones 3.1.1 and 11.2.1 have no mutations. Moreover, the mutations in the other clones are generally conservative changes, involving substitutions of amino acids with similar properties to the amino acids in the germline. Mutations within many of the CDR1 and CDR2 sequences are particularly conservative in nature. Three of the heavy chains represented in Figure 2, 4.10.2, 4.13.1, and 4.14.3, are clearly derived from a single recombination event (i.e., derive from an identical terminal center) and are nearly identical in sequence. If these three are considered as a single sequence, then, among the 10 different antibodies containing the DP-50 heavy chain, in CDR1 and CDR2 there are 3 positions in which a nonpolar residue is replaced by another nonpolar residue, 12 in which a polar uncharged residue is replaced by another polar uncharged residue, and 1 in which a polar charged residue is replaced by another polar charged residue. Further, there are two positions in which two residues which are very similar structurally, glycine and alanine, are substituted for one another. The only mutations not strictly conservative involve 3 substitutions of a polar charged residue for a polar uncharged residue and one substitution of a nonpolar residue for a polar residue.

The light chains of these antibodies are derived from 5 different Vk genes. The A27 gene is the most heavily represented and is the source of 6 different light chains. Comparison of these 6 sequences reveals two noteworthy features. First, three of them, 4.8.1, 4.14.3, and 6.1.1, contain deletions of one or two residues in CDR1, a rare event. Second, there is a strong prejudice against the germline serine at position six in CDR3 in that the serine has been replaced in every sequence. This suggests that a serine at this position is incompatible with CTLA-4 binding.

It will be appreciated that many of the above-identified amino acid substitutions exist in close proximity to or within a CDR. Such substitutions would appear to bear some effect upon the binding' of the antibody to the CTLA-4 molecule. Further, such substitutions could have significant effect upon the affinity of the antibodies.

### EXAMPLE 5

### N-Terminal Amino Acid Sequence Analysis of Antibodies in Accordance with the Invention

In order to further verify the composition and structure of the antibodies in accordance with the invention, identified above, we sequenced certain of the antibodies using a Perkin-Elmer sequencer. Both heavy and kappa light chains of the antibodies were isolated and purified through use of preparative gel electrophoresis and electroblotting techniques and thereafter directly sequenced as described in Example 6. A majority of the heavy chain sequences were blocked on their amino terminus. Therefore, such antibodies were first treated with pyroglutamate aminopeptidase and thereafter sequenced.

The results from this experiment are shown in Figure 9. Figure 9 also provides the molecular weight of the heavy and light chains as determined by mass spectroscopy (MALDI).

### EXAMPLE 6

### Additional Characterization of Antibodies

Figure 10 provides certain additional characterizing information about certain antibodies. In the Figure, data related to clones 3.1.1, 4.1.1, 4.8.1, 4.10.2, 4.14.3, and 6.1.1 is summarized. The following data is provided: Concentration, isoelectric focusing (IEF), SDS-PAGE, size exclusion chromatography, FACS, mass spectroscopy (MALDI), and light chain N-termimal sequences.

Generally, the data was generated as follows:

### Materials and Methods

Protein concentration was determined at 280 nm from a UV scan (200-350 nm), where 1.58 absorbance units at 280 nm equaled 1 mg/ml.

SDS-PAGE was performed using the Novex NuPAGE electrophoresis system with a 10% NuPAGE gel and MES running buffer. Samples were prepared by diluting 3:1 with 4x NuPAGE sample buffer (+/-beta-mercaptoethanol) heated and ~ 5 ug of protein was loaded onto the gel. The gel was then stained with Brilliant Blue R staining solution (Sigma, Cat # B-6529) and molecular size estimates were made by comparing stained bands to "Perfect Protein Markers" Novagen, Cat# 69149-3).

For N-terminal sequencing, samples were run as above on NuPAGE gels, transferred to Pro Blot immobilization membrane (Applied Biosystems) then stained with Coomassie Blue R-250. The stained protein bands were excised and subjected to sequence analysis by automated Edman degradation on an Applied Biosystems 494 Precise HT Sequencer.

Isoelectric focusing (IEF) was performed using Pharmacia IEF 3-9 Phast Gels (Cat# 17-0543-01). Samples were diluted in 10% glycerol to -0.8 mg/ml and 1ul was loaded onto the gel and then silver stained. The pI estimates were made by comparing stained bands to broad range (pH3-10) IEF standards (Pharmacia, Cat, # 17-0471-01)

Size exclusion chromatography (SEC) was carried out in phosphate buffered saline (PBS) on the Pharmacia SMART system using the Superdex 75 PC 3.2/30 column. Molecular size estimates were made by comparing peak retention time to the retention times in the gel.

For FACS studies, human peripheral T cells were prepared and stimulated for 48 hours. T cells were washed once, resuspended in FACS buffer at 1x10⁶ cells/100 ul and stained for CD3 surface expression with 10 ul of anti-CD3-FITC (Immunotech, Marseille, France) for 30 minutes at room temperature. Cells were washed twice, then fixed, permeabilized (Fix and Perm, Caltag), and stained for intracellular CTLA-4 expression with 10 ul anti-CD152-PE (Pharmingen). Flow cytometry was performed using a Becton Dickinson FACSort. Quadrants were set by analysis of relevant isotype control antibodies (Caltag).

As was discussed above, anti-CTLA-4 antibodies have been demonstrated to possess certain powerful immune modulation activities. The following experiments were carried out in order to determine if antibodies in accordance with the present invention possessed such activities. In general, the experiments were designed to assess the ability of the antibodies to inhibit the interaction between CTLA-4 and B7 molecules, be selective as between CTLA-4 and B7 molecules and CD28, and promote T cell cytokine production, including, but not limited to IL-2 and/or IFN-γ expression. Further, examination of cross-reactivity of antibodies of the invention with certain human tissues and CTLA-4 molecules in other species (e.g., mouse and primate) was undertaken.

### EXAMPLE 7

### Competition ELISA: Inhibition of CTLA-4/B7-1 or B7-2 Interaction by Antibodies in Accordance with the Invention

An *in vitro* assay was conducted to determine if antibodies in accordance with the present invention were capable of inhibiting the binding of CTLA-4 with either B7-1 or B7-2. As will be appreciated, antibodies of the invention that are capable of inhibiting the binding of CTLA-4 with B7 molecules would be expected to be candidates for immune regulation through the CTLA-4 pathway. In the assay, the following materials and methods were utilized:

### Materials and Methods

3 nM B7-1-Ig(G1) or B7-2-Ig(G1) (Repligen, Inc., Needham, MA) in Dulbecco's PBS was coated on 96-well MaxiSorp plates (Nunc, Denmark, #439454) and incubated at 4°C overnight. On day 2, B7-Ig was removed and plates were blocked with 1% BSA plus 0.05% Tween-20 in D-PBS for two hours. Plates were washed 3X with wash buffer (0.05% Tween-20 in D-PBS). Antibody at appropriate test concentrations and CTLA-4-Ig(G4) (0.3 nM final conc.) (Repligen, Inc., Needham, MA) were pre-mixed for 15 minutes and then added to the B7-Ig coated plate (60 ul total volume) and incubated at RT for 1.5 hours. Plates were washed 3X and 50 µl of a 1 to 1000 dilution of HRP-labeled mouse anti-human IgG4 antibody (Zymed, San Francisco, CA, #05-3820) was added and incubated at RT for 1 hour. Plates were washed 3X and 50 µl TMB Microwell peroxidase substrate (Kirkegaard & Perry, Gaithersburg, MD, #50-76-04) was added and incubated at RT for 20 minutes, an then 50 µl 1N H₂SO₄ was added to the plates. Plates were read at 450 nm using a Molecular Devices plate reader (Sunnyvale, CA). All samples were tested in duplicate. Maximal signal was defined as CTLA-4-Ig binding in the absence of test antibody. Non-specific binding was defined as absorbance in the absence of CTLA-4-4-Ig and test antibody.

The results from the assay are provided in Table IIIA and IIIB. In Table IIIA. results are shown for a variety of antibodies. In Table IIIB, results are shown comparing the 4.1.1 antibody with the 11.2.1 antibody of the invention from a separate experiment.

**TABLE IIIA**

| Clone | Isotype | CTLA-4/B7.2 | CTLA-4/B7.1 |
|---|---|---|---|
| CTLA-4-Ig | | Comp. ELISA | Comp. ELISA |
| | | IC50 (nM) | IC50 (nM) |
| CT3.1.1 | IgG2 | 0.45 ± 0.07 (n=3) | 0.63 ± 0.10 (n=2) |
| CT4.1.1 | IgG2 | 038 ± 0.06 (n=5) | 0.50 ± 0.05 (n=2) |
| CT4.8.1 | IgG2 | 0.57 ± 0.03 (n=3) | 0.17 ± 0.28 (n=2) |
| CT4.9.1 | IgG2 | Non-competitive (n=3) | non-competitive (n=2) |
| CT4.10.2 | IgG2 | 1.50 ± 037 (n=3) | 3.39 ± 0.31 (n=2) |
| CT4.13.1 | IgG2 | 0.49 ± 0.05 (n=3) | 0.98 ± 0.11 (n=2) |
| CT4.14.3 | IgG2 | 0.69 ± 0.11 (n=3) | 1.04 ± 0.15 (n=2) |
| CT6.1.1 | IgG2 | 0.39 ± 0.06 (n=3) | 0.67 ± 0.07 (n=2) |

**TABLE IIIB**

| Clone | Isotype | CTLA-4/B7.2 | CTLA-4/B7.1 |
|---|---|---|---|
| CTLA-4-Ig | | Comp. ELISA | Comp. ELISA |
| | | IC50 (nM) | IC50 (nM) |
| CT4.1.1 | IgG2 | 0.55 ± 0.08 (n=4) | 0.87 ± 0.14 (n=2) |
| CT11.2.1 | IgG2 | 0.56 ± 0.05 (n=4) | 0.81 ± 0.24 (n=2) |

### EXAMPLE 8

### Selectivity Ratios of Antibodies of the_Invention with Respect to CTLA-4 Versus Either CD28 or B7-2

Another *in vitro* assay was conducted to determine the selectivity of antibodies of the invention with respect to CTLA-4 versus either CD28 or B7-2. The following materials and methods were utilized in connection with the experiments:

### CTLA-4 Selectivity ELISA: Materials and Methods

A 96-well Fluro-NUNC plate (Nunc, Cat. No.475515) was coated with four antigens: CTLA-4/Ig, CD44/Ig, CD28/Ig, and B7-2/Ig (antigens generated in-house). The antigens were platecoated overnight at +4°C at 1 ug/ml 100ul/well in 0.1 M sodium bicarbonate buffer, pH 9.6. The plate was then washed with PBST (PBS + 0.1 % Tween-20) three times using a NUNC plate washer. The plate was blocked with PBST+0.5%BSA at 150 ul/well. The plate was incubated at RT for 1 hour, then washed with PBST three times. Next the anti-CTLA-4 antibodies of the invention were diluted in block at 1 µg/ml and were added to the plate. The plate was incubated at RT for 1 hour then washed with PBST three times. The wells that contained the antibodies of the invention were then treated with 100 µl/well anti-human IgG2-HRP (Southern Biotech Cat No.9070-05) at a 1:4000 dilution in block. Also, one row was treated with anti-human IgG (Jackson Cat No. 209-035-088) to normalize for platecoating. This antibody was diluted to 1:5000 in block and added at 100 ul/well. Also, one row was treated with anti-human CTLA-4-HRP (Pharmingen Cat No. 345815/Custom HRP conjugated) as a positive control. This antibody was used at 0.05 ug/ml diluted in block. The plate was incubated at RT for 1 hour then washed with PBST three times. LBA chemiluminescent substrate (Pierce) was added at 100 µl/well and the plate was incubated on a plateshaker for 5 min. The plate was then read using a lumi-imager for a 2 min. exposure.

### IGEN CTLA-4-Ig Selectivity Binding Assay: Materials and Methods

M-450 Dynabeads (Dynal A.S, Oslo, Norway, #140.02) were washed 3X with Na phosphate buffer, pH 7.4, and resuspended in Na-phosphate buffer. 1.0 µg CTLA-4-Ig(G1), 1.0 µg CD28-Ig(G1) or 1.0 to 3.0 µg B7-2-Ig(G1) (Repligen, Inc., Needham, MA) were added to 100 µl of beads and incubated overnight on a rotator at 4°C. On day 2 the beads were washed 3X in 1% BSA plus 0.05% Tween-20 in Dulbecco's PBS and blocked for 30 minutes. Beads were diluted 1 to 10 with blocking buffer and 25 µl of the coated beads were added to 12×75 mm polypropylene tubes. All samples were tested in duplicate. 50 µl test antibody (1 µg/ml final concentration) or blocking buffer was added to the tubes and incubated for 30 minutes on the Origen 1.5 Analyzer carousel (IGEN International, Inc., Gaithersburg, MD) at RT, while vortexing at 100 rpm. 25 µl of ruthenylated murine anti-human IgG1, IgG2 or IgG4 (Zymed, Inc., San Francisco, CA,#05-3300, 05-3500 and 05-3800) (final concentration of 3 µg/ml in 100 µl total volume) was added to the tubes. Tubes were incubated for 30 minutes at RT on the carousel while vortexing at 100 rpm. 200 µl of Origen assay buffer (IGEN International, Inc., Gaithersburg, MD, #402-050-03) per tube was added and briefly vortexed and then the tubes were counted in the Origen Analyzer and ECL (electrochemiluminescence) units were determined for each tube. Normalization factors were determined to correct for differences in binding of fusion proteins to Dynabeads, and ECL units were corrected for non-specific binding before calculating selectivity ratios.

The results from the assays are provided in Tables IVA and IVB.

**TABLE IVA**

| Clone | Isotype | CTLA-4/CD28 | CTLA-4/B7.2 | CTLA-4/CD44 | CTLA-4/CD28 | CTLA-4/B7.2 |
|---|---|---|---|---|---|---|
| | | ELISA | ELISA | ELISA | IGEN | IGEN |
| 3.1.1 | IgG2 | >500:1 (n=3) | >500:1 (n=3) | >500:1 (n=3) | >500:1 (n=2) | >500:1 (n=1) |
| | | | | | | 195:1 (n=1) |
| 4.1.1 | IgG2 | >500:1 (n=3) | >500:1 (n=2) | >500:1 (n=3) | >500:1 (n=1) | >500:1 (n=1) |
| | | | 485:1 (n=1) | | 261:1 (n=1) | 107:1 (n=1) |
| 4.8.1 | IgG2 | >500:1 (n=3) | >500:1 (n=2) | >500:1 (n=3) | >500:1 (n=2) | >500:1 (n=2) |
| | | | 190:1 (n=1) | | | |
| 4.9.1 | IgG2 | >500:1 (n=2) | >500:1 (n=2) | >500:1 (n=3) | >500:1 (n=1) | >500:1 (n=1) |
| | | 244:1 (n=1) | 33:1 (n=1) | | | |
| 4.10.2 | IgG2 | >500:1 (n=3) | >500:1 (n=3) | >500:1 (n=3) | >500:1 (n=1) | >500:1 (n=1) |
| 4.13.1 | IgG2 | >500:1 (n=2) | >500:1 (n=3) | >500:1 (n=3) | >500:1 (n=1) | >500:1 (n=2) |
| | | 46:1 (n=1) | | | 329:1 (n=1) | |
| 4.14.3 | IgG2 | >500:1 (n=2) | >500:1 (n=2) | >500:1 (n=2) | >413:1 (n=1) | >234:1 (n=1) |
| | | 80:1 (n=1) | 10:1 (n=1) | 126:1 (n=1) | | |
| 6.1.1 | IgG2 | >500:1 (n=2) | >500:1 (n=3) | >500:1 (n=3) | >500:1 (n=2) | >500:1 (n=2) |
| | | 52:1 (n=1) | | | | |

**TABLE IVB**

| Clone | Isotype | CTLA4/CD26 | CTLA4/B7-2 | CTLA-4/hIgG |
|---|---|---|---|---|
| | | ELISA | ELISA | ELISA |
| 4.1.1 | IgG2 | >500:1 (n=3) | >500:1 (n-2) | >500:1 (n=3) |
| 11.2.1 | IgG2 | >500:1 (n=3) | >500:1 (n=3) | >500:1 (n=3) |

### EXAMPLE 9

### Human T-Cell Signal Model

In order to further define the activity of antibodies in accordance with the invention to act as immune regulators, we developed certain T cell assays in order to quantify the enhancement of T cell IL-2 production upon blockade of CTLA-4 signal with the antibodies. The following materials and methods were utilized in connection with the experiments:

### Materials and Methods

Freshly isolated human T cells were prepared by using Histopaque (Sigma, St. Louis, MO, #A-70543) and T-Kwik (Lympho-Kwik, One Lambda, Canoga Park, CA, #LK-50-T), and stimulated with PHA (1 µg/ml) (purified Phytohemagglutinin, Murex Diagnostics, Ltd., Dartford, England, #HA 16) in medium (RPMI-1640 containing L-glutamine, MEM non-essential amino acids, penicillin, streptomycin, 25 mM HEPES and 10% FBS) at a concentration of 1x10⁶ cells/ml and incubated at 37°C for 2 days. The cells were washed and diluted in medium to 2x10⁶ cells/ml. Raji cells (Burkitt lymphoma, Human ATCC No.: CCL 86 Class II, American Type Culture Collection, Rockville, MD) were treated with Mitomycin C (Sigma, St. Louis, MO, # M-4287) (25 µg/ml) for one hour at 37°C. The Raji cells were washed 4X in PBS and resuspended at 2x10⁶ cells/ml. Human T cell blasts (5x10⁵/ml), Raji cells (5x10⁵/ml) and anti-CTLA-4 antibodies or an isotyped-matched control antibody at various concentrations were added to 96-well microtiter plates and the plates were incubated at 37°C for 72 hours. Total volume per well was 200 µl. Seventy-two hours post stimulation, the plates were spun down, and the supernatant was removed and frozen for later determination of IL-2 (Quantikine IL-2 ELISA Kit, R&D Systems, Minneapolis, MN, #D2050) and IFN-y (Quantikine IFN-γ ELISA Kit, R&D Systems). Cytokine enhancement was defined as the difference between cytokine levels in cultures containing an anti-CTLA-4 blocking mAb versus an isotype-matched control antibody. For flow cytometry experiments, Raji cells were washed 1x with FACS buffer (PBS containing 2% heat inactivated FCS, 0.025% sodium azide). Cell pellets were resuspended in FACS buffer at 1x10⁶ cells/100 µl and incubated with 10 µl of anti-CD80-PE (Becton Dickinson, Sam Jose, CA) or anti-CD86-PE (Pharmingen, San Diego, CA) for 30 minutes at room temperature. Cells were washed twice and resuspended in 1 ml FACS buffer. Flow cytometry was performed using a Becton Dickinson FACSort. Histogram markers were set by analysis of relevant isotype control antibodies (Caltag, Burlingame, CA).

In general, we have developed an assay that can be used for rapid determination of T-cell IL-2 up-regulation. As will be appreciated, stimulation of T cells is B7 and CD28 dependent. Further, washed T blasts do not make detectable IL-2, and Raji cells do not make detectable IL-2 even when stimulated with LPS or PWM. However, in combination, the T blasts co-cultured with Raji cells can model B7, CTLA-4, and CD28 signaling events and the effects of antibodies thereon can be assessed.

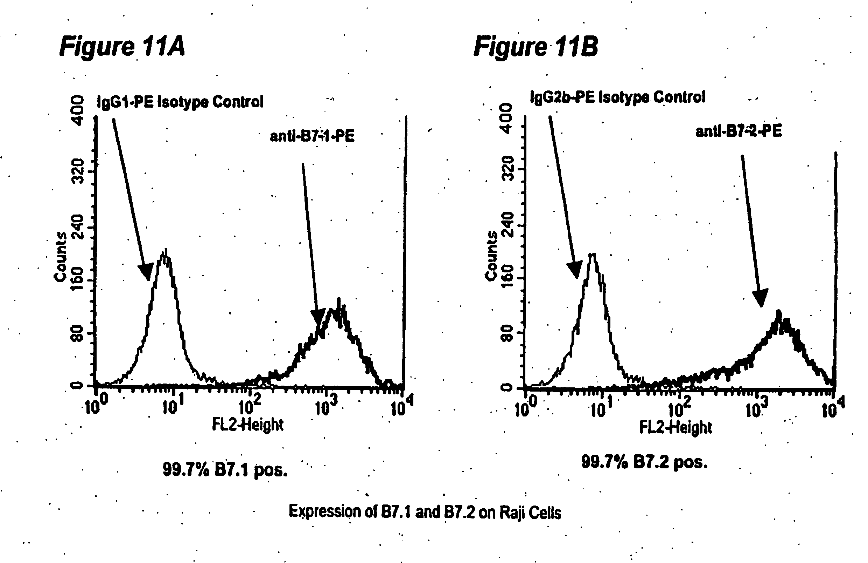

Figure 11 shows the expression of B7-1 and B7-2 on Raji cells using anti-CD80-PE and anti-CD86-PE mAbs using flow cytometry (FACS) as described in Example 6.

Figure 12 shows the concentration dependent enhancement of IL-2 production in the T cell blast/Raji assay induced by CTLA-4 blocking antibodies (BNI3 (Pharmingen), and the 4.1.1, 4.8.1, and 6.1.1 antibodies of the invention).

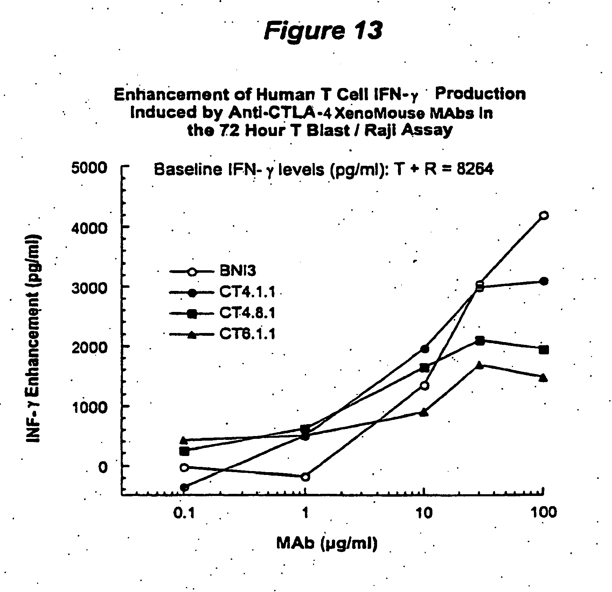

Figure 13 shows the concentration dependent enhancement of IFN-y production in the T cell blast/Raji assay induced by CTLA-4 blocking antibodies (BNI3 (PharMingen) and the 4.1.1, 4.8.1, and 6.1.1 antibodies of the invention) (same donor T cells).

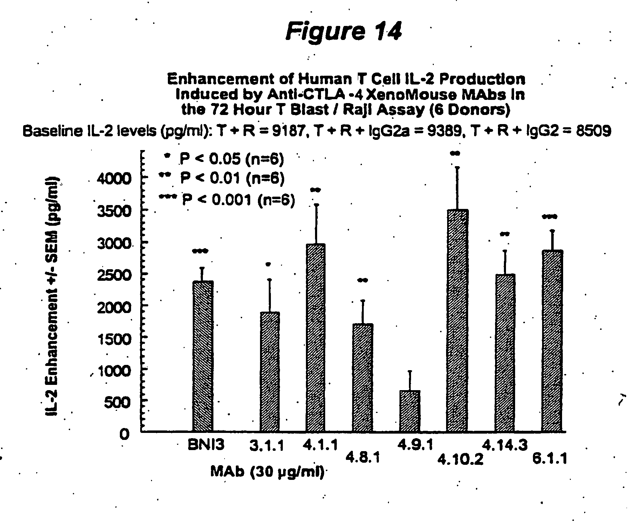

Figure 14 shows the mean enhancement of IL-2 production in T cells from 6 donors induced by CTLA-4 blocking antibodies in the T cell blast/Raji assay. It is interesting to consider that the mAb, CT4.9.1, binds..to CTLA-4 but does not block B7 binding. Thus, simply binding to CTLA-4 is insufficient by itself to provide a functional antibody of the invention.

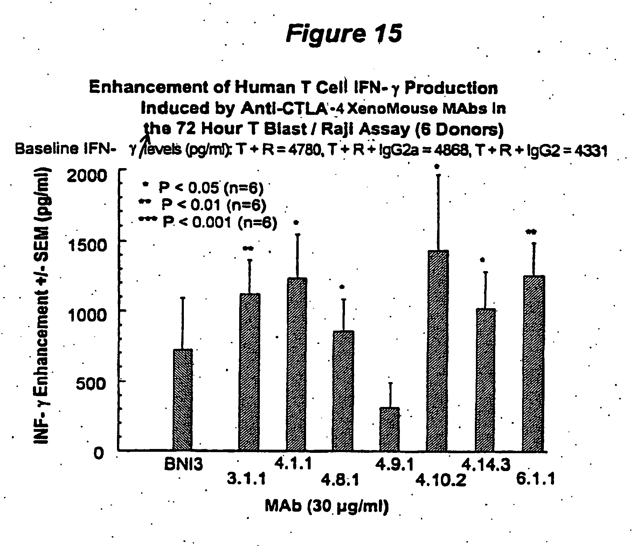

Figure 15 shows the mean enhancement of IFN-γ production in T cells from 6 donors induced by CTLA-4 blocking antibodies in the T cell blast/Raji assay.

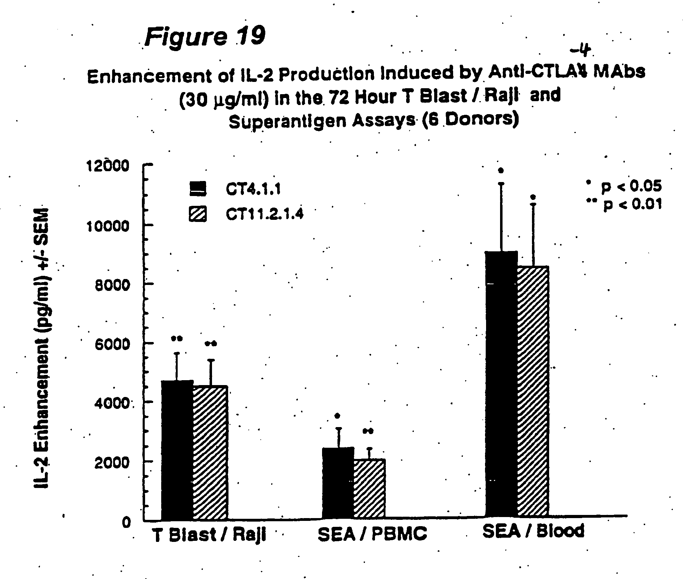

Figure 19 shows a comparison between the 4.1.1 and 11.2.1 antibodies of the invention at a concentration of 30µg/ml in the 72 hour T cell blast/Raji assay, as described in this Example 9, and the Superantigen assay described in Example 10.

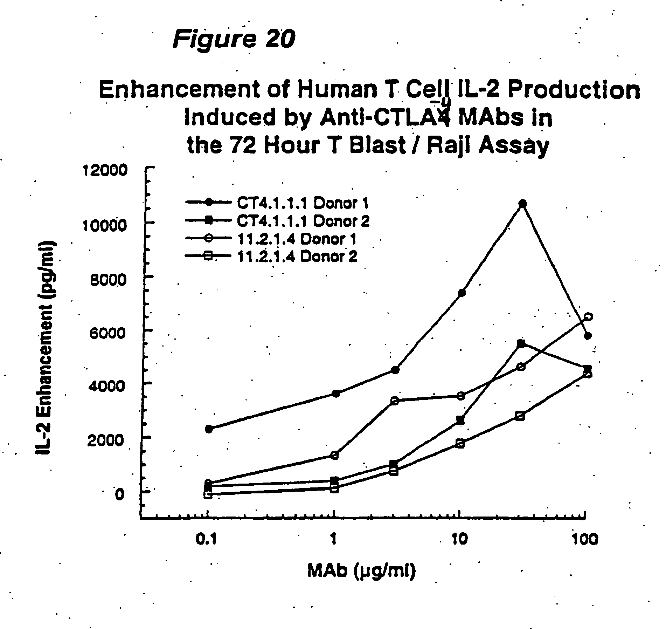

Figure 20 shows the concentration dependent enhancement of IL-2 production in the T cell blast/Raji assay induced by the 4.1.1 and 11.2.1 CTLA-4 antibodies of the invention.

The following Table IVc provides information related to mean enhancement and range of enhancement of cytokine response in the Raji and SEA assays of the invention. Each of the experiments included in the results are based on antibody at a dose of 30 µg/ml and measured at 72 hours. Numbers of donors used in the experiments as well as responses are shown.

**TABLE IVC**

| Assay | mAb | Cytokine | Mean Enhancement | SEM | Range Enhancement | n | Donor Response |
|---|---|---|---|---|---|---|---|
| | | | pg/ml | | pg/ml | | |
| T cell blast/Raji | 4.1.1 | IL-2 | 3329 | 408 | 0 to 8861 | 42 | 19 of 21 |
| T cell blast/Raji | 4.1.1 | IFN-γ | 3630 | 980 | 600 to 13939 | 17 | 13 of 13 |
| T cell blast/Raji | 11.2.1 | IL-2 | 3509 | 488 | 369 to 6424 | 18 | 14 of 14 |
| SEA (PBMC) | 4.1.1 | IL-2 | 2800 | 312 | 330 to 6699 | 42 | 17 of 17 |
| SEA (PBMC) | 11.2.1 | IL-2 | 2438 | 366 | 147 to 8360 | 25 | 15 of 15 |
| SEA | 4.1.1 | IL-2 | 6089 | 665 | -168 to 18417 | 46 | 15 of 17 |
| (Whole Blood) | | | | | | | |
| SEA | 11.2.1 | IL-2 | 6935 | 700 | -111 to 11803 | 25 | 12 of 14 |
| (Whole Blood) | | | | | | | |

### EXAMPLE 10

### Human T Cell Signal Model

We developed a second cellular assay in order to quantify the enhancement of T cell IL-2 upregulation upon blockade of CTLA-4 signal with the antibodies. The following materials and methods were utilized in connection with the experiments:

### Materials and Methods

Human PBMCs were prepared using Accuspin. Microtiter plates were precoated with an anti-CD3 antibody (Leu4, Becton Dickinson) (60 ng/ml) and incubated for 2 hours at 37°C. hPBMCs were added to the wells at 200,000 cells per well. Staphylcoccus Enterotoxin A (SEA) (Sigma) was added to the wells at 100 ng/ml. Antibodies were added to the wells, usually at 30 µg/ml. Cells were then stimulated for 48, 72 or 96 hours. Plates were centrifuged at the desired time-point, and supernatants were removed from the wells. Thereafter, supernatants were checked for IL-2 production using ELISA (R&D Systems).

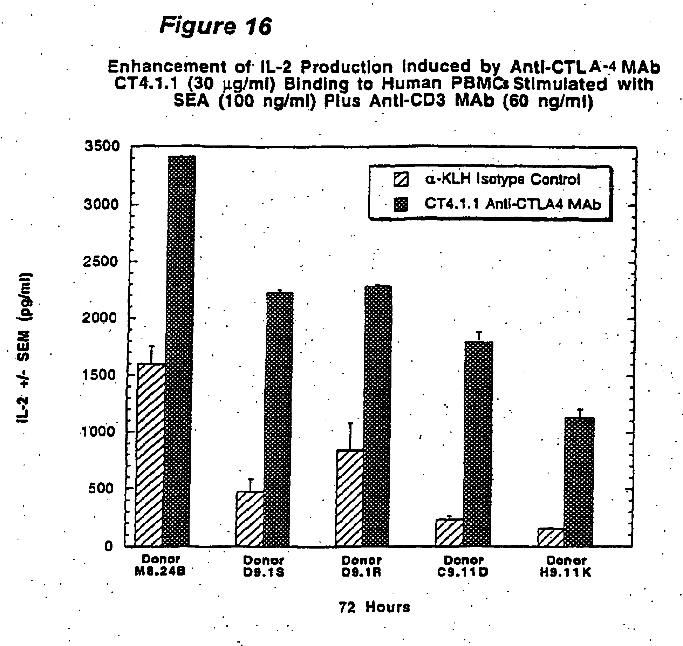

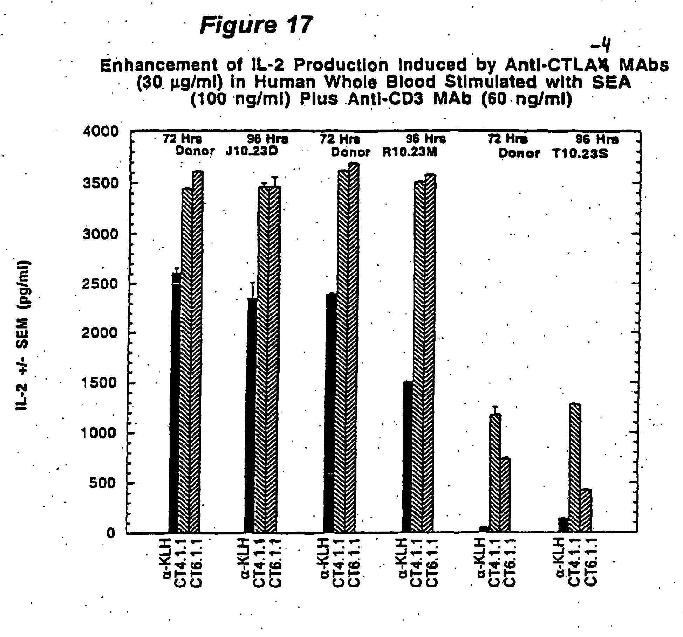

Results from these experiments are shown in Figures 16, 17, and 21. In Figure 16, induction of IL-2 production in hPBMCs from 5 donors was measured 72 hours after stimulation. In Figure 17, results are shown from measurement of whole blood, analyzing the difference in induction of IL-2 production in the blood of 3 donors as measured at 72 and 96 hours after stimulation.

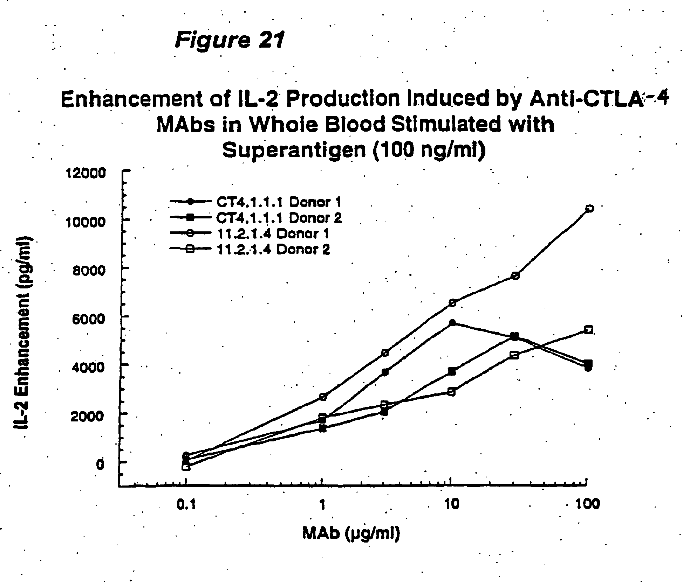

In Figure 21, the enhancement of IL-2 production in whole blood of 2 donors as measured at 72 hours after stimulation.

### EXAMPLE 11

### Tumor Animal Model

We have established an animal tumor model for the *in vivo* analysis of anti-murine-CTLA-4 antibodies in inhibiting tumor growth. In the model, a murine fibrosarcoma tumor is grown, and the animals are treated with anti-murine-CTLA-4 antibodies. The materials and methods for establishment of the model are provided below:

### Materials and Methods

Female A/J mice (6-8 weeks old) were injected subcutaneously on the dorsal side of the neck with 0.2 ml of Sa1N tumor cells (1x10⁶) (Baskar, 1995). Anti-murine CTLA-4 or an isorype-matched control antibody (Pharmingen, San Diego, CA, 200 ug/animal) were injected intraperitioneally on days 0, 4, 7 and 14 following the injection of tumor cells. Tumor measurements were taken during the course of the 3-4-week, experiments using a Starrett SPC Plus electronic caliper (Athol, MA) and tumor size was expressed as the surface area covered by tumor growth (nm²).

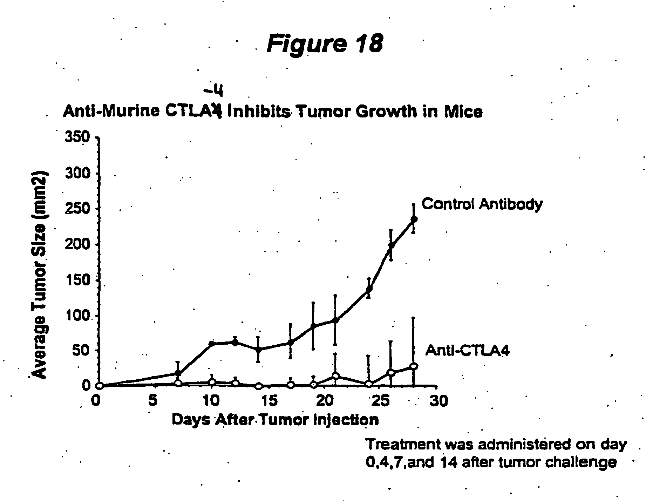

Figure 18 shows the inhibition of tumor growth with an anti-murine CTLA-4 antibody in a murine fibrosarcoma tumor model. As shown in Figure 18, animals treated with anti-CTLA-4 had a redaction in tumor growth as compared to animals treated with an isotype control antibody. Accordingly, anti-murine CTLA-4 mAbs are capable of inhibiting growth of a fibrosarcoma in a mouse tumor model.

It is expected that antibodies that are cross-reactive with murine CTLA-4 would perform similarly in the model. However, of the antibodies of the invention that have been cheeked for cross-reactivity, none are cross-reactive with murine CTLA-4.

### EXAMPLE 12

### Tumor Animal Model

In order to further investigate the activity of antibodies in accordance with the invention, a xenograft SCID mouse model was designed to test the eradication of established tumors and their derived metastases. In the model, SCBD mice are provided with grafted human T cells and are implanted with patient-derived non-small cell lung cancer (NSCC) or colorectal carcinoma (CC) cells. Implantation is made into the gonadal fat pads of SCID mice. The tumors are allowed to grow, and thereafter removed. The mice develop human-like tumor and liver metastases. Such a model is described in Bumpers et al, J. Surgical Res. 61:282-288 (1996).

It is expected that antibodies of the invention will inhibit the growth of tumors formed in such mice.
Alegre et al., J. Immunol. 157:4762-70 (1996).
Allison and Krummel, Science 270:932-933 (1995).
Balzano et al., Int. J. Cancer Suppl. 7:28-32 (1992).
Blair et al. J. Immunol. 160:12-5 (1998).
Blake and Litzi-Davis, BioConjugate Chem. 3:510-513 (1992).
Boussiotis et al., Proc.Natl.Acad.Sci.U.S.A.90:11059-63 (1993).
Bowie et al., Science 253:164 (1991).
Bruggeman et al., PNAS USA 86:6709-6713 (1989).
Bruggeman, M. and Neuberger, M.S., in Methods: A companion to Methods in Enzymology 2:159-165 (Lerner et al., eds., Academic Press (1991)).
Bruggemann et al., "Human antibody production in transgenic mice: expression from 100 kb of the human IgH locus," Eur. J. Immuno/. 21:1323-1326 (1991).
Bruggemann, M. and Neuberger, M.S., "Strategies for expressing human antibody repertoires in transgenic mice," Immunology Today 17:391-397 (1996).
Brunet et al., Nature 328:267-270 (1987).
Bumpers et al., J. Surgical Res. 61:282-288 (1996).
Capsey et al., Genetically Engineered Human Therapeutic Drugs (Stockton Press, NY (1988)).
Castan et al., Immunology 90:265-71 (1997).
Cepero et al., J.Exp.Med. 188:199-204 (1998).
Chen et al., "Immunoglobulin gene rearrangement in B-cell deficient mice generated by targeted deletion of the JH locus", International Immunology 5:647-656 (1993).
Chen et al., Cell 71:1093-1102 (1992).
Chen et al., Human Gene Therapy 5:95-601 (1994).
Chiswell and McCafferty, TIBTECH 10:80-84 (1992).
Choi et al., "Transgenic mice containing a human heavy chain immunoglobulin gene fragment cloned in a yeast artificial chromesome", Nature Genetics 4:117-123 (1993).
Chothia & Lesk, J. Mol. Biol. 196:901-917 (1987).
Chothia et al., Nature 342:878-883 (1989).
Chuang et al., J. Immunol. 159:144-151 (1997)
Coligan et al., Unit 2.1, "Enzyme-linked immunosorbent assays," in Current protocols in immunology (1994).
Cwirla et al., PNAS USA 87:6378-6382 (1990).
Dariavach et al. Eur. J. Immuno/. 18:1901-1905 (1988).
Dayhoff, M.O., in Atlas of Protein Sequence and Structure, pp. 101-110 (Volume 5, National Biomedical Research Foundation (1972)) and Supplement 2 to this volume, pp. 1-10.
de Boer et al., Eur.J.Immunol. 23:3120-5 (1993).
Eckstein, Ed., Oxford University Press, Oxford England (1991)).
Evans et al., J. Med. Chem. 30:1229 (1987).
Fallarino et al., J. Exp. Med. 188:205-10 (1998).
Fanger et al., Immunol. Methods 4:72-81 (1994).
Fauchere, J. Adv. Drug Res. 15:29 (1986).
Fishwild et al., "High-avidity human IgGγ monoclonal antibodies from a novel strain of minilocus transgenic mice," Nature Biotech. 14:845-851 (1996).
Freeman et al., J.Exp.Med. 178:2185-92 (1993).
Freeman et al. J. Immunol. 161:2708-15 (1998).
Freeman et al., Science 262:907-9 (1993).
Fundamental Immunology, Ch. 7 (Paul, W., ed., 2nd ed. Raven Press, N.Y. (1989)).
Galfre, G. and Milstein, C.; "Preparation of monoclonal antibodies: strategies and procedures," Methods Enzymol. 73:3-46 (1981).
Gorman et al., P.N.A.S. 79:6777 (1982).
Green and Jakobovits, J. Exp. Med. 188:483-495 (1998).
Green et al., Nature Genetics 7:13-21 (1994).
Grosschedl et al., Cell 41:885 (1985).
Hanes and Plucthau, PNAS USA 94:4937-4942 (1997).
Harding et al., Nature 356:607-609 (1994).
Harper et al., J. Immunol. 147:1037-44 (1991).
Hathcock et al., Science 262:905-7 (1993).
Hoganboom et al., Immunol. Reviews 130:43-68 (1992).
Horspool et al., J.Immunol. 160:2706-14 (1998).
Houghten et al., Biotechniques 13:412-421 (1992).
Houghten, PNAS USA 82:5131-5135 (1985).
Hurwitz et al., J.Neuroimmunol. 73:57-62 (1997).
Hurwitz et al., Proc.Natl.Acad.Sci.U.S.A. 95:10067-71 (1998).
Immunology - A Synthesis (2nd Edition, E.S. Golub and D.R. Gren, Eds., Sinauer Associates, Sunderland, Mass. (1991)).
Introduction to Protein Structure (C. Branden and J. Tooze, eds., Garland Publishing, New York, N.Y. (1991)).
Jakobovits et al., "Germ-line transmission and expression of a human-derived yeast artificial-chromosome" Nature 362:255-258 (1993).
Jakobovits, A. et al., "Analysis of homozygous mutant chimeric mice: Deletion of the immunoglobulin heavy-chain joining region blocks B-cell development and antibody production" Proc. Natl. Acad. Sci. USA 90:2551-2555 (1993).
Jakobovits, A., "Humanizing the mouse genome" Current Biology 4:761-763 (1994).
Jakobovits, A., "Production of fully human antibodies by transgenic mice" Current Opinion in Biotechnology 6:561-566 (1995).
Junghans et al., in Cancer Chemotherapy and Biotherapy, 655-686 (2d edition, Chafner and Longo, eds., Lippincott Raven (1996)).
Kabat et al. (1991), Sequences of Proteins of Immunological Interest, N.I.H. Publication No.91-3242.
Kabat, Sequences of Proteins of Immunological Interest (National Institutes of Health, Bethesda, MD. (1987 and 1991)).
Kostelny et al., J. Immunol. 148:1547-1553 (1992).
Krummel and Allison, J.Exp.Med. 182:459-65 (1995).
Krummel et al., Int.Immunol.8:519-23 (1996).
Kuchroo et al., Cell 80:707-18 (1995).
Kwon et al., PNAS USA 94: 8099-103 (1997).
LaPlanche et al., Nucl. Acids Res. 14:9081 (1986).
Lenschow et al., Proc.Natl.Acad.Sci.U.S.A.90:11054-8 (1993).
Lenschow et al., Science 257:789-792 (1992).
Lin et al., J.Exp.Med.188:199-204 (1998).
Linsley et al., J.Exp.Med.176:1595-604 (1992).
Linsley et al., J.Exp.Med. 174:561-569 (1991).
Linsley et al., Science 257:792-795 (1992).
Liu et al., J.Immunol.139:3521 (1987).
Liu et al., P.N.A.S. 84:3439 (1987).
Lonberg et al., "Antigen-specific human antibodies from mice comprising four distinct genetic modifications" Nature 368:856-859 (1994).
Luhder et al., J.Exp.Med. 187:427-32 (1998).
Marasco, Gene Therapy 4:11-15 (1997).
Markees et al., J.Clin.Invest.101:2446-55 (1998).
Marks et al., "Oligonucleotide primers for polymerase chain reaction amplification of human immunoglobulin variable genes and design of family-specific oligonucleotide probes" Eur.J.Immuno/. 21:985-991 (1991).
McCoy et al., J.Exp.Med.186:183-7 (1997).
Mendez et al., Nature Genetics 15:146-156 (1997).
Needleman and Wunsch, J. Mol. Biol. 48:443 (1970).
Okayama et al., Mol. Cell. Bio. 3:280 (1983).
Parmley and Smith, Gene 73:305-318 (1988).
Pearson and Lipman, Proc. Natl.Acad. Sci. (U.S.A.) 85:2444 (1988).
Perez et al., Immunity 6:411-7 (1997).
Perrin et al., Immunol Res 14:189-99 (1995).
Perrin et al., J.Immunol.157:1333-6 (1996).
Pinalla et al., Biotechniques 13:901-905 (1992).
Proteins, Structures and Molecular Principles (Creighton, Ed., W. H. Freeman and Company, New York (1984)).
Razi-Wolf et al., Proc.Natl.Acad.Sci.U.S.A.90:11182-6 (1993).
Remington's Pharmaceutical Sciences (15th ed, Mack Publishing Company, Easton, PA (1975)), particularly Chapter 87 by Blaug, Seymour.
Rizo and Gierasch, Ann. Rev. Biochem. 61:387 (1992).
Russel et al., Nuc/. Acids Research 21:1081-1085 (1993).
Schwartz, Cell 71: 1065 (1992).
Scott, TIBS 17:241-245 (1992).
Smith and Waterman, Adv. Appl. Math. 2:482 (1981).
Songsivilai & Lachmann, Clin. Exp. Immunol. 79: 315-321 (1990).
Stec et al., J. Am. Chem. Soc. 106:6077 (1984).
Stein et al., Nucl. Acids Res. 16:3209 (1988).
Taylor et al., "A transgenic mouse that expresses a diversity of human sequence heavy and light chain immunoglobulins" Nucleic Acids Research 20:6287-6295 (1992).
Taylor et al., "Human immunoglobulin transgenes undergo rearrangement, somatic mutations and class switching in mice that lack endogenous IgM." International Immunology 6:579-591 (1994).
The McGraw-Hill Dictionary of Chemical Terms (Parker, S., Ed.; McGraw-Hill, San Francisco (1985)).
Thornton et at., Nature 354:105 (1991).
Tivol et al., Immunity 3:541-7 (1995).
Townsend and Allison, Science 259:368 (1993).
Traunecker et al., Int. J. Cancer (Suppl.) 7:51-52 (1992).
Tuaillon et al., "Analysis of direct and inverted DJH rearrangements in a human Ig heavy chain transgenic minilocus", J. Immunol. 154:6453-6465 (1995).
Tuaillon et al., "Human immunoglobulin heavy-chain minilocus recombination in transgenic mice: gene-segment use in µ and γ transcripts" Proc. Natl. Acad. Sci. USA 90:3720-3724 (1993).
Uhlmann and Peyman, Chemical Reviews 90:543 (1990).
Van Parijs et al., J. Exp. Med.186:1119-28 (1997).
Veber and Freidinger, TINS p.392 (1985).
Vitetta, Immunol Today 14:252 (1993).
Walunas et al., Immunity 1:405-13 (1994).
Walunas et al., J.Exp.Med. 183:2541-50 (1996).
Waterhouse et al., Science 270:985-988 (1995).
Winter and Harris, Immunol. Today 14:43-46 (1993).
Wright et al., Crit. Reviews in Immunol. 12125-168 (1992).
Yang et al., Cancer Res 57:4036-41 (1997).
Yi-qun et al., Int.Immunol.8:37-44 (1996).
Zon et al., Anti-Cancer Drug Design 6:539 (1991).
Zon et al., Oligonucleotides and Analogues: A Practical Approach, pp. 87-108 (F. Eckstein, Ed., Oxford-University Press, Oxford England (1991)).
Fry et al., "Specific, irreversible inactivation of the epidermal growth factor receptor and erbB2, by a new class of tyrosine kinase inhibitor", Proc.Natl.Acad. Sci. U.S.A. 95:12022-7 (1998).
Hoffman et al., "A model of Cdc25 phosphatase catalytic domain and Cdk-interaction surface based on the presence of a rhodanese homology domain", J. Mol.Biol.282:195-208 (1998).
Ginalski et al., "Modelling of active forms of protein kinases: p38-a case study", Acta Biochim.Pol.44:557-64 (1997).
Jouko et al., "Identification of csk tyrosine phosphorylation sites and a tyrosine residue important for kinase domain structure", Biochem. J.322:927-35 (1997).
Singh et al., "Structure-based design of a potent, selective, and irreversible inhibitor of the catalytic domain of the erbB receptor subfamily of protein tyrosine kinases", J.Med.Chem.40:1130-5 (1997).
Mandel et al., "ABGEN: A knowledge-based automated approach for antibody structure modeling", Nat.Biotechnol.14:323-8 (1996).
Monfardini et al., "Rational design, analysis, and potential utility of GM-CSF antagonists", Proc.Assoc.Am.Physicians 108:420-31 (1996).
Furet et al., "Modelling study of protein kinase inhibitors: binding mode of staurosporine and origin of the selectivity of CGP 52411", J.Comput.Aided Mol. Des 9:465-72 (1995).
III et al., "Design and construction of a hybrid immunoglobulin domain with properties of both heavy and light chain variable regions", Protein Eng.10:949-57 (1997).
Martin et al. "The affinity-selection of a minibody polypeptide inhibitor of human interleukin-6", EMBO J.13:5303-9 (1994).

U.S. Patent Application Serial No. 07/466,008, filed January 12, 1990
U.S. Patent Application Serial No. 07/574,748, filed August 29, 1990
U.S. Patent Application Serial No. 07/575,962, filed August 31,1990
U.S. Patent Application Serial No. 07/610,515, filed November 8, 1990
U.S. Patent Application Serial No. 07/810,279, filed December 17, 1991
U.S. Patent Application Serial No. 07/853,408, filed March 18, 1992
U.S. Patent Application Serial No. 07/904,068, filed June 23, 1992
U.S. Patent Application Serial No. 07/919,297, filed July 24, 1992
U.S. Patent Application Serial No. 07/922,649, filed July 30, 1992
U.S. Patent Application Serial No. 07/990,860, filed December 16, 1992
U.S. Patent Application Serial No. 08/031,801, filed March 15,1993
U.S. Patent Application Serial No. 08/053,131, filed April 26, 1993
U.S. Patent Application Serial No. 08/096,762, filed July 22, 1993
U.S. Patent Application Serial No. 08/112,848, filed August 27, 1993.
U.S. Patent Application Serial No. 08/155,301, filed November 18,1993
U.S. Patent Application Serial No. 08/161,739, filed December 3, 1993
U.S. Patent Application Serial No. 08/165,699, filed December 10, 1993
U.S. Patent Application Serial No. 08/209,741, filed March 9, 1994
U.S. Patent Application Serial No. 08/234,145, filed April 28, 1994
U.S. Patent Application Serial No. 08/724,752, filed October 2, 1996
U.S. Patent Application Serial No. 08/730,639, filed October 11, 1996
U.S. Patent Application Serial No. 08/759,620, filed December 3, 1996
U.S. Patent Application Serial No. 08/759,620, filed December 3, 1996

U.S. Patent No. 4,399,216
U.S. Patent No. 4,681,581
U.S. Patent No. 4,683,195
U.S. Patent No. 4,683,202
U.S. Patent No. 4,735,210
U.S. Patent No. 4,740,461
U.S. Patent No. 4,816,397
U.S. Patent No. 4,912,040
U.S. Patent No. 4,959,455
U.S. Patent No. 5,101,827
U.S. Patent No. 5,102,990 (RE 35,500)
U.S. Patent No. 5,151,510
U.S. Patent No. 5,194,594
U.S. Patent No. 5,434,131
U.S. Patent No. 5,530,101
U.S. Patent No. 5,545,806
U.S. Patent No. 5,545,807
U.S. Patent No. 5,585,089
U.S. Patent No. 5,591,669
U.S. Patent No. 5,612,205
U.S. Patent No. 5,625,126
U.S. Patent No. 5,625,825
U.S. Patent No. 5,633,425
U.S. Patent No. 5,643,763
U.S. Patent No. 5,648,471
U.S. Patent No. 5,661,016
U.S. Patent No. 5,693,761
U.S. Patent No. 5,693,792
U.S. Patent No. 5,697,902
U.S. Patent No. 5,703,057
U.S. Patent No. 5,714,350
U.S. Patent No. 5,721,367
U.S. Patent No. 5,733,743
U.S. Patent No. 5,770,197
U.S. Patent No. 5,770,429
U.S. Patent No. 5,773,253
U.S. Patent No. 5,777,085
U.S. Patent No. 5,789,215
U.S. Patent No. 5,789,650
U.S. Patent No. 5,811,097

European Patent No. EP 0 546 073 B1
European Patent No. EP 0 463 151 B1, grant published June 12, 1996

International Patent Application No. WO 92/02190
International Patent Application No. WO 92/03918
International Patent Application No. WO 92/22645
International Patent Application No. WO 92/22647
International Patent Application No. WO 92/22670
International Patent Application No. WO 93/00431
International Patent Application No. WO 93/12227
International Patent Application No. WO 94/00569
International Patent Application No. WO 94/02602, published February 3, 1994
International Patent Application No. WO 94/25585
International Patent Application No. WO 94/29444
International Patent Application No. WO 95/01994
International Patent Application No. WO 95/03408
International Patent Application No. WO 95/24217
International Patent Application No. WO 95/33770
International Patent Application No. WO 96/14436
International Patent Application No. WO 96/34096, published October 31, 1996
International Patent Application No. WO 97/13852
International Patent Application No. WO 97/20574
International Patent Application No. WO 97/38137
International Patent Application No. WO 98/24884
International Patent Application No. WO 98/24893, published June 11, 1998

## Claims

1. A human monoclonal antibody that binds to CTLA-4, or an antigen binding fragment thereof, wherein the heavy chain comprises the amino acid sequence of SEQ ID NO: 9 or an amino acid sequence at least 90% identical thereto and the light chain comprises the amino acid sequence of SEQ ID NO: 22 or an amino acid sequence at least 90% identical thereto, which antibody or fragment has the following properties:
(a) a binding affinity for CTLA-4 of 10⁻⁹ M or greater;
(b) inhibits binding between CTLA-4 and B7-1 with an IC₅₀ of 100 nM or lower;
(c) inhibits binding between CTLA-4 and B7-2 with an IC₅₀ of 100 nM or lower; and
(d) enhances cytokine production in an assay of human T cells by 500 pg/ml or greater.

2. The antibody or antigen binding fragment thereof according to claim 1, wherein the heavy chain amino acid sequence comprises the CDR1. CDR2 and CDR3 amino acid sequences of antibody 11.2.1 as shown in Figure 2.

3. The antibody or antigen binding fragment according to claim 1 or 2, wherein the light chain amino acid sequence comprises the CDR1, CDR2 and CDR3 amino acid sequences of antibody 11.2.1 as shown in Figure 5.

4. The antibody or antigen binding fragment thereof according to any one of claims 1 to 3, wherein the heavy chain comprises the amino acid sequence of antibody 11.2.1 as shown in Figure 2.

5. The antibody or antigen binding fragment according to any one of claims 1 to 4, wherein the light chain comprises the amino acid sequence of SEQ ID NO: 22.

6. A human monoclonal antibody that binds to CTLA-4, wherein the heavy chain comprises the amino acid sequence of antibody 11.2.1 as shown in Figure 2 and the light chain comprises the amino acid sequence of antibody 11.2.1 as shown in Figure 5.

7. A human monoclonal antibody that binds to CTLA-4, wherein the heavy chain comprises the amino acid sequence of SEQ ID NO: 70 and the light chain comprises the amino acid sequence of SEQ ID NO: 71.

8. A human monoclonal antibody that binds to CTLA-4, or an antigen binding fragment thereof, wherein the heavy chain of said antibody comprises the amino acid sequence in SEQ ID NO: 5 and the light chain of said antibody comprises the amino acid sequence in SEQ ID NO: 18.

9. A human monoclonal antibody that binds to CTLA-4, or an antigen binding fragment thereof, wherein the heavy chain of said antibody comprises the amino acid sequence in SEQ ID NO: 10 and the light chain of said antibody comprises the amino acid sequence in SEQ ID NO: 23.

10. A pharmaceutical composition comprising the antibody or antigen binding fragment according to any one of claims 1 to 9 and a pharmaceutically acceptable carrier.

11. A cell line that produces the antibody or antigen binding fragment according to any one of claims 1 to 9.

12. The cell line of claim 11 that is a mammalian cell line.

13. The cell line of claim 12 that is a CHO cell line or an NSO cell line.

14. A nucleic acid that encodes the light and/or heavy chain of the antibody or antigen binding fragment as defined in any one of claims 1 to 9.

15. A pharmaceutical composition, comprising the antibody or antigen binding fragments according to any one of claims 1 to 9 and a pharmaceutically acceptable carrier, for treating cancer.

16. A method for production of the human CTLA-4 antibody or antigen binding fragment according to any one of claims 1 to 9 comprising expressing said antibody or antigen binding fragment in a host cell line and recovering said antibody or antigen binding fragment.

17. The method according to claim 16, wherein said host cell line is a mammalian cell line.

18. The method according to claim 17, wherein said mammalian cell line is a CHO cell line or an NSO cell line.

19. The antibody or antigen-binding fragment according to any one of claims 1 to 9 for use in treating cancer.

## Patentansprüche

1. Menschlicher monoclonaler Antikörper, welcher an CTLA-4 bindet, oder ein Antigen-bindendes Fragment davon, wobei die schwere Kette die Aminosäuresequenz von SEQ ID NO: 9 oder eine Aminosäuresequenz, die zu mindestens 90% identisch dazu ist, und die leichte Kette die Aminosäuresequenz von SEQ ID NO: 22 oder eine Aminosäuresequenz, die zu mindestens 90% identisch dazu ist, umfasst, wobei der Antikörper oder das Fragment folgende Eigenschaften hat:
(a) Bindeaffinität für CTLA-4 von 10⁻⁹ M oder größer;
(b) hemmt die Bindung zwischen CTLA-4 und B7-1 mit einem IC₅₀-Wert von 100 nM oder weniger;
(c) hemmt die Bindung zwischen CTLA-4 und B7-2 mit einem IC₅₀-Wert von 100 nM oder weniger; und
(d) erhöht die Cytokinherstellung in einem Assay von menschlichen T-Zellen um 500 pg/ml oder höher.

2. Antikörper oder Antigen-bindendes Fragment davon nach Anspruch 1, wobei die Aminosäuresequenz der schweren Kette die CDR1-, CDR2-und CDR3-Aminosäuresequenzen von Antikörper 11.2.1 wie in Figur 2 gezeigt umfasst.

3. Antikörper oder Antigen-bindendes Fragment davon nach Anspruch 1 oder 2, wobei die Aminosäuresequenz der leichten Kette die CDR1-, CDR2- und CDR3-Aminosäuresequenzen von Antikörper 11.2.1 wie in Figur 5 gezeigt umfasst.

4. Antikörper oder Antigen-bindendes Fragment davon nach einem der Ansprüche 1 bis 3, wobei die schwere Kette die Aminosäuresequenz von Antikörper 11.2.1 wie in Figur 2 gezeigt umfasst.

5. Antikörper oder Antigen-bindendes Fragment davon nach einem der Ansprüche 1 bis 4, wobei die leichte Kette die Aminosäuresequenz von SEQ ID NO: 22 umfasst.

6. Menschlicher monoclonaler Antikörper, welcher an CTLA-4 bindet, wobei die schwere Kette die Aminosäuresequenz von Antikörper 11.2.1 wie in Figur 2 gezeigt und die leichte Kette die Aminosäuresequenz von Antikörper 11.2.1 wie in Figur 5 gezeigt umfasst.

7. Menschlicher monoclonaler Antikörper, welcher an CTLA-4 bindet, wobei die schwere Kette die Aminosäuresequenz von SEQ ID NO: 70 und die leichte Kette die Aminosäuresequenz von SEQ ID NO: 71 umfasst.

8. Menschlicher monoclonaler Antikörper, welcher an CTLA-4 bindet, oder ein Antigen-bindendes Fragment davon, wobei die schwere Kette des Antikörpers die Aminosäuresequenz in SEQ ID NO: 5 und die leichte Kette des Antikörpers die Aminosäuresequenz in SEQ ID NO: 18 umfasst.

9. Menschlicher monoclonaler Antikörper, welcher an CTLA-4 bindet, oder ein Antigen-bindendes Fragment davon, wobei die schwere Kette des Antikörpers die Aminosäuresequenz in SEQ ID NO: 10 und die leichte Kette des Antikörpers die Aminosäuresequenz in SEQ ID NO: 23 umfasst.

10. Arzneimittel, welches den Antikörper oder das Antigen-bindende Fragment nach einem der Ansprüche 1 bis 9 und einen pharmazeutisch verträglichen Träger umfasst.

11. Zelllinie, welche den Antikörper oder das Antigen-bindende Fragment nach einem der Ansprüche 1 bis 9 produziert.

12. Zelllinie nach Anspruch 11, welche eine Säugerzelllinie ist.

13. Zelllinie nach Anspruch 12, die eine CHO-Zelllinie oder eine NSO-Zelllinie ist.

14. Nucleinsäure, welche die leichte und/oder schwere Kette des Antikörpers oder des Antigen-bindenden Fragments gemäß der Definition in einem der Ansprüche 1 bis 9 codiert.

15. Arzneimittel, welches den Antikörper oder das Antigen-bindende Fragment nach einem der Ansprüche 1 bis 9 und einen pharmazeutisch verträglichen Träger umfasst, zur Behandlung von Krebs.

16. Verfahren zur Herstellung des menschlichen CTLA-4 Antikörpers oder Antigen-bindenden Fragments nach einem der Ansprüche 1 bis 9, umfassend die Expression des Antikörpers oder Antigen-bindenden Fragments in einer Wirtszelllinie und Gewinnung des Antikörpers oder Antigen-bindenden Fragments.

17. Verfahren nach Anspruch 16, wobei die Wirtszelllinie eine Säugerzelllinie ist.

18. Verfahren nach Anspruch 17, wobei die Säugerzelllinie eine CHO-Zelllinie oder NSO-Zelllinie ist.

19. Antikörper oder Antigen-bindendes Fragment nach einem der Ansprüche 1 bis 9 zur Verwendung in der Behandlung von Krebs.

## Revendications

1. Anticorps monoclonal humain qui se lie à CTLA-4, ou un fragment de liaison d'antigène de celui-ci, où la chaîne lourde comprend la séquence d'acides aminés de SEQ ID NO: 9 ou bien une séquence d'acides aminés au moins à 90% identique à celle-ci, et la chaîne légère comprend la séquence d'acides aminés de SEQ ID NO: 22 ou bien une séquence d'acides aminés au moins à 90% identique à celle-ci, ledit anticorps ou fragment possède les propriétés suivantes:
(a) une affinité de liaison pour CTLA-4 de 10⁻⁹ M ou plus grande;
(b) empêche la liaison entre CTLA-4 et B7-1 avec un IC₅₀ de 100 nM ou plus bas;
(c) empêche la liaison entre CTLA-4 et B7-2 avec un IC₅₀ de 100 nM ou plus bas; et
(d) fait augmenter la production de cytokine dans un dosage de cellules T humaines de 500 pg/ml ou plus élevée.

2. Anticorps ou fragment de liaison d'antigène de celui-ci selon la revendication 1, dans lequel la séquence d'acides aminés de la chaîne lourde comprend les séquences d'acides aminés CDR1, CDR2 et CDR3 de l'anticorps 11.2.1, comme représenté sur la Figure 2.

3. Anticorps ou fragment de liaison d'antigène selon la revendication 1 ou 2, où la séquence d'acides aminés de la chaîne légère comprend les séquences d'acides aminés CDR1, CDR2 et CDR3 de l'anticorps 11.2.1, comme représenté sur la Figure 5.

4. Anticorps ou fragment de liaison d'antigène de celui-ci selon l'une quelconque des revendications 1 à 3, où la chaîne lourde comprend la séquence des acides aminés de l'anticorps 11.2.1, comme représenté sur la Figure 2.

5. Anticorps ou fragment de liaison d'antigène selon l'une quelconque des revendications 1 à 4, où la chaîne légère comprend la séquence des acides aminés de SEQ ID NO: 22.

6. Anticorps monoclonal humain qui se lie à CTLA-4, où la chaîne lourde comprend la séquence des acides aminés de l'anticorps 11.2.1, comme représenté sur la Figure 2, et la chaîne légère comprend la séquence des acides aminés de l'anticorps 11.2.1, comme représenté sur la Figure 5.

7. Anticorps monoclonal humain qui se lie à CTLA-4, ou la chaîne lourde comprend la séquence des acides aminés de SEQ ID NO: 70, et la chaîne légère comprend la séquence des acides aminés de SEQ ID NO: 71.

8. Anticorps monoclonal humain qui se lie à CTLA-4, ou un fragment de liaison d'antigène de celui-ci, où la chaîne lourde dudit anticorps comprend la séquence des acides aminés dans SEQ ID NO: 5, et la chaîne légère dudit anticorps comprend la séquence des acides aminés dans SEQ ID NO: 18.

9. Anticorps monoclonal humain qui se lie à CTLA-4, ou un fragment de liaison d'antigène de celui-ci, où la chaîne lourde dudit anticorps comprend la séquence des acides aminés dans SEQ ID NO: 10, et la chaîne légère dudit anticorps comprend la séquence des acides aminés dans SEQ ID NO: 23.

10. Composition pharmaceutique comprenant l'anticorps ou le fragment de liaison d'antigène selon l'une quelconque des revendications 1 à 9 et un support pharmaceutiquement acceptable.

11. Ligne de cellules qui produit l'anticorps ou le fragment de liaison d'antigène selon l'une quelconque des revendications 1 à 9.

12. Ligne de cellules selon la revendication 11, qui est une ligne de cellule de mammifère.

13. Ligne de cellules selon la revendication 12, qui est une ligne de cellules CHO ou une ligne de cellule NSO.

14. Acide nucléique qui code pour la chaîne légère et/ou lourde de l'anticorps ou du fragment de liaison d'antigène tel que défini dans l'une quelconque des revendications 1 à 9.

15. Composition pharmaceutique, comprenant l'anticorps ou le fragment de liaison d'antigène selon l'une quelconque des revendications 1 à 9 et un support pharmaceutiquement acceptable, pour traiter le cancer.

16. Procédé de production de l'anticorps CTLA-4 humain ou fragment de liaison d'antigène selon l'une quelconque des revendications 1 à 9, comprenant l'expression dudit anticorps ou fragment de liaison d'antigène dans une ligne de cellules hôtes et la récupération dudit anticorps ou fragment de liaison d'antigène.

17. Procédé selon la revendication 16, dans lequel ladite ligne de cellules hôtes est une ligne de cellules de mammifère.

18. Procédé selon la revendication 17, où ladite ligne de cellules de mammifère est une ligne de cellules CHO ou une ligne de cellules NSO.

19. Anticorps ou fragment de liaison d'antigène selon l'une quelconque des revendications 1 à 9 pour utilisation dans le traitement du cancer.
